(19) Europäisches Patentamt
European Patent Office
Office européen des brevets

(11) EP 2 601 208 B1

(12) **EUROPEAN PATENT SPECIFICATION**

(45) Date of publication and mention of the grant of the patent:
**11.02.2015 Bulletin 2015/07**

(21) Application number: **11739065.8**

(22) Date of filing: **03.08.2011**

(51) Int Cl.:
***C07K 1/22*** *(2006.01)*

(86) International application number:
**PCT/EP2011/063392**

(87) International publication number:
**WO 2012/017021 (09.02.2012 Gazette 2012/06)**

# (54) LIGANDS FOR ANTIBODY AND Fc-FUSION PROTEIN PURIFICATION BY AFFINITY CHROMATOGRAPHY

LIGANDEN FÜR ANTIKÖRPER- UND FC-FUSIONSPROTEINAUFREINIGUNG MITTELS AFFINITÄTSCHROMATOGRAFIE

LIGANDS POUR LA PURIFICATION D'ANTICORPS ET DE PROTÉINE DE FUSION À FC PAR CHROMATOGRAPHIE D'AFFINITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2010 EP 10008089**

(43) Date of publication of application:
**12.06.2013 Bulletin 2013/24**

(73) Proprietor: **Graffinity Pharmaceuticals GmbH**
**69120 Heidelberg (DE)**

(72) Inventors:
- **BITTERMANN, Holger**
  **69198 Schriesheim (DE)**
- **BURKERT, Klaus**
  **69126 Heidelberg (DE)**
- **ARNOLD, Marc**
  **74937 Spechbach (DE)**
- **KEIL, Oliver**
  **69120 Heidelberg (DE)**
- **NEUMANN, Thomas**
  **69126 Heidelberg (DE)**
- **OTT, Inge**
  **68775 Ketsch (DE)**
- **SCHMIDT, Kristina**
  **69198 Schriesheim (DE)**
- **SCHWIZER, Daniel**
  **69118 Heidelberg (DE)**
- **SEKUL, Renate**
  **69198 Schriesheim (DE)**

(74) Representative: **Huhn, Michael et al**
**Stolmár & Partner**
**Blumenstr. 17**
**80331 München (DE)**

(56) References cited:
**WO-A1-97/41856**
**WO-A1-2009/138714**
**WO-A1-2010/084425**
**WO-A2-2009/141384**
**US-A1- 2005 065 066**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 601 208 B1

## Description

The present invention relates to the field of protein separation, preferably the purification of monoclonal and polyclonal antibodies and fusion proteins containing an immunoglobulin Fc segment, by affinity separation techniques, in particular chromatography using small molecule ligands.

Immunoglobulins are a class of soluble proteins found in body fluids of humans and other vertebrates. They are also termed "antibodies" and play a key role in the processes of recognition, binding and adhesion of cells. Antibodies are oligomeric glycoproteins which have a paramount role in the immune system by the recognition and elimination of antigens, in general bacteriae and viruses.

The polymeric chain of antibodies is constructed such that they comprise so-called heavy and light chains. The basic immunoglobulin unit consists of two identical heavy and two identical light chains connected by disulfide bridges. There are five types of heavy chains (a, $\gamma$, $\delta$, $\varepsilon$, $\mu$), which determine the immunoglobulin classes (IgA, IgG, IgD, IgE, IgM). The light chain group comprises two subtypes, $\lambda$ and $\kappa$.

IgGs are soluble antibodies, that can be found in blood and other body fluids. They are built by B-cell derived plasma cells as response to and to neutralize bacterial or other pathogens. An IgG is an Y-shaped glycoprotein with an approximate molecular weight of 150 kDa, consisting of two heavy and two light chains. Each chain is distinguished in a constant and in a variable region. The two carboxy terminal domains of the heavy chains are forming the Fc fragment ("constant fragment"), the amino terminal domains of the heavy and light chains are recognizing the antigen and are named Fab fragment ("antigen-binding fragment").

Fc fusion proteins are created through a combination of an antibody Fc fragment and a protein or protein domain that provides the specificity for a given drug target. Examples are domain antibody-Fc fusion proteins, where the two heavy chains of the Fc fragments are linked either to the variable domains of the heavy ($V_H$) or light chains ($V_L$) of specific antibodies. Other Fc fusion proteins are combinations of the Fc fragment with any type of therapeutic proteins or protein domains. The Fc part is considered to add stability and deliverability to the protein drug.

Therapeutic antibodies and Fc fusion proteins are used to treat various diseases, prominent examples include rheumatoid arthritis, psoriasis, multiple sclerosis and many forms of cancer. Therapeutic antibodies can be monoclonal or polyclonal antibodies. Monoclonal antibodies are derived from a single antibody producing cell line, showing identical specificity towards a single antigen. Possible treatments for cancer involve antibodies that are neutralizing tumour cell specific antigens. Bevacizumab (Avastin, Genentech) is a monoclonal antibody which neutralizes the vascular endothelial growth factor (VEGF), thereby preventing the growth of new blood vessels into the tumour tissue.

Therapeutic fusion proteins such as Etanercept (Enbrel, Amgen, TNF-Receptor domain linked to Fc fragment) or Alefacept (Amevive, Biogen Idec, LFA-3 linked to Fc portion of human IgG1) are used or developed as drugs against autoimmune diseases.

Protein bioseparation which refers to the recovery and purification of protein products from various biological feed streams is an important unit operation in the food, pharmaceutical and biotechnological industry. More and more therapeutic monoclonal antibodies (Mabs) and fusion proteins are entering the market or are currently in clinical development. Such proteins require an exceptionally high purity which is achieved by elaborate multi-step purification protocols. Downstream processing and purification constitute about 50 to 80 % of the manufacturing cost, hence considerable efforts are under way to develop new or improve existing purification strategies (**1**).

Affinity chromatography is one of the most effective chromatographic methods for protein purification. It is based on highly specific protein-ligand interactions. The ligand is immobilized covalently on the stationary phase which is used to capture the target protein from the feed stock solution. Affinity ligands can bind their target with high specificity and selectivity, enabling up to thousand fold higher enrichment at high yields even from complex mixtures.

Typically, affinity chromatography on protein A is the first step in most Mab and Fc fusion protein purification schemes. Protein A is a cell wall associated protein exposed on the surface of the bacterium Staphylococcus aureus. It binds with nanomolar affinity to the constant part (Fc domain) of immunoglobulins from various species, in particular to human subtypes IgG1, IgG2 and IgG4 (**2**). However, the use of Protein A is limited by leaching into the product and poor stability under harsh conditions applied for sanitization and cleaning in place procedures. The chemical stability of Protein A can be improved by using genetically engineered Protein A variants for Mab purification. Yet, the high costs of Protein A resins have resulted in the search for suitable alternatives, in particular selected from small molecules.

Mabsorbent A2P (Prometic Biosciences) is a small molecule ligand for the purification of immunoglobulins. However, the ligand does not show an appropriate selectivity (**3**) and is not applied in process chromatography.

Another approach uses mixed mode chromatography as primary capture step in antibody purification. The most common material is based on immobilized 2-mercaptoethylpyridine (MEP HyperCel, Pall Corporation), which effectively captures IgG from fermentation broth but shows lower clearance of host cell proteins (HCP) in comparison to protein A. (**4**).

Synthetic affinity ligands that are more readily available, preferably cheaper than protein-based ligands, are more robust under stringent conditions and have a selectivity comparable to or even higher than Protein A would provide a suitable solution for antibody and Fc fusion protein purification.

Synthetic small molecule affinity ligands are of particular interest for the purification of therapeutic proteins due to their generally higher chemical stability and their lower production costs. Synthetic affinity ligands that are more readily available, preferably cheaper than protein-based ligands, are more robust under stringent conditions and have a selectivity comparable to or even higher than Protein A would provide a suitable solution for antibody and Fc fusion protein purification. Depending on the target protein, such affinity ligands should preferably offer the same broad applicability as Protein A, recognizing the constant Fc region of IgG type immunoglobulins and Fc fusion proteins.

The problem underlying the present invention is the provision of a matrix comprising a small affinity ligand binding to the Fc region of antibodies and Fc fusion proteins.

The problem is solved by the embodiments of the present invention as laid out hereinafter.

The present invention is directed to the use, for affinity purification of a protein, preferably an antibody or fragment of an antibody, of a ligand-substituted matrix comprising a support material and at least one ligand covalently bonded to the support material, the ligand being represented by formula (I)

(I)

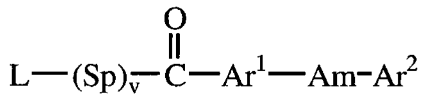

wherein

L is the linking point on the support material to which the ligand is attached;
Sp is a spacer group;
v is 0 or 1;
Am is an amide group -$NR^1$-C(O)-, and wherein either $NR^1$ is attached to $Ar^1$ and -C(O)- is attached to $Ar^2$, or -C(O)- is attached to $Ar^1$ and $NR^1$ is attached to $Ar^2$; and
$R^1$ is hydrogen or $C_1$ to $C_4$ alkyl, preferably hydrogen or methyl; and more preferably hydrogen;
$Ar^1$ is a divalent 5- or 6-membered substituted or unsubstituted aromatic ring, wherein the substituents are selected from $C_1$ to $C_4$ alkoxy, $C_1$ to $C_4$ alkyl, and combinations thereof;
$Ar^2$ is 5- or 6-membered unsubstituted or substituted heterocyclic aromatic ring which is

(a) attached to a further 5- or 6-membered aromatic ring via a single bond; or
(b) fused to a further 5- or 6-membered aromatic ring as part of a multicyclic ring system; or
(c) attached to at least one substituent selected from $C_1$ to $C_4$ alkyl; $C_2$ to $C_4$ alkenyl; $C_2$ to $C_4$ alkynyl; a halogen; $C_1$ to $C_4$ haloalkyl; hydroxyl-substituted $C_1$ to $C_4$ alkyl; $C_1$ to $C_4$ alkoxy; hydroxyl-substituted $C_1$ to $C_4$ alkoxy; $C_1$ to $C_4$ alkylamino; $C_1$ to $C_4$ alkylthio; and combinations thereof, and wherein the first aromatic ring or the second aromatic ring or both, in alternative (a) and (b) may optionally carry one or more further substituents from those mentioned under (c).

The ligand-substituted matrix of the present invention, in accordance with the formula (I), has the structure according to formula (Ia), in one embodiment of the invention. In a further embodiment of the invention, the ligand-substituted matrix of the present invention, in accordance with the formula (I), has the structure according to formula (Ib). In either formula (Ia) and (Ib), L, Sp, N, $R^1$, $Ar^1$ and $Ar^2$ and the integer v have the meanings defined above in connection with formula (I)

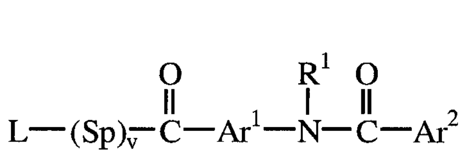 (Ia)

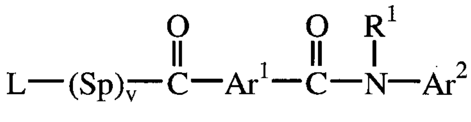 (Ib)

$Ar^1$ may also be denoted $AR^1$, and $Ar^2$ may also be denoted $AR^2$. Furthermore, Sp may also be denoted V. This is the nomenclature as used in the patent application EP 10008089.4 of which the present application claims priority. Still, $R^1$ as defined in the context with formula (I) and as shown in formulae (Ia) and (Ib) corresponds to $R^4$ as defined and shown in the said priority document.

Ligands according to the present invention bind to polyclonal and monoclonal IgG of human origin, in particular human $IgG_1$, $IgG_2$ and $IgG_4$ and polyclonal and monoclonal IgGs from different animal species such as rabbit and mouse immunoglobulins.

The present invention is also directed to the ligand-substituted matrix and the ligands (which may also be termed "compounds") attached to the matrix at the linking point L, optionally via a spacer group Sp.

L in the above formula (I) is the linking point, also referred to as "point of attachment". The person skilled in the art is aware of appropriate linking points/points of attachment.

It is understood that the linking point L is either directly connected with the support material or via a spacer. Typically L is part of a moiety resulting from the reaction of an appropriate functional group on the support material with a corresponding functional group on the precursor compound of the ligand to form the ligand.

In one embodiment, L is a bond, preferably a single bond, which is directly attached to the support material, in general via an appropriate functional group on the material. In this context, the term "support material" refers to polymers known to the person skilled in the art and available on the market which lend themselves for the purposes of the present invention. A definition of "support material" is provided below. In general, the support material comprises functional groups for the attachment of molecules, in general those of the present invention. In the context of the present invention, the functional groups on the support material are regarded as a part of the support material; this also applies in cases where the ligands of the present invention are connected to the support material via a bond (i. e. L is a bond and does not comprise a spacer group Sp, see below), and wherein the bond is directly formed between the respective ligand according to the invention and the functional group on the support material.

"Functional group" in the present context refers, on the one hand, to appropriate groups ("precursor groups") on the support material and, on the other hand, to appropriate groups ("precursor groups") present in the ligands according to the invention (e.g. present on the C=O group, or present on the spacer Sp). "Functional group" in the present context also refers to appropriate groups ("precursor groups") on the spacer group Sp. "Precursor groups" or "functional groups" in the present context are groups which are capable of a chemical reaction with complementary "precursor groups" or "functional groups", under formation of a chemical bond. If necessary, additional reagents may be used for the formation of said chemical bond. As is clear from the foregoing, during the reaction the functional groups/precursor groups are transformed into a chemical bond under a transformation of the original functionality into a "final functionality" or "connecting unit". From the foregoing, it is clear that a functional group/precursor group also is or can be a "complementary group".

Examples of functional groups on the support material are known to the person skilled in the art and include -OH, -SH, $-NH_2$, >NH -COOH, $-SO_3H$, -CHO, - $NHNH_2$, $-P(=O)(OH)_2$, -O-PH(=O)(OH), $-P(OH)_2$, $-O-P(=O)(OH)_2$, ester, ether, thioether, epoxide, natural or non natural amino acids, carboxylic amides, maleimide, ketone, sulphoxide, sulfone, urea, isourea, imidocarbonate, sulphonamide, sulphonylhydrazide, sulphonic ester, phosphate, phosphonate, phosphoric amide, phosphonic amide, alkyne, oxime ether, oxime, azide, alkoxyamine, semicarbazide, sulphonic halide, phosphonic halide, phosphoric halide, carboxylic active esters, sulphonic active esters, phosphonic active esters, phosphoric active esters, phosphoramidite, cyanate, thiocyanate, isocyanate, isothiocyanate, maleimide, vinyl sulphonyl, Cl, Br, I, alkene, alkyl phosphonium.

Examples of functional groups attached to the -C=O group on the ligands of the invention are known to the person skilled in the art and include hydroxy, thiol, F, Cl, Br, ester, ether, thioether, carboxylic acid, epoxide, amine, amide, amino acids, carboxylic amides, maleimide, aldehyde, ketone, sulphonic acid, sulphoxide, sulphone, urea, isourea, imidocarbonate, sulphonamide, sulphonic ester, phosphate, phosphonate, phosphoric amide, phosphonic amide, hydrazine, oxime, azide, alkene, alkyne,, hydroxylamine, thiourea, isocyanate, isothiocyanate, N-hydroxysuccinimidyl, 1-hydroxybenzotriazolyl, 7-aza-1-hydroxybenzotriazolyl, 6-chloro-1-hydroxybenzotriazolyl.

In this embodiment of the invention, the bond or the chemical unit resulting from the reaction of an appropriate functional group on the support material with an appropriate functional group (or a "precursor group") on the -C=O group on the ligands of the invention constitutes the linking point L by which the ligands of the invention are attached to the support material/the matrix. Accordingly, the chemical reaction between the functional group on the support material and the functional group (or "complementary group") on the -C=O group on the ligands of the invention connects the ligand according to the invention with the support material via the linking point L.

In a further embodiment of the invention, L is connected to a spacer group -Sp-. In this embodiment, L is a bond or a chemical unit resulting from the reaction of an appropriate functional group on the support material with an appropriate functional group (or a "precursor group") on the spacer group Sp. Accordingly, the chemical reaction between the functional group on the support material and the functional group (or "complementary group") on the spacer group Sp connects the ligand according to the invention with the support material via the linking point L.

If v in formula (I) is 0, then the ligand is directly bonded to L. In other embodiments the ligand is bonded to the support material via a spacer group Sp. This applies when v in formula (I) is different from 0.

The spacer group Sp preferably is a hydrocarbon group which may contain, in addition to C and H atoms, further atoms. Appropriate further atoms are known to the person skilled in the art. Preferred atoms include O, S, N, P, Si. The hydrocarbon group may be linear or branched or cyclic. Sp is linked to the -C=O group of the ligands according to the invention, in general Sp is found in alpha-position to the -C=O group. Furthermore, Sp contains functional groups (precursor groups) by which the ligands of the invention can be covalently linked with the matrix in a chemical reaction under formation of a final functionality (which may also be termed connecting unit).

The spacer group Sp preferably contains a connecting unit/final functionality which covalently connects the ligand with the support material via linking point L. Appropriate connecting unit/final functionalities are known to the person skilled in the art. Preferably the connecting unit is derived from suitable precursor groups including $-NH_2$, >NH, -SH, - COOH, $-SO_3H$, -CHO, -OH, $-NHNH_2$, $-P(=O)(OH)_2$, -O-PH(=O)(OH), $-P(OH)_2$, $-O-P(=O)(OH)-NHNH_2$, natural or non natural amino acids, carboxylic amides, ethers, ester, thioether, epoxide, maleimide, ketone, sulphoxide, sulphone, urea, isourea, imidocarbonate, sulphonamide, sulphonylhydrazide, sulphonic ester, phosphate, phosphonate, phosphoric amide, phosphonic amide, hydrazine, oxime, azide, alkoxyamine, semicarbazide, sulphonic halide, phosphonic halide, phosphoric halide, carboxylic active esters, sulphonic active esters, phosphonic active esters, phosphoric active esters, phosphoramidite, cyanate, thiocyanate, alkyl halide, alkyl sulphonate, isocyanate, isothiocyanate, vinyl sulphonyl, carboxylic halide, alkene and alkyne groups.Typically the connecting unit is attached to a carbon chain that may be interrupted by one or more heteroatoms such as oxygen atoms or carboxamide groups. In one embodiment, the connecting unit is attached to an alkylene or an alkylene-polyoxyalkylene group such as $-CH_2-CH_2-(O-CH_2-CH_2)x-$ with x ranging from 1 to 10, preferably from 1 to 6.

Typically the functional groups on the spacer group are attached to a hydrocarbon group which may contain one or more heteroatoms such as N, O, P, S, Si. The spacer group therefore is a hydrocarbon group containing one or more heteroatoms preferably selected from. N, O, P, S, Si Preferably the atom which is linked to the C=O group of the ligands of the invention is nitrogen, oxygen, carbon or sulfur. Appropriate chemical entities/chemical units contained in Sp are known to the person skilled in the art. Sp in general contains at least one unit selected from alkylene, alcohol, amine, amide, carbonyl, alkyleneoxy and phosphate. The alkylene unit preferably has 1-30, preferably 1-12, in particular 1-6 carbon atoms. The alcohol can be a primary, secondary or tertiary alcohol. The amine can be a non organic or an organic amine. The amine, furthermore, can be a diamine or a triamine, preferably a diamine. The amide can be a non organic or an organic amide, and can be derived from a diamine or a triamine, preferably a diamine. The alkyleneoxy unit can comprise a linear or a branched alkylene unit. The alkyleneoxy unit is preferably an ethylenoxy or a propyleneoxy unit, preferably an ethylenoxy unit. The unit can also comprise two or more of the above cited entities/units, e.g. an amino acid. The amino acid can be a natural or a non natural amino acid. The amino acid can be linear or branched. Branched amino acids may serve as a branching point, enabling the connection of more than one ligand moiety to one point of attachment ("L"). Examples for preferred amino acids include 6-aminohexanoic acid, 8-amino-3,6-dioxaoctanoic acid, 5-amino-3-oxapentanoic acid, glycine, beta-alanine, lysine, ornithine, glutamic acid, 2,3-diaminopropionic acid. 2,4-diaminobutyric acid and serine.

The spacer group Sp may consist of one unit as described before, or of more than one unit. If the spacer group Sp consists of more than one unit as described before, said units are interconnected by connecting groups. Such connecting groups include amide, urea, hydrazide, oxalic diamide, oxime, hydrazone, triazole, thiourea, isourea, ether, with amide and urea being preferred. A phosphate group may as well serve as a connecting group. The phosphate group may be linked to one, two or three units as described before. If the phosphate group is linked to three units as described before, it may serve as a branching point, enabling the connection of more than one ligand moiety to one point of attachment ("L").

Preferred units of the spacer group Sp include amines, preferably organic amines, diamines, like ethylenediamine, piperazine, homopiperazine, $-(CH_2)_n-C(=O)$, $-NH-((CH_2)_2O)_n-(CH_2)_n-NH-$; $-NH-(CH_2)_n-NH-$, $-NH-(CH_2)_n-$, -NH-CH-(C(=O)NH-, $-NH-(O-C_2H_4)_n-CH_2-C(=O)-$ wherein n is an integer from 1-30, preferably 1-12. In one embodiment, n is an integer from 1-6, i.e. 1, 2, 3, 4, 5 or 6. Further preferred units of the spacer group Sp include amino acids, which may be natural or unnatural amino acids, in particular 6-aminohexanoic acid, 8-amino-3,6-dioxaoctanoic acid, 5-amino-3-oxapentanoic acid, lysine and glutamic acid. The named units and functional groups are thus part of the spacer group Sp and of the linking point L.

Particularly preferred examples of spacer groups Sp are $-NH-(CH_2)_nNH-$, $-NH-((CH_2)_2O)_n-(CH_2)_2-NH-$, wherein n is integer from 1-30, preferably 1-12. In one embodiment, n is an integer from 1-6, i.e. 1, 2, 3, 4, 5 or 6.

Particularly preferred examples of the spacer group Sp are also:

- $NH-CH_2-CH_2-CH_2-NH$ with -NH- being attached to the linking point L;
- $NH-(CH_2)_3-NH-$ with N being attached to the linking point L and the other N being attached to the C(=O) of the ligand;
- $N'H-CH(C(=O)NH-(CH_2)_3-N''H-)((CH_2)_2C(=O)NH-(CH_2)_3-N'''H-)$ with N' being attached to the linking point L and

both N" and N'" being attached to the C(=O) of two individual ligands;

- $NH-(CH_2)_3-N'(CH_3)-$ with N being attached to the linking point L and N' being attached to the C(=O) of the ligand;
- $N'H-(CH_2)_3-NH-C(=O)-CH(-N''H-)(-(CH_2)_4-N'''H-)$ with N' being attached to the linking point L and both N" and N'" being attached to the C(=O) of two individual ligands;
- $N'H-(CH_2)_3-NH-C(=O)-CH(-NH-C(=O)-CH_2-(O-C_2H_4)_2-N''H-)(-(CH_2)_4-NH-C(=O)-CH_2-(O-C_2H_4)_2-N'''H-)$ with N' being attached to the linking point L and both N" and N'" being attached to the C(=O) of two individual ligands;
- $N'H-(CH_2)_3-NH-C(=O)-CH(-NH-C(=O)-CH_2-O-C_2H_4-N''H-)(-(CH_2)_4-NH-C(=O)-CH_2-O-C_2H_4-N'''H-)$ with N' being attached to the linking point L and both N" and N'" being attached to the C(=O) of two individual ligands;
- $N'H-(CH_2)_3-NH-C(=O)-CH_2-(O-C_2H_4)_2-N''H-$ with N' being attached to the linking point L and N" being attached to the C(=O) of the ligand;
- $N'H-(CH_2)_3-NH-C(=O)-CH_2-(O-C_2H_4)_2-NH-C(=O)-CH_2-(O-C_2H_4)_2-N''H-$ with N' being attached to the linking point L and N" being attached to the C(=O) of the ligand;
- $N'H-(C_2H_4-O)_2-C_2H_4-NH-C(=O)-NH-C_2H_4-(O-C_2H_4)_2-N'H-$ with N' being attached to the linking point L and the other N' being attached to the C(=O) of the ligand;
- $N'H-(CH_2)_5-C(=O)-NH-(CH_2)_3-N''H-$ with N' being attached to the linking point L and N" being attached to the C(=O) of the ligand;
- $N'H-C_2H_4-(O-C_2H_4)_2-NH-C(=O)-(CH_2)_5-N''H-$ with N' being attached to the linking point L and N" being attached to the C(=O) of the ligand;
- $N'H-(CH_2)_6-NH-C(=O)-CH_2-(O-C_2H_4)_2-N''H-$ with N' being attached to the linking point L and N" being attached to the C(=O) of the ligand.

Typically, the total length of the spacer group Sp is less than 100 atoms, preferably less than 60 atoms, in particular less than 30 atoms.

In one embodiment of the invention, Sp may also be branched. The term "branched" in the present context means that more than 1 ligand of the present invention according to formula (II) is present on the spacer group Sp. This embodiment allows the linkage of more than one ligand to one matrix attachment point (linking point) L.

"Final functionality" or "connecting unit" in the present context designates a unit formed in the reaction between the precursor groups (functional group) on the support material and on the ligand of the invention and/or on the spacer group Sp. The connecting unit incorporates the bond between the support material and the ligands of the present invention (which may include the spacer Sp), resulting in the ligand-substituted matrix.

Connecting units incorporated in L and/or Sp are known to the person skilled in the art and may include the following groups: ether, thioether, C-C single bond, alkenyl, alkynyl, ester, amine, amide, carboxylic amide, sulfonic ester, sulfonic amide, phosphonic ester, phosphonic amide, phosphoric ester, phosphoric amide, phosphoric thioester, thiophosphoric ester, secondary amine, secondary alcohol, tertiary alcohol, 2-hydroxyamine, urea, isourea, imidocarbonate, alkylthiosuccinic imide, dialkylsuccinic imide, triazole, ketone, sulfoxide, sulfone, oxime ether, hydrazone, silyl ether, diacyl hydrazide, acyl sulphonyl hydrazide, N-acyl sulphonamide, hydrazide, N-alkoxy amide, acyl semicarbazide, acyloxy amide, sulphonyloxy amide, phosphoryloxy amide, phosphonyloxy amide, acyl hydrazone, N-carboxamidoisourea, N-carboxamidoimidocarbonate, N-carboxamidoisothiourea, N-carboxamidoimidothiocarbonate, acyl urea, acyl thiourea, N-carboxamidourea, N-carboxamidoisourea, alkylthioethylsulphonyl, hydrazinylsuccinimide, acylhydrazinylsuccinimide, alkoxyaminosuccinimide, aminosuccinimide, hydrazinylethylsulphonyl, acylhydrazinylethylsulphonyl, alkoxyaminoethylsulphonyl, aminoethylsulphonyl, tetrazole, alkoxyamidine.

If v in formula (I) is 0, then the ligand is directly bonded to L. In other embodiments the ligand is bonded to the support material via a spacer group Sp.

In an embodiment of the present invention, $R^1$ is selected from: hydrogen; and $C_1$ to $C_4$ alkyl, typically linear and branched $C_1$ to $C_4$ alkyl which may comprise a cycloalkyl unit such as methyl, ethyl, n-propyl, i-propyl, n-butyl, i-butyl, sec-butyl, tert-butyl, cyclopropyl, methylcyclopropyl.

Preferably $R^1$ is selected from hydrogen and methyl. More preferably, $R^1$ is hydrogen.

$Ar^1$ can be an alicyclic or heterocyclic aromatic ring; preferably $Ar^1$ is an alicyclic aromatic ring, i.e. $Ar^1$ is phenylene, for example. In an even more preferred embodiment, $Ar^1$ is phenylene. If $Ar^1$ is a heterocyclic aromatic ring it comprise one, two or more heteroatoms which are preferably selected from N, S, O and combinations thereof. Examples of appropriate 5- or 6-membered heterocylic aromatic rings include but are not restricted to pyrrole, furan, thiophene, imidazole, oxazole, isoxazole, thiazole, isothiazole, pyrazole, 1,2,3-triazole, 1,2,4-triazole (4H-1,2,4-triazole and 1H-1,2,4-triazole), 1H-tetrazole, 2H-tetrazole, pyridine, pyrimidine, pyrazine, pyridazine, 1,2,3-triazine, 1,2,4-triazine, and 1,3,5-triazine. Preferred 5- or 6-membered heterocylic aromatic rings are imidazole, thiazole, and pyridine.

In formula (I) $Ar^1$ is bonded to a C=O group and an NH group (see formula Ia) or to 2 C=O groups (see formula Ib).

In the case depicted by formula Ia, in some embodiments the C=O and NH group are in meta position to each other. If $Ar^1$ is phenylene the C=O group and the NH group are positioned meta or para, preferably meta, to each other. If the aromatic ring is imidazole, the C=O group is typically bonded in 2-position and the NH group in 4-position. If the

aromatic ring is thiazole, the C=O group is typically bonded in 4-position and the NH group in 2-position. If the aromatic ring is pyridine the C=O group and the NH group are preferably positioned meta to each other whereas the nitrogen ring atom may have different positions. In some embodiments the C=O group is typically bonded in 3-position of the pyridine ring and the NH group in 5-position. The divalent 5- or 6-membered aromatic ring including the preferred embodiments described above may be substituted or unsubstituted. Typically the 5- or 6-membered aromatic ring is substituted, preferably monosubstituted. Examples of appropriate substituents are $C_1$ to $C_4$ alkoxy, typically linear and branched $C_1$ to $C_4$ alkoxy which may comprise a cycloalkyl unit such as methoxy, ethoxy, n-propoxy, i-propoxy, n-butoxy, i-butoxy, sec-butoxy, tert-butoxy, cyclopropoxy, methylcyclopropoxy, and cyclobutoxy; $C_1$ to $C_4$ alkyl, typically linear and branched $C_1$ to $C_4$ alkyl which may comprise a cycloalkyl unit such as methyl, ethyl, n-propyl, i-propyl, n-butyl, i-butyl, sec-butyl, tert-butyl, cyclopropyl, methylcyclopropyl, and cyclobutyl; hydroxyl; and combinations thereof. Preferred substituents include methoxy, ethoxy, and methyl, with methoxy being most preferred.

In most preferred embodiments $Ar^1$ is methoxy-substituted phenylene wherein the methoxy radical is positioned ortho to the C=O group and para to the NH group (the C=O group and the NH group are positioned meta to each other). In other preferred embodiments $Ar^1$ is methoxy-substituted phenylene wherein the methoxy radical is positioned para to the C=O group and ortho to the NH group (the C=O group and the NH group are positioned meta to each other). In further preferred embodiments $Ar^1$ is an N-methyl-substituted imidazole ring wherein the C=O group is bonded in 2-position and the NH group in 4-position. In still other preferred embodiments $Ar^1$ is thiazole wherein the C=O group is bonded in 4-position and the NH group in 2-position. In yet other preferred embodiments $Ar^1$ is methyl-substituted phenylene wherein the methyl radical is positioned ortho to the C=O group and ortho to the NH group (the C=O group and the NH group are positioned meta to each other). In still other preferred embodiments $Ar^1$ is methyl-substituted phenylene wherein the methyl radical is positioned para to the C=O group and ortho to the NH group (the C=O and the NH group are positioned meta to each other). In yet other preferred embodiments $Ar^1$ is a pyridine ring wherein the C=O group is bonded in 3-position and the NH group in 5-position. In still other preferred embodiments $Ar^1$ is unsubstituted phenylene wherein the C=O and the NH group are positioned meta or para to each other.

In the case depicted by formula Ib, in some embodiments, both C=O groups are in meta position to each other. If $Ar^1$ is phenylene the both C=O groups are positioned meta or para, preferably meta, to each other.

$Ar^2$ is 5- or 6-membered unsubstituted or substituted heterocyclic aromatic ring which is

(a) attached to a further 5- or 6-membered aromatic ring via a single bond; or
(b) fused to a further 5- or 6-membered aromatic ring as part of a multicyclic ring system; or
(c) attached to at least one substituent selected from $C_1$ to $C_4$ alkyl; $C_2$ to $C_4$ alkenyl; $C_2$ to $C_4$ alkynyl; a halogen; $C_1$ to $C_4$ haloalkyl; hydroxyl-substituted $C_1$ to $C_4$ alkyl; $C_1$ to $C_4$ alkoxy; hydroxyl-substituted $C_1$ to $C_4$ alkoxy; $C_1$ to $C_4$ alkylamino; $C_1$ to $C_4$ alkylthio; and combinations thereof.

$Ar^2$ is either attached to a C=O group (see formula Ia) or to the $NR^1$ group (see formula Ib)

The 5- or 6-membered heterocyclic aromatic ring of $Ar^2$ comprises at least one heteroatom which is preferably selected from N, S, O and combinations thereof. More preferably, the 5-or 6-membered heterocyclic aromatic ring contains two or more nitrogen atoms or a nitrogen atom and an oxygen atom. A C=O group may also be part of the first aromatic ring. Typically, the 5- or 6-membered heterocyclic aromatic ring of $Ar^2$ is attached to the C=O group via a carbon ring atom which is adjacent to a ring heteroatom, preferably a nitrogen or oxygen atom. In some embodiments the 5- or 6-membered heterocyclic aromatic ring of $Ar^2$ is attached to the $NR^1$ group via a carbon ring atom which is adjacent to a ring heteroatom, preferably a nitrogen, oxygen or sulfur atom.

Examples of appropriate 5- or 6-membered heterocyclic aromatic rings of $Ar^2$ and directly bonded to the C=O group or directly bonded to the $NR^1$ group in formula (I) include pyrrole, furan, thiophene, imidazole, oxazole, isoxazole, thiazole, isothiazole, pyrazole, 1,2,3-triazole, 1,2,4-triazole (4H-1,2,4-triazole and 1H-1,2,4-triazole), 1H-tetrazole, 2H-tetrazole, pyridine, pyrimidine, pyrazine, pyridazine, 1,2,3-triazine, 1,2,4-triazine, 1,3,5-triazine, 1,2,4 oxadiazole, 1,3,4 oxadiazole, 1,2,4-thiadiazole and 1,3,4-thiadiazole. Preferred 5- or 6-membered heterocylic aromatic rings are pyrazole, imidazole, isoxazole, pyrrole, furan, 1,2,4-triazole, pyridine, 1,2,4-triazine, pyridazine, pyrimidine, 1,2,4-oxadiazole, 1,3,4-oxadiazole thiazole and 1,3,4-thiadiazole, with pyrazole, imidazole, isoxazole, 1,2,4-oxadiazole, 1,3,4 oxadiazole, thiazole and 1,3,4-thiadiazole being most preferred. In case of a pyrazole ring the C=O group is typically bonded to the pyrazole ring in 3-position, 4-position or 5-position, preferably in 3-position. In case of a pyrazole ring the N atom of the $NR^1$-group is typically bonded to the pyrazole ring in 3-position, 4-position or 5-position, preferably in 3-position. In case of an imidazole ring the C=O group is typically bonded to the imidazole ring in 4-position or 2-position. In case of an isoxazole ring the C=O group is typically bonded to the isoxazole ring in 5-position or 3-position. In case of a pyrrole ring the C=O group is typically bonded to the pyrrole ring in 3-position. In case of a furan ring the C=O group is typically bonded to the furan ring in 2-position. In case of a 1,2,4-triazole ring the C=O group is typically bonded to the 1,2,4-triazole ring in 3-position. In case of a pyridine ring the C=O group is typically bonded to the pyridine ring in 3-position or 2-position, preferably in 2- and 3-position. In case of a 1,2,4-triazine ring the C=O group is typically bonded to the

1,2,4-triazine ring in 3-position. In case of a pyridazine ring the C=O group is typically bonded to the pyridazine ring in 4-position. In case of a 1,2,4-oxadiazole ring the C=O group is typically bonded to the 1,2,4-oxadiazole ring in 5-position. In case of a 1,3,4-oxadiazole ring the C=O group is typically bonded to the 1,3,4-oxadiazole ring in 2-or 5-position. In case of a thiazole ring the N atom of the $NR^1$-group is typically bonded to the thiazole ring in 2-position. In case of a 1,3,4 thiadiazole ring the N atome of the $NR^1$-group is typically bonded to the 1,3,4 thiadiazole ring in 2-position.

In alternative (a) of $Ar^2$ described above for formula (I) the 5- or 6-membered heterocylic aromatic ring is attached to a further 5- or 6-membered aromatic ring ("second aromatic ring of $Ar^2$" in the following) via a single bond. In these embodiments, it is especially preferred that the 5- or 6-membered heterocyclic aromatic ring directly bonded to the C=O group ("first aromatic ring of $Ar^2$" in the following) is pyrazole, pyridine, isoxazole, 1,2,4-oxadiazole or 1,3,4-oxadiazole ring. In case of a pyrazole ring as first aromatic ring, the C=O group is typically bonded to the pyrazole ring in 3-position. The second aromatic ring is preferably bonded to the pyrazole ring in 5-position. In other embodiments the C=O group is bonded to the pyrazole ring in 5-position and the second aromatic ring is bonded to the pyrazole ring in 3-position. If the first aromatic ring is pyridine the C=O group and the second aromatic ring are preferably positioned meta to each other whereas the nitrogen ring atom may have different positions. In some embodiments the C=O group is bonded to the pyridine ring in 3-position and the second aromatic ring is bonded to the pyridine ring in 5-position. In other embodiments the C=O group is bonded to the pyridine ring in 2-position and the second aromatic ring is bonded to the pyridine ring in 4-position. In case of an isoxazole ring as first aromatic ring, the C=O group is typically bonded to the isoxazole ring in 3-position. The second aromatic ring is preferably bonded to the isoxazole ring in 5-postion. In other embodiments the C=O group is bonded to the isoxazole ring in 5-position and the second aromatic ring is bonded to the isoxazole ring in 3-position. In case of a 1,2,4-oxadiazole ring as first aromatic ring, the C=O group is typically bonded to the 1,2,4-oxadiazole ring in 5-position. The second aromatic ring is preferably bonded to the 1,2,4-oxadiazole ring in 3-postion. In case of a 1,3,4-oxadiazole ring as first aromatic ring, the C=O group is typically bonded to the 1,3,4-oxadiazole ring in 2 position. The second aromatic ring is preferably bonded to the 1,3,4-oxadiazole ring in 5-postion. In some embodiments, it is especially preferred that the 5- or 6-membered heterocyclic aromatic ring directly bonded to the N atom of the of the $NR^1$-group ("first aromatic ring of $Ar^2$" in the following) is pyrazole, thiazole, 1,3,4 thiadiazole ring. In case of a pyrazole ring as first aromatic ring, the N atom of the $NR^1$-group is typically bonded to the pyrazole ring in 5-position. The second aromatic ring is preferably bonded to the pyrazole ring in 3-position. If the first aromatic ring is thiazole ring the N atom of the $NR^1$-group is typically bonded to the thiazole ring in 2-position. The second aromatic ring is preferably bonded to the thiazole ring in 4-postion. In other embodiments the second aromatic ring is preferably bonded to the thiazole ring in 5-postion. In case of a 1,3,4 thiadiazole ring as first aromatic ring, the N atom of the of the $NR^1$-group is typically bonded to the 1,3,4 thiadiazole ring in 2-position. The second aromatic ring is preferably bonded to the 1,3,4 thiadiazole ring in 5-postion.

In alternative (b) the first aromatic ring is fused to a second 5- or 6-membered aromatic ring as part of a multicyclic, preferably bicyclic ring system. In case of a pyrazole ring as first aromatic ring and being part of a fused ring system, the C=O group is typically bonded to the pyrazole ring in 3-position. The pyrazole ring is preferably fused to the second aromatic ring via the 4- and 5-position. In other cases the C=O group is bonded to the pyrazole ring in 4-position and the second aromatic ring is bonded to the pyrazole ring via the 1- and 5-position.

In other cases the C=O group is bonded to the pyrazole ring in 3-position and the second aromatic ring is bonded to the pyrazole ring via the 1- and 5-position. In case of an isoxazole ring as first aromatic ring and being part of a fused ring system, the C=O group is typically bonded to the isoxazole ring in 5-position. The isoxazole ring is preferably fused to the second aromatic ring via the 3- and 4-position. In case of an imidazole ring as first aromatic ring and being part of a fused ring system, the C=O group is typically bonded to the imidazole ring in 4-position. The imidazole ring is preferably fused to the second aromatic ring via the 1- and 2-position. In other cases the C=O group is bonded to the imidazole ring in 2-position and second aromatic ring is fused to the imidazole ring via 4- and 5-position. In case of a 1,2,4-triazole ring as first aromatic ring and being part of a fused ring system, the C=O group is typically bonded to the 1,2,4-triazole ring in 3-position. The 1,2,4-triazole ring is preferably fused to the second aromatic ring via the 4- and 5-position. In case of a pyridazine as first aromatic ring and being part of a fused ring system, the C=O group is typically bonded to the pyridazine ring in 4-position. The pyridazine ring is preferably fused to the second aromatic ring via the 5- and 6-position. In case of a pyrrole ring as first aromatic ring and being part of a fused ring system, the C=O group is typically bonded to the pyrrole ring in 3-position. The pyrrole ring is preferably fused to the second aromatic ring via the 4- and 5-position. In case of a pyridine ring as first aromatic ring and being part of a fused ring system, the C=O group is typically bonded to the pyridine ring in 2-position. The pyridine ring is preferably fused to the second aromatic ring via the 3- and 4-position. In case of a 1,2,4-triazine ring as first aromatic ring and being part of a fused ring system, the C=O group is typically bonded to the 1,2,4-triazine ring in 6-position. The 1,2,4-triazine ring is preferably fused to the second aromatic ring via the 3- and 4-position. In case of a pyrazole ring as first aromatic ring and being part of a fused ring system, the N atom of the $NR^1$-group is typically bonded to the pyrazole ring in 3-position. The pyrazole ring is preferably fused to the second aromatic ring via the 4- and 5-position. In case of an imidazole ring as first aromatic ring and being part of a fused ring system, the N atom of the $NR^1$-group is typically bonded to the imidazole ring in 4-position. The imidazole ring is preferably fused to the second aromatic ring via the 1- and 2-position. In case of a thiazole ring as first aromatic ring and being part of a fused ring system, the N atom of the $NR^1$-group is typically bonded to the thiazole ring in 2-position. The thiazole ring is preferably fused to the second aromatic ring via the 4- and 5-position.

The second aromatic ring of $Ar^2$ in both alternatives (a) and (b) and including the preferred embodiments described above is typically a 5- oder 6-membered alicyclic or heterocyclic ring. Examples of appropriate 5- or 6-membered aromatic rings include but are not restricted to benzene, pyrrole, furan, thiophene, imidazole, oxazole, isoxazole, thiazole, isothiazole, pyrazole, 1,2,3-triazole, 1,2,4-triazole (4H-1,2,4-triazole and 1H-1,2,4-triazole), 1H-tetrazole, 2H-tetrazole, pyridine, pyrimidine, pyrazine, pyridazine, 1,2,3-triazine, 1,2,4-triazine, and 1,3,5-triazine. Preferred second aromatic rings are benzene, furan, thiophene, pyrrole, pyridine, thiazole, pyrimidine, and pyrazole, with benzene, thiazole, furan and thiophene being most preferred.

In alternative (a) the second aromatic ring of $Ar^2$ is typically furan, thiophene, pyrrole, benzene, pyridine or thiazole, with furan, thiophene, benzene and thiazole being preferred and furan and thiazole being most preferred. In case of a furan, thiophene, pyrrole, benzene or thiazole ring as second aromatic ring in alternative (a) those rings are preferably bonded to the first aromatic ring via their 2-, 3- or 4-position, with the 2- and 4-position being most preferred. In a typical embodiment of alternative (a) the first aromatic ring is a pyrazole ring and the second aromatic ring is a furan ring, i.e. $Ar^2$ is furylpyrazyl such as 5-(2-furyl)pyrazol-3-yl. In further typical embodiments, $Ar^2$ is 5-(2-methylthiazol-4-yl)isoxazol-3-yl, 4-(2-furyl)pyridin-2-yl, 3-(4-methoxyphenyl)-[1,2,4]oxadiazol-5-yl, 3-(2-furyl)-[1,2,4]oxadiazol-5-yl, or 5-(2-furyl)-[1,3,4]oxadiazol-2-yl.

In alternative (b) the second aromatic ring of $Ar^2$ is preferably benzene, thiazole, pyrrole, pyrimidine or pyridine, with benzene and thiazole being most preferred. In typical embodiments of alternative (b) $Ar^2$ is benzo[c]isoxazol-3-yl, imidazo[2,1-b]thiazol-6-yl, cinnolin-4-yl, indol-3-yl, indazol-3-yl, benzo[c]furan-1-yl, 1-isoquinolinyl, 5-oxo-5H-[1,3]thiazol[3,2-a]pyrimidin-6-yl, pyrazolo[1,5-a]pyrimidin-3-yl, [1,2,4]triazolo[4,3-a]pyrimidin-3-yl, and pyrazolo[5,1-c][1,2,4]triazin-3-yl, with benzo[c]isoxazol-3-yl, indazol-3-yl and imidazo[2,1-b]thiazol-6-yl being preferred.

In alternative (c) of $Ar^2$ described above for formula (I) the 5- or 6-membered heterocylic aromatic ring is attached to at least one (preferably one or two) substituent selected from $C_1$ to $C_4$ alkyl, typically linear or branched $C_1$ to $C_4$ alkyl which may comprise a cycloalkyl unit such as methyl, ethyl, n-propyl, i-propyl, n-butyl, i-butyl, sec-butyl, tert-butyl, cyclopropyl, methylcyclopropyl, and cyclobutyl; $C_2$ to $C_4$ alkenyl; $C_2$ to $C_4$ alkynyl such as ethynyl; a halogen such as fluoro, chloro, bromo, and iodo; $C_1$ to $C_4$ haloalkyl such as trifluoromethyl; hydroxyl-substituted $C_1$ to $C_4$ alkyl, typically mono- or dihydroxyalkyl such as mono- or dihydroxyethyl or mono- or dihydroxypropyl; $C_1$ to $C_4$ alkoxy, typically linear and branched $C_1$ to $C_4$ alkoxy which may comprise a cycloalkyl unit such as methoxy, ethoxy, n-propoxy, i-propoxy, n-butoxy, i-butoxy, sec-butoxy, tert-butoxy, cyclopropoxy, methylcyclopropoxy, and cyclobutoxy; hydroxyl-substituted $C_1$ to $C_4$ alkoxy, typically monohydroxyl-substituted $C_1$ to $C_4$ alkoxy; $C_1$ to $C_4$ alkylamino; $C_1$ to $C_4$ alkylthio, and combinations thereof. The substituent(s) may be bonded to the ring via ring carbon and/or ring heteroatoms, preferably via carbon and/or nitrogen ring atoms. Preferred substituents of the 5- or 6-membered heterocylic aromatic ring in alternative (c) are cyclopropyl, ethynyl, methyl, and trifluoromethyl. The at least one substituent is preferably positioned meta to the C=O group. In typical embodiments of alternative (c) $Ar^2$ is 3-cyclopropylpyridin-5-yl, 3-ethynylpyridin-5-yl, 5-(trifluoromethyl)pyrazol-3-yl, 5-(trifluoromethyl)pyrazol-3-yl, or 1-methyl-5-(trifluoromethyl)pyrazol-3-yl.

It is understood that the aromatic rings of $Ar^2$, i.e. the first aromatic ring or the second aromatic ring or both, in alternative (a) and (b) may optionally carry one or more further substituents which are typically selected from the substituents mentioned above for alternative (c). The substituent(s) may be bonded to the ring(s) via ring carbon or ring heteroatoms, preferably via carbon or nitrogen atoms. Preferred substituents of the first and/or second aromatic ring of $Ar^2$ are $C_1$ to $C_4$ alkyl such as methyl; halo such as bromo and chloro; hydroxoalkyl such as methoxy, 2-hydroxyethyl or 2,3-dihydroxypropyl, alkylamino such as aminopropyl and haloalkyl such as trifluoromethyl. If the first aromatic ring is a pyrazole ring it is typically methyl-substituted, preferably N-methyl-substituted, more preferably in 1-position. N-Methyl substitution of the pyrazole is especially preferred in alternative (a).

A typical example of a substituted $Ar^2$ in alternative (a) is 5-(2-furyl)-1-methylpyrazol-3-yl.

If the first aromatic ring is a pyridine ring it typically does not carry further substituents.

Typical examples of substituted $Ar^2$ in alternative (b) are 5-chloro-1H-indazol-3-yl, 1-methylindol-3-yl 1-methylindazol-3-yl 5,7-dimethyl-[1,2,4]triazolo[4,3-alpyrimidin-3-yl 4,7-dimethylpyrazolo[5,1-c][1,2,4]triazin-3-yl, 1-ethylindazol-3-yl, 1-(2-hydroxyethyl)indazol-3-yl and 1-(2,3-dihydroxyproyl)indazol-3-yl.

It is also encompassed by the present invention that the second aromatic ring in both alternatives (a) and (b) may optionally be fused to a further aromatic ring such as benzene. A typically example of a second aromatic ring fused to a further aromatic ring is benzothiophene.

In a preferred embodiment of the present invention, the group in alpha position to the carbonyl function is an amino group $NR^2$, wherein $R^2$ can have various meanings as defined below. The ligand-substituted matrix of the invention, in these cases, can be depicted as in the following figures (II), (IIa) and (IIb) which are covered by the above figures (I), (Ia) and (Ib).

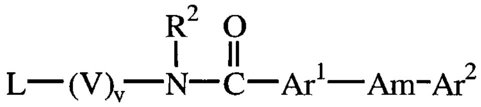

(II)

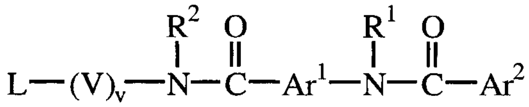

(IIa)

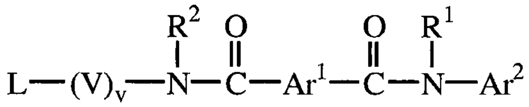

(IIb)

In the above figures (II), (IIa) and (IIb), the $NR^2$ group is not shown as a part of the linking point L or the spacer group Sp, but as a part of the ligand as such, for clarity reasons. It has to be kept in mind, however, that $NR^2$ together with V forms the spacer group Sp as shown in figures (I), (Ia) and (Ib), or is a part of the linking point L.

In an embodiment of the present invention, $R^2$ is selected from: hydrogen; and $C_1$ to $C_4$ alkyl, typically linear and branched $C_1$ to $C_4$ alkyl which may comprise a cycloalkyl unit such as methyl, ethyl, n-propyl, i-propyl, n-butyl, i-butyl, sec-butyl, tert-butyl, cyclopropyl, methylcyclopropyl.

Preferably $R^2$ is selected from hydrogen and methyl. More preferably, $R^1$ is hydrogen.

The other symbols $Ar^1$, $Ar^2$ and Am have the meanings and preferred meanings as defined above in the context of the figures (I), (Ia) and (Ib).

$R^2$ as defined in the context with formula (II) and as shown in formulae (Ha) and (IIb) corresponds to $R^3$ as defined and shown in the patent application EP 10008089.4 of which the present application claims priority.

The present invention also includes the ligands of the present invention (which, after being attached to the support material) form together with the support material the ligand-substituted matrix which is used in the method of the present invention. The ligands are in accordance with the following formulae wherein the symbols Sp, $Ar^1$, $Ar^2$ and Am have the meanings defined above, including the preferred meanings:

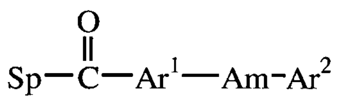

(III)

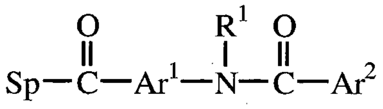

(IIIa)

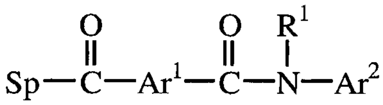

(IIIb)

The ligands according to formulaa (III), (IIIa) and (IIIb) comprise the spacer group Sp attached to the ligand.

The ligands according to formulae (III), (IIIa) and (IIIb) can serve as precursors for the synthesis of further compounds not having the spacer group attached. The named compounds are generated after cleavage of the spacer group and, as the case may be, conversion of the - C=O containing functionality present in the part of the molecule which binds to antibodies, to any other appropriate functionality known to the person skilled in the art. Appropriate reactions to that end are known to person skilled in the art. The resulting compounds are also included in the present invention.

Preferred ligands of the matrix according to the present invention are depicted below wherein in each formula the very left N atom is connected to the linking point L which is not shown. All formulae below show the spacer group attached to the ligands:

5-[5-(2-Furyl)-1-methylpyrazol-3-yl]carboxamido-2-methoxybenzoic acid N-(3-aminopropyl)amide (L1)

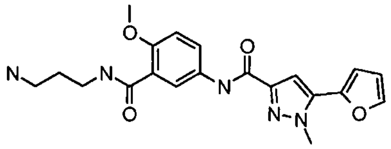

5-[5-(4-Bromo-2-thienyl)-1-methylpyrazol-3-yl]carboxamido-2-methoxybenzoic acid N-(3-aminopropyl)amide (L2)

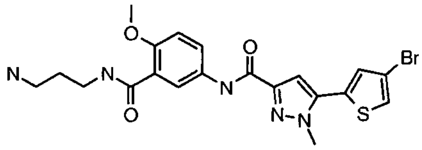

5-[5-(2,5-Dimethyl-3-thienyl)-1-methylpyrazol-3-yl]carboxamido-2-methoxybenzoic acid N-(3-aminopropyl)amide (L3)

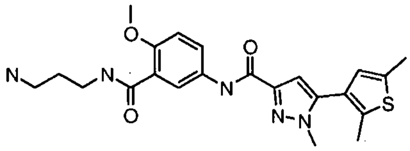

5-((5-Chloro-1H-indazol-3-yl)carboxamido-2-methoxybenzoic acid N-(3-aminopropyl)amide (L4)

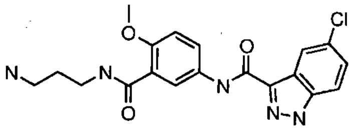

2-Methoxy-5-[3-(2-thienyl)pyridin-5-yl]carboxamidobenzoic acid N-(3-aminopropyl)amide (L5)

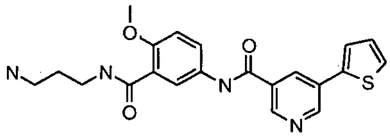

2-Methoxy-5-[1-methyl-5-(2-thienyl)pyrazol-3-yl]carboxamidobenzoic acid N-(3-aminopropyl)amide (L6)

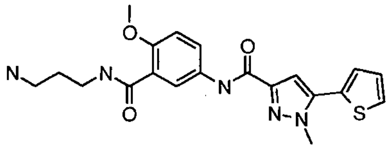

5-[5-(3-Chlorophenyl)-1-methylpyrazol-3-yl]carboxamido-2-methoxybenzoic acid N-(3-aminopropyl)amide (L7)

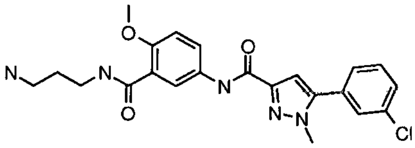

2-Methoxy-5-[1-methyl-5-(3-thienyl)pyrazol-3-yl]carboxamidobenzoic acid N-(3-aminopropyl)amide (L8)

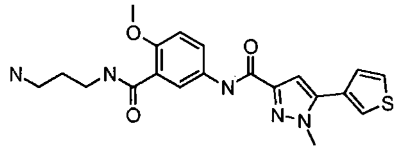

2-Methyl-3-[1-methyl-5-(2-thienyl)pyrazol-3-yl]carboxamidobenzoic acid N-(3-aminopropyl)amide (L9)

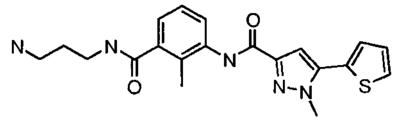

4-Methyl-3-[1-methyl-5-(2-thienyl)pyrazol-3-yl]carboxamidobenzoic acid N-(3-aminopropyl)amide (L10)

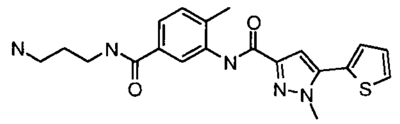

2-Methoxy-5-(3-phenylpyridin-5-yl)carboxamidobenzoic acid N-(3-aminopropyl)amide (L11)

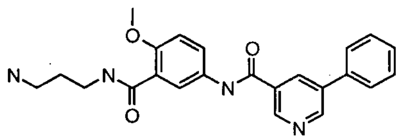

2-Methoxy-5-{3-[4-(trifluoromethyl)phenyl]pyridin-5-yl}carboxamidobenzoic acid N-(3-aminopropyl)amide (L12)

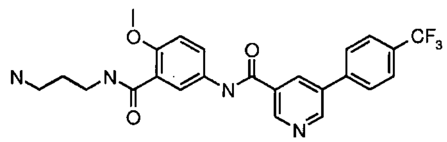

5-[3-(2-Furyl)pyridin-5-yl]carboxamido-2-methoxybenzoic acid N-(3-aminopropyl)amide (L13)

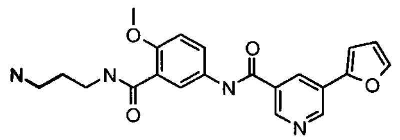

5-[3-(2-Benzothienyl)pyridin-5-yl]carboxamido-2-methoxybenzoic acid N-(3-aminopropyl)amide (L14)

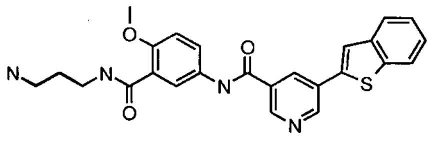

2-Methoxy-5-(1-methyl-5-phenylpyrazol-3-yl)carboxamidobenzoic acid N-(3-aminopropyl)amide (L15)

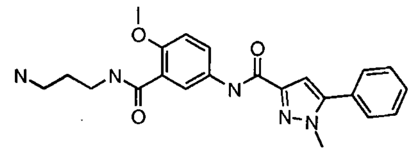

2-Methoxy-5-[5-(1-methyl-2-pyrrolyl)-1-methylpyrazol-3-yl]carboxamidobenzoic acid N-(3-aminopropyl)amide (L16)

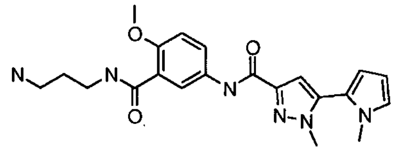

3-[5-(2-Furyl)-1-methylpyrazol-3-yl]carboxamidobenzoic acid N-(3-aminopropyl)amide (L17)

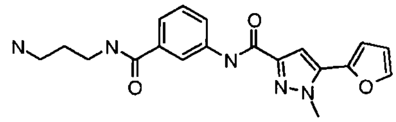

4-[5-(2-Furyl)-1-methylpyrazol-3-yl]carboxamidobenzoic acid N-(3-aminopropyl)amide (L18)

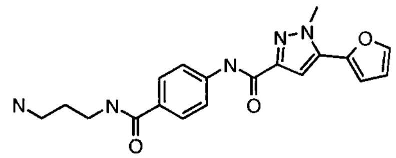

2-[1-Methyl-5-(2-thienyl)pyrazol-3-yl]carboxamidothiazole-4-carboxylic acid N-(3-aminopropyl)amide (L19)

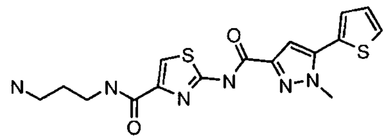

5-[5-(2-Furyl)pyrazol-3-yl]carboxamido-2-methoxybenzoic acid N-(3-aminopropyl)amide (L20)

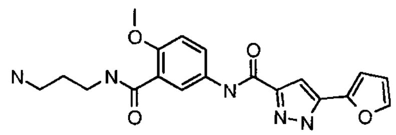

3-{N-[5-(2-Furyl)pyrazol-3-yl]carbamoyl}benzoic acid *N*'-(3-aminopropyl)amide (L21)

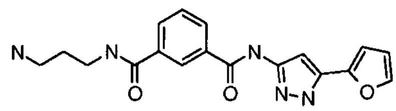

5-[3-(3-Furyl)pyridin-5-yl]carboxamido-2-methoxybenzoic acid N-(3-aminopropyl)amide (L22)

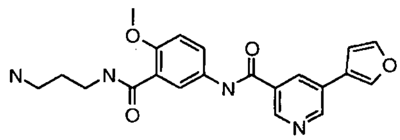

5-((3-Cyclopropylpyridin-5-yl)carboxamido-2-methoxybenzoic acid N-(3-aminopropyl)amide (L23)

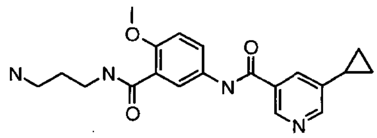

5-((3-Ethynylpyridin-5-yl)carboxamido-2-methoxybenzoic acid N-(3-aminopropyl)amide (L24)

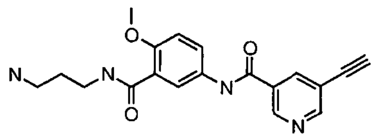

2-Methoxy-5-(1-methylindol-3-yl)carboxamidobenzoic acid N-(3-aminopropyl)amide (L25)

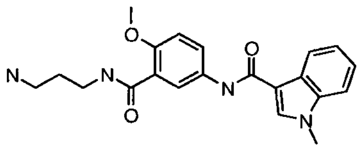

5-((Indol-3-yl)carboxamido-2-methoxybenzoic acid N-(3-aminopropyl)amide (L26)

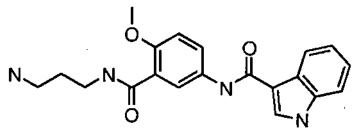

2-Methoxy-5-(1-methylindazol-3-yl)carboxamidobenzoic acid N-(3-aminopropyl)amide (L27)

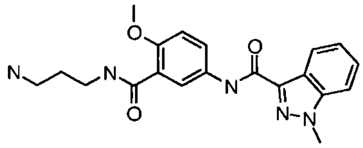

5-[5-(2-Furyl)-1-methylpyrazol-3-yl]carboxamido-2-methoxybenzoic acid N-(3-aminopropyl)-N-methylamide (L28)

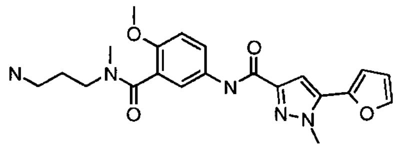

5-[5-(2-Furyl)-1-methylpyrazol-3-yl]carboxamido-2-ethoxybenzoic acid N-(3-aminopropyl)amide (L29)

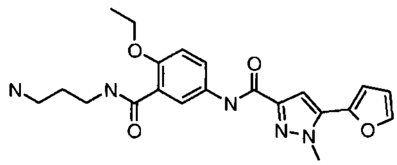

5-[5-(2-Furyl)-1-methylpyrazol-3-yl]carboxamido-2-hydroxybenzoic acid N-(3-aminopropyl)amide (L30)

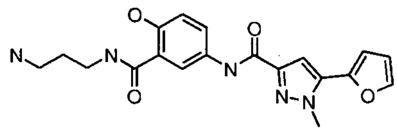

5-((Benzo[c]isoxazol-3-yl)carboxamido-2-methoxybenzoic acid N-(3-aminopropyl)amide (L31)

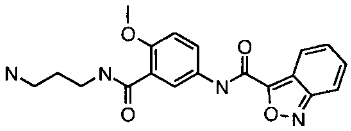

5-((Benzo[c]furan-1-yl)carboxamido-2-methoxybenzoic acid N-(3-aminopropyl)amide (L32)

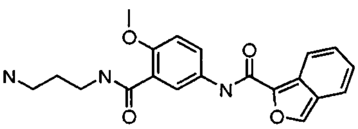

5-((1,5-Dimethylpyrazol-3-yl)carboxamido-2-methoxybenzoic acid N-(3-aminopropyl)amide (L33)

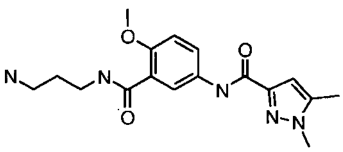

2-Methoxy-5-[1-methyl-5-(trifluoromethyl)pyrazol-3-yl]carboxamidobenzoic acid N-(3-aminopropyl)amide (L34)

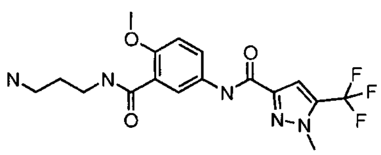

5-((1-Isoquinolinyl)carboxamido-2-methoxybenzoic acid N-(3-aminopropyl)amide (L35)

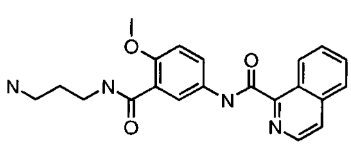

5-((Indazol-3-yl)carboxamido-2-methoxybenzoic acid N-(3-aminopropyl)amide (L36)

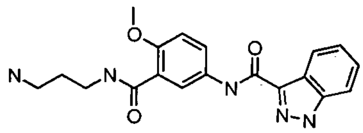

5-((Imidazo[2,1-b]thiazol-6-yl)carboxamido-2-methoxybenzoic acid N-(3-aminopropyl)amide (L37)

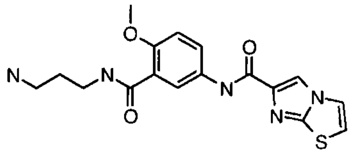

5-((1-Ethylindazol-3-ylcarboxamido)-2-methoxybenzoic acid (3-amino-1-propyl)amide (L38)

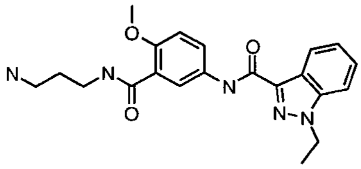

5-[N-(1-Methylindazol-3-yl)carbamoyl]-2-methoxybenzoic acid (3-amino-1-propyl)amide (L39)

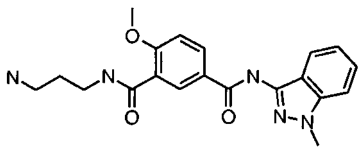

(RS)-5-[1-(2,3-Dihydroxy-1-propyl)indazol-3-ylcarboxamido]-2-methoxybenzoic acid (3-amino-1-propyl)amide (L40)

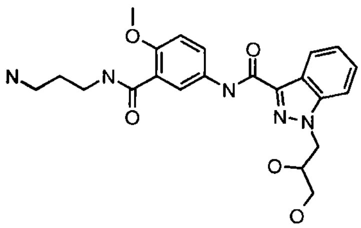

5-((3H-Imidazo[4,5-b]pyridin-2-ylcarboxamido)-2-methoxybenzoic acid (3-amino-1-propyl)amide (L41)

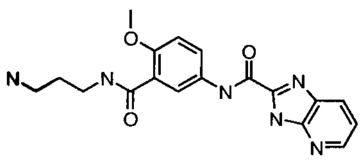

2-Methoxy-5-[5-(2-methylthiazol-4-yl)isoxazol-3-ylcarboxamido]benzoic acid (3-amino-1-propyl)amide (L42)

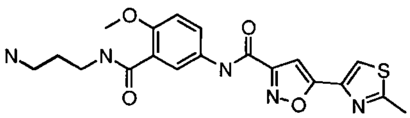

2-Methoxy-5-([1,2,4]triazolo[4,3-a]pyridin-3-ylcarboxamido)benzoic acid (3-amino-1-propyl)amide (L43)

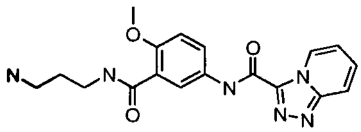

2-Methoxy-5-(1-methylpyrazolo[3,4-b]pyridin-3-ylcarboxamido)benzoic acid (3-amino-1-propyl)amide (L44)

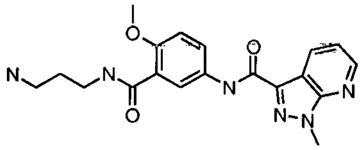

5-[5-(2-Furyl)isoxazol-3-ylcarboxamido]-2-methoxybenzoic acid (3-amino-1-propyl)amide (L45)

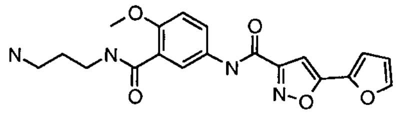

2-Methoxy-5-(pyrazolo[1,5-a]pyridin-2-ylcarboxamido)benzoic acid (3-amino-1-propyl)amide (L46)

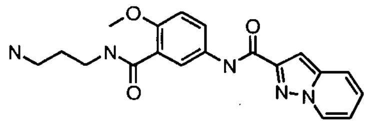

5-((1H-Benzimidazol-2-ylcarboxamido)-2-methoxybenzoic acid (3-amino-1-propyl)amide (L47)

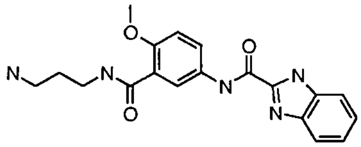

5-((Imidazo[1,2-b]pyridazin-2-ylcarboxamido)-2-methoxybenzoic acid (3-amino-1-propyl)amide (L48)

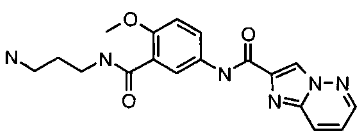

(S)-2,6-Bis[5-(Imidazo[2,1-b]thiazol-6-ylcarboxamido)-2-methoxyphenylcarboxamido]hexanoic acid (3-amino-1-propyl)amide (L49)

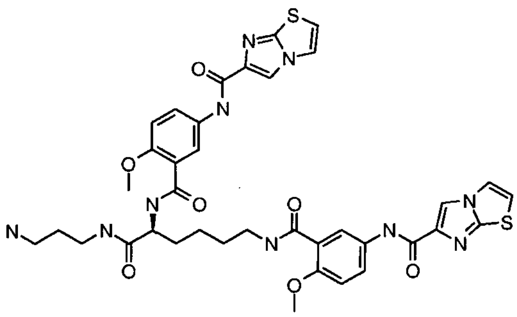

(S)-2,6-Bis{8-[5-(Imidazo[2,1-b]thiazol-6-ylcarboxamido)-2-methoxyphenylcarboxamido]-3,6-dioxaoctanoylamido}hexanoic acid (3-amino-1-propyl)amide (L50)

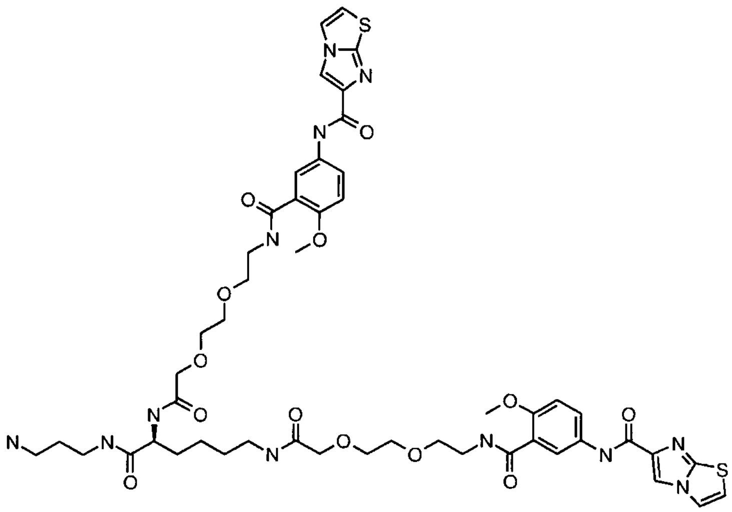

2-Methoxy-5-(3-phenylisoxazol-5-ylcarboxamido)benzoic acid (3-amino-1-propyl)amide (L51)

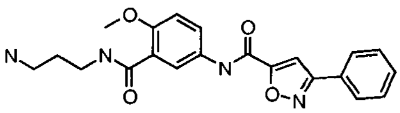

5-[4-(2-Furyl)pyridin-2-ylcarboxamido]-2-methoxybenzoic acid (3-amino-1-propyl)amide (L52)

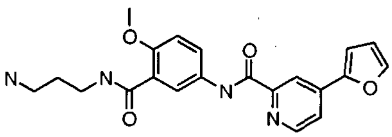

(S)-2,6-Bis{5-[5-(Imidazo[2,1-b]thiazol-6-ylcarboxamido)-2-methoxyphenylcarboxamido]-3-oxapentanoylamido}hexanoic acid (3-amino-1-propyl)amide (L53)

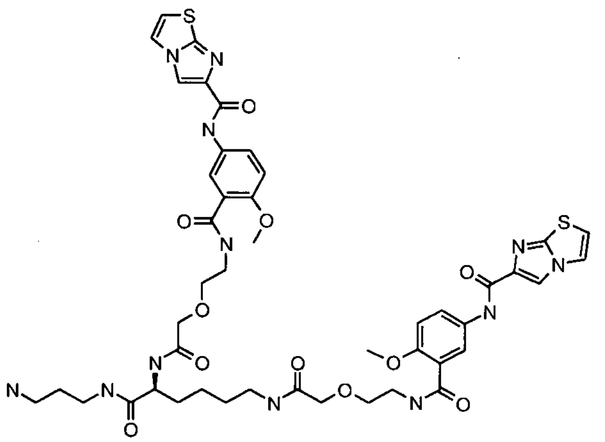

2-Methoxy-5-[3-(4-methoxyphenyl)-1,2,4-oxadiazol-5-ylcarboxamido]benzoic acid (3-amino-1-propyl)amide (L54)

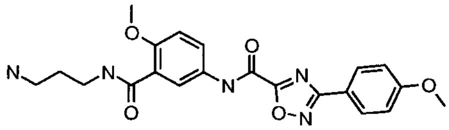

5-[3-(2-Furyl)-1,2,4-oxadiazol-5-ylcarboxamido]-2-methoxybenzoic acid (3-amino-1-propyl)amide (L55)

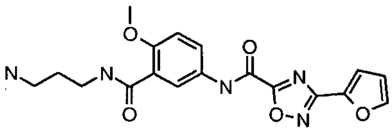

2-Methoxy-5-(pyrazolo[1,5-a]pyrimidin-2-ylcarboxamido)benzoic acid (3-amino-1-propyl)amide (L56)

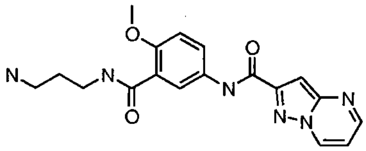

5-((Imidazo[1,2-a]pyridin-2-ylcarboxamido)-2-methoxybenzoic acid (3-amino-1-propyl)amide (L57)

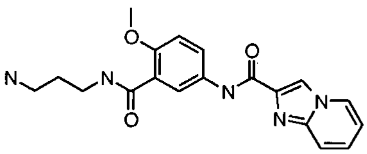

5-[5-(2-Furyl)-1,3,4-oxadiazol-2-yl]carboxamido-2-methoxybenzoic acid (3-amino-1-propyl)amide (L58)

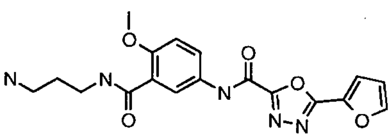

5-[1-(2-Hydroxyethyl)indazol-3-ylcarboxamido]-2-methoxybenzoic acid(3-amino-1-propyl)amide (L59)

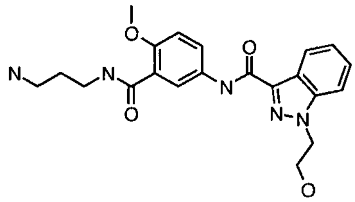

8-[2-Methoxy-5-(1-methylindazol-3-ylcarboxamido)phenylcarboxamido]-3,6-dioxaoctanoic acid (3-amino-1-propyl)amide (L60)

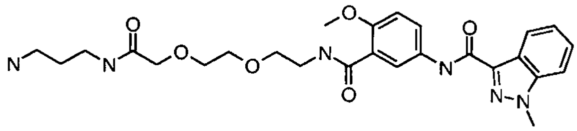

8-{8-[2-Methoxy-5-(1-methylindazol-3-ylcarboxamido)phenylcarboxamido]-3,6-dioxaoctanoylamido}-3,6-dioxaoctanoic acid (3-amino-1-propyl)amide (L61)

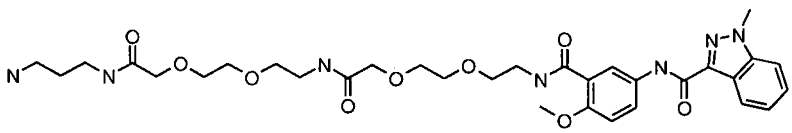

(S)-2,6-Bis {8-[5-(Imidazo[1,2-b]pyridazin-2-ylcarboxamido)-2-methoxyphenylcarboxamido]-3,6-dioxaoctanoylamido}hexanoic acid (3-amino-1-propyl)amide (L62)

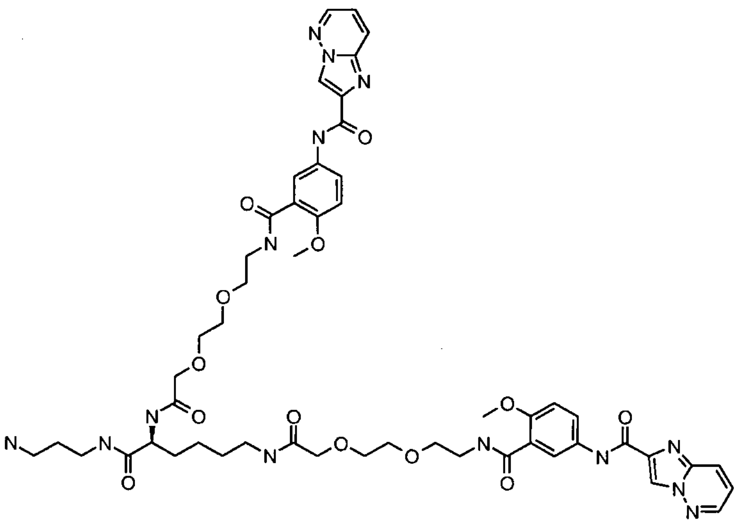

5-((Imidazo[1,2-a]pyrimidin-2-ylcarboxamido)-2-methoxybenzoic acid (3-amino-1-propyl)amide (L63)

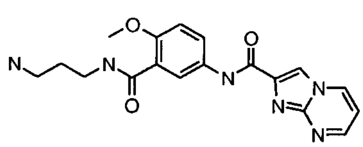

(S)-1-Aminopropane-1,3-dicarboxylic acid bis{3-[5-(imidazo[2,1-b]thiazol-6-ylcarboxamido)-2-methoxyphenylcarboxamido]propyl}amide (L64)

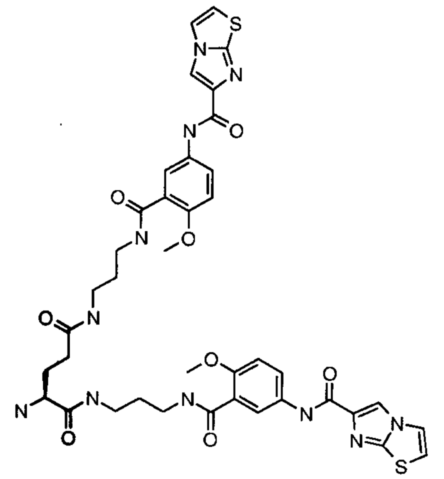

8-{8-{5-[5-(2-Furyl)-1-methylpyrazol-3-ylcarboxamido]-2-methoxyphenylcarboxamido}-3,6-dioxaoctanoylamido}-3,6-dioxaoctanoic acid (3-amino-1-propyl)amide (L65)

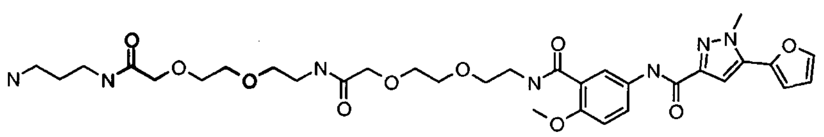

8-{8-[5-(Imidazo[2,1-b]thiazol-6-ylcarboxamido)-2-methoxyphenylcarboxamido]-3,6-dioxaoctanoylamido}-3,6-dioxaoctanoic acid (3-amino-1-propyl)amide (L66)

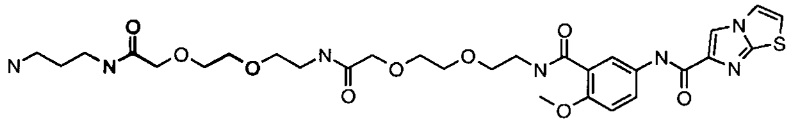

8-{8-{5-[5-(2-Furyl)-1,3,4-oxadiazol-2-ylcarboxamido]-2-methoxyphenylcarboxamido}-3,6-dioxaoctanoylamido}-3,6-dioxaoctanoic acid (3-amino-1-propyl)amide (L67)

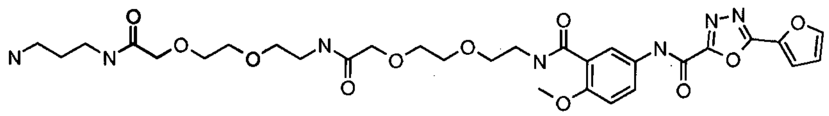

8-{8-{2-Methoxy-5-[5-(2-methylthiazol-4-yl)isoxazol-3-ylcarboxamido]phenylcarboxamido}-3,6-dioxaoctanoylamido}-3,6-dioxaoctanoic acid (3-amino-1-propyl)amide (L68)

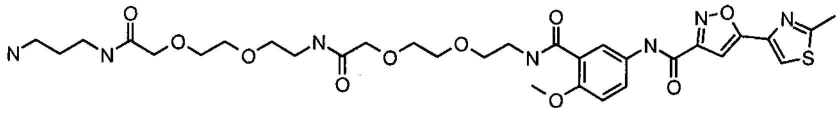

N-(8-Amino-3,6-dioxaoctyl)-*N'*-{8-[5-(imidazo[2,1-b]thiazol-6-ylcarboxamido)-2-methoxyphenylcarboxamido]-3,6-dioxaoctyl}urea (L69)

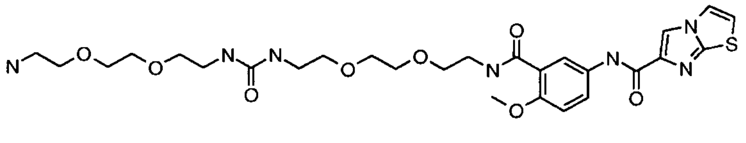

Isophthalic acid N-(3-aminopropyl)-N'-[5-(2-furyl)-1,3,4-thiadiazol-2-yl]amide (L70)

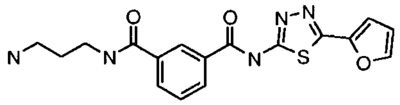

Isophthalic acid N-(3-aminopropyl)-N'-(1-methylindazol-3-yl)amide (L71)

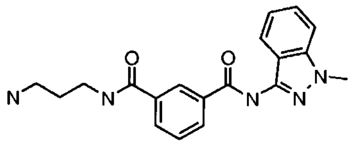

Isophthalic acid N-(3-aminopropyl)-N'-(benzothiazol-2-yl)amide (L72)

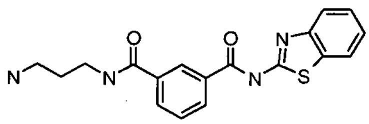

Isophthalic acid N-(3-aminopropyl)-N'-(4-phenylthiazol-2-yl)amide (L73)

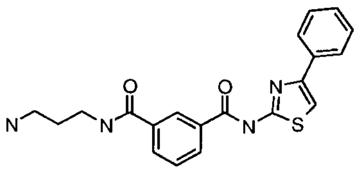

Isophthalic acid N-(3-aminopropyl)-N'-(5-phenylthiazol-2-yl)amide (L74)

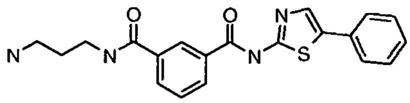

5-((Imidazo[2,1-b]thiazol-6-ylcarboxamido)-2-methoxybenzoic acid [3-(6-aminohexanoylamido)-1-propyl]amide (L75)

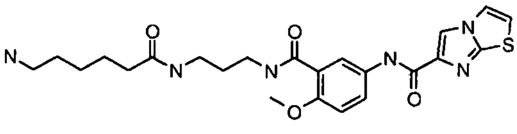

6-[5-(Imidazo[2,1-b]thiazol-6-ylcarboxamido)-2-methoxyphenylcarboxamido]hexanoic acid (8-amino-3,6-dioxa-1-octyl)amide (L76)

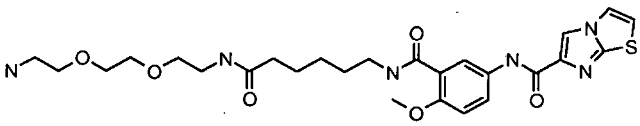

8-[5-(Imidazo-[2,1-b]thiazol-6-ylcarboxamido)-2-methoxyphenylcarboxamido]-3,6-dioxaoctanoic acid (3-amino-1-propyl)amide (L77)

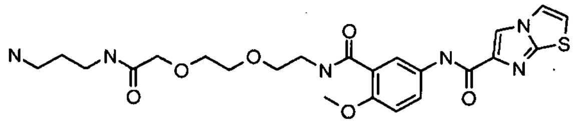

8-[5-(Imidazo-[2,1-b]thiazol-6-ylcarboxamido)-2-methoxyphenylcarboxamido]-3,6-dioxaoctanoic acid (6-amino-1-hexyl)amide (L78)

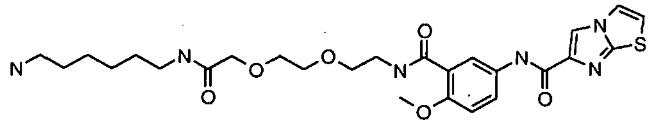

The synthesis of the ligands (II) can, for example, be carried out on insoluble supports, also known as resins, e.g. polystyrene resins, preferably pre-loaded with the desired linker bearing a reactive group, e. g. an amino group, to which a spacer group, e. g. an Fmoc protected amino acid, may attached by amide formation. After deprotection of the spacer group, the remaining parts of the molecule are attached by any reaction leading to an appropriate bond formation and, where necessary, additional synthetic steps, e. g. nucleophilic displacement reactions. Finally, the ligands including the spacer group (linker moieties) are released from the said insoluble support/resin by a suitable cleavage protocol known to the person skilled in the art and purified by chromatographic methods also known to the person skilled in the art.

If a suitable solid-phase synthesis protocol should not be applicable to a particular ligand, the synthesis can alternatively be carried out in solution. Typically, such a solution-phase synthesis consists of conjugation reactions between the ligand precursors and suitable spacer groups (e. g. amide formation) and of synthetic steps necessary for the assembly of the ligand itself. In some cases protective groups are necessary to prevent side-reactions.

The term "antibody" means an immunoglobulin, including both natural or wholly or partially synthetically produced and furthermore comprising all fragments and derivatives thereof which maintain specific binding ability. Typical fragments are Fc, Fab, heavy chain, and light chain. The term also comprises any polypeptide having a binding domain which is homologous or largely homologous, such as at least 95% identical when comparing the amino acid sequence, to an immunoglobulin binding domain. These polypeptides may be derived from natural sources, or partly or wholly synthetically produced. An antibody may be monoclonal or polyclonal and may be of human or non-human origin or a chimeric protein where the human Fc part is fused to murine Fab fragments (therapeutic antibodies with ending ...ximab, e.g. Rituximab) or to Fab fragments comprising human and murine sequences (therapeutic antibodies with the ending ...zumab, e.g. Bevacizumab) or linked to different Fab fragments (bispecific antibodies). The antibody may be a member of any immunoglobulin class, preferentially IgG, in case of human antibodies more preferentially $IgG_1$, $IgG_2$ and $IgG_4$.

In the most preferred embodiment of the present invention, the antibody comprises an Fc fragment or domain, as it is supposed that the ligands according to the present invention bind to an antibody's Fc part. Accordingly, an antibody of the present invention most preferably is an antibody containing an Fc fragment or domain of an immunoglobulin class, preferably IgG, more preferably of human IgG or of polyclonal or monoclonal IgG of human origin, in particular of $IgG_1$, $IgG_2$ and $IgG_4$.

The term "Fc fusion protein" refers to any combination of an Fc fragment with one or more proteins or protein domains. Examples of Fc fusion proteins include, but are not limited to, chimers of the human $IgG_1$ Fc domain with soluble receptor domains (therapeutic proteins with the ending....cept) such as Etanercept (human $IgG_1$ Fc combined with two tumor necrosis factor receptor domains) or Rilonacept (human $IgG_1$ fused with interleukin-1-receptor domain). The Fc fusion protein may be produced by any means. For instance, the Fc fusion protein may be enzymatically or chemically produced by coupling the Fc fragment to the appropriate protein or protein domain or it may be recombinantly produced from a gene encoding the Fc and the protein/protein domain sequence. Alternatively, the Fc fusion protein may be wholly or partially synthetically produced. The Fc fusion protein may also optionally be a multimolecular complex. A functional Fc fusion protein will typically comprise at least about 50 amino acids and more typically will comprise at least about 200 amino acids.

The present invention is drawn to the affinity purification, preferably by affinity chromatography, of antibodies or Fc fusion proteins from complex mixtures e.g. fermentation supernatants or plasma of human or animal origin, making use of the affinity ligands of formula (I) and preferred embodiments thereof, as disclosed elsewhere in the specification.

Accordingly, in a preferred embodiment, the present invention comprises a method for purifying a protein, preferably an immunoglobulin or Fc fusion protein, by affinity purification, preferably affinity chromatography. The affinity ligands according to the present invention bind to the Fc region of an antibody.

Bevacizumab (Avastin®, Genentech/Roche) is a humanized monoclonal antibody. It stops tumor growth by preventing the formation of new blood vessels by targeting and inhibiting the function of the protein vascular endothelial

growth factor-A (VEGF-A) that stimulates new blood vessel formation (angiogenesis).

Tocilizumab (RoActemra®, Genentech/Roche) is a humanized monoclonal antibody. It is directed against the Interleukin-6 receptor and blocks the action of the proinflammatory cytokine Interleukin-6. It is approved for the treatment of rheumatoid arthritis.

Palivizumab (Synagis®, Abbott) is a humanized monoclonal antibody directed against the A-epitope of the respiratory syncytial virus (RSV) F-protein. It is used in the prevention of RSV infections.

Polyclonal human and rabbit IgGs are investigated to demonstrate the universal applicability of the described ligands for the affinity purification of immunoglobulins via the Fc part.

When practicing the invention, the ligands according to the general formula (I) are attached to a support matrix of an appropriate support material, resulting in a ligand-substituted matrix, typically a matrix for affinity purification, preferably affinity chromatography (also referred to as affinity matrix in the context of the present invention) for protein separation. The ligands of the general formula are attached to the support matrix via L, optionally including a spacer -Sp-.

Accordingly, the present invention includes a ligand-substituted matrix (an affinity matrix) for protein separation, comprising a support material and at least one ligand as specified in the specification beforehand, wherein the ligand is attached thereto via L.

The support matrix - which may be denoted M - may comprise any appropriate support material which is known to the person skilled in the art. The material may be soluble or insoluble, particulate or non-particulate, or of a monolithic structure, including fibers and membranes, porous or non-porous. It provides a convenient means of separating ligands of the invention from solutes in a contacting solution. Examples of support matrix include carbohydrate and crosslinked carbohydrate matrices such as agarose, Sepharose, Sephadex, cellulose, dextran, starch, alginate or carrageenan; synthetic polymer matrices such as polystyrene, styrene-divinylbenzene copolymers, polyacrylates, PEG polyacrylate copolymers, polymethacrylates, (e.g. poly(hydroxyethylmethacrylate), polyvinyl alcohol, polyamides or perfluorocarbons; inorganic matrices such as glass, silica or metal oxides; and composite materials.

The affinity matrix is prepared by providing a support matrix of an appropriate support material and attaching a ligand of formula (I) thereto. Methods for attaching the ligand (I) to the support material are known to the person skilled in the art.

The present invention also relates to a method for affinity purification, preferably affinity chromatography, of a protein wherein a protein to be purified is contacted with the ligand-substituted matrix as described before.

The term "affinity purification" (which is used interchangeably with the term "affinity separation") refers to any separation technique involving molecular recognition of a protein by a ligand of formula (I). The ligand may be immobilized on a solid support facilitating separation of the ligand-antibody complex later on. Separation techniques may include affinity chromatography on packed columns, monolithic structures or membranes. The term further includes adsorption in batch-mode or affinity precipitation.

Independently of the flavor, purification techniques are composed by an initial recognition phase where ligand is contacted with antibody in crude. In a second phase, either impurities are separated from the ligand-antibody complex (e.g. column chromatography) or ligand-antibody complex is separated from impurities (e.g. affinity precipitation). In a third step, the antibody is released from the ligand-antibody complex by alteration of chemical and/or physical conditions like change in pH, ionic strength and/or addition of modifiers like organic solvents, detergents or chaotropes.

The invention will now be illustrated by the following examples.

Documents cited in the specification:

1. A.Cecilia, A.Roque, C.R. Lowe, M.A. Taipa: Antibodies and Genetically Engineered Related Molecules: Production and Purification, Biotechnol. Prog. 2004, 20, 639-654

2. K.L.Carson: Flexibility-the guiding principle for antibody manufacturing, Nature Biotechnology, 2005, 23, 1054-1058; S.Hober, K.Nord, M. Linhult: Protein A chromatography for antibody purification, J. Chromatogr. B. 2007, 848, 40-47

3. T.Arakawa, Y.Kita, H.Sato, D. Ejima, Protein Expression and Purification. 2009, 63, 158-163

4. S. Ghose, B. Hubbard, S.M. Cramer, Journal of Chromatography A, 2006, 1122, 144-152

## Examples

### Materials and Methods

If not otherwise stated, all chemicals and solvents were of analytical grade, with the exception of example 2. Reagents used in example 2 were of preparative to analytical grade depending on particulate requirements and availability. 96 and 384-well filter plates having hydrophilic membrane filters with 0.45 μm average pore size were purchased from Pall GmbH (Dreieich/Germany). Top frits made from polyethylene with 10 μm average pore size were provided by Porex (Bautzen/Germany). General purpose microtiterplates for collection of fractions and analytical assays were ordered from Greiner Bio One GmbH (Frickenhausen/Germany). Analytical assays were read out using a Fluostar Galaxy plate reader from BMG Labtech GmbH (Offenburg/Germany).

Column chromatography with antibody and purification of antibody fragments was conducted on a Waters HPLC system (Waters GmbH, Eschborn/Germany). Omnifit column housings (Diba Industries Ltd, Cambridge/United Kingdom) were used for packing of columns. NHS-activated Sepharose 4 FF, rProtein A Sepharose FF and Superdex 70 chromatography media were bought from GE Healthcare (Uppsala/Sweden). Mabsorbent A2P HF was purchased from Prometic Life Sciences (Cambridge/United Kingdom) and MEP Hypercel from Pall Corporation (Port Washington NY, USA).

Analytical chromatography of ligands was conducted on a Shimadzu HPLC system (Shimadzu Deutschland GmbH, Duisburg/Germany) including a diode array detector and single-quad mass spectrometer. The monolithic C18 reversed phase column was purchased from Merck KGaA (Darmstadt/Germany). Solvents used in analyses were of mass spectrometry grade.

Antibodies used in the present invention were Bevacizumab (Avastin, F. Hoffmann-La Roche, Switzerland), Tocilizumab (RoActemra, F. Hoffmann-La Roche, Switzerland), Palivizumab (Synagis, Abbott, USA), poly-IgG from human serum (Sigma-Aldrich, USA) and poly-IgG from rabbit serum (Sigma-Aldrich, USA). Immobilized papain for preparation of antibody fragments was purchased from Thermo Scientific (Bonn/Germany).

Flowthrough from protein A chromatography (referred to as host cell proteins) was derived from the supernatant of antibody-producing CHO cell culture in serum-free medium. Coomassie brilliant blue dye reagent for Bradford assay, was purchased from Thermo Scientific (Bonn/Germany).

## Example 1

### SPR Screening of chemical microarrays

Graffinity has developed high density chemical microarrays comprising thousands of small molecules immobilized onto gold chips. The arrays are constructed using maleimide-thiol coupling chemistry in combination with high density pintool spotting. For the construction, glass plates are microstructured by photolithographical methods in order to define up to 9,216 sensor fields per array. A subsequently applied gold coating provides the basis for the SPR effect and enables the formation of a binary, mixed self assembled monolayer (SAM) of two different thiols. One of the thiols exhibits a reactive maleimide moiety to which thiol-tagged array compounds can couple to during pintool spotting. The resulting microarrays comprise 9,216 sensor fields, each containing multiply copies of a defined and quality controlled array compound.

Surface plasmons are collective oscillations of electrons at a metal surface which can be resonantly excited by polarized light of appropriate wavelength and incidence angle. At surface plasmon resonance conditions, the light intensity reflected from the gold chip exhibits a sharp attenuation (SPR minimum). The angle and wavelength position of this reflectance minimum strongly depends on the refractive index of the medium adjacent to the metal surface. Processes which alter the local refractive index in close proximity to the metal surface (such as antibody binding to the immobilized ligands) can therefore be monitored sensitively. For SPR detection, the chemical microarray is incubated with the target protein under optimized screening conditions. If targets bind to the immobilized fragments a shift in the wavelength dependent SPR minimum (referred to as the SPR shift) occurs with respect to the buffer control and thus indicates protein-ligand interaction. The SPR imaging approach utilizes an expanded beam of parallel light to illuminate the entire microarray sensor area at a fixed angle. The reflected light is then captured by means of a CCD camera. While scanning over a range of wavelengths covering the SPR resonance conditions, reflection images are recorded stepwise. Automated spot finding routines and subsequent grey-scale analysis of the obtained pictures lead to SPR resonance curves of all 9,216 sensor fields per chip in parallel.

### Screening of Antibodies and Fragments thereof by SPR

The platform allowed screening of a proteins, antibodies and fragments thereof against Graffinity's immobilized compound library of 110,000 small molecules in a high-throughout fashion. For each of the targets, the SPR screening conditions were optimized individually before screening of the targets against the entire library. Among such optimized screening parameters were a) the composition of the screening buffer including appropriate detergents, b) the concentration of the antibody or protein target in solution and c) the surface density of immobilized ligands on the chip surface. Neumann et al. (1) summarizes the screened targets and the corresponding screening conditions applied. In total four different full-length antibodies were screened and compared. Additionally, the Fc and Fab fragments of two antibodies

were obtained by proteolytic digestion and were subjected to array screening as well. In order to identify antibody specific ligands, the screening campaign included also antibody free host cell protein (HCP) of CHO cell lines as controls.

The experimental results of the microarray screening campaigns were analyzed by manual hit selection using in-house developed software routines as well as by an in-depth data analysis by commercially available data mining tools. The analysis resulted in a number of hit series which showed clear binding to antibodies and their Fc fragments but only weak to negligible binding to the HCP controls. Such primary hit series were used for hit confirmation in secondary assays and could be used as starting point for the synthesized of focused libraries around the primary hits. For example, the Fc specific ligand L1 showed distinct binding to the full length Bevacizumab and its Fc fragment but only minor binding to the Bevacizumab Fab fragment. Furthermore it clearly bound the other full length antibodies Anti RhD IgG, Tocilicumab and Palivizumab.

**Example 2**

**Synthesis of Ligands**

**General Procedure A: Synthesis of 5-Arylcarboxamido-2-methoxybenzoic acid N-(3-aminopropyl)amides** (L1, L2, L3, L6, L8, L7, L15, L16, L5, L11, L20, L27, L25, L4, L26, L31, L32, L33, L34, L35, L36, L42, L44, L45, L46, L47, L48, L51, L56, L57, L63) )

N-Fmoc-5-amino-2-methoxybenzoic acid (0.2-0.5 mmol) and HOAt (1 equivalent relative to the carboxylic acid) were dissolved in DMF, NMP, DMF/DMSO or NMP/DMSO mixtures (1-3 mL) and treated with DIC (1 equivalent relative to the carboxylic acid). After agitating for 2-5 min, the mixture was added to 0.1-0.15 mmol of 1,3-diaminopropane linked to either trityl-polystyrene resin or 2-chlorotrityl polystyrene resin. The mixture was shaken for several hours or overnight. Next, the resin was washed (DMF or DMF, dichloromethane or DMF, methanol, dichloromethane, each solvent several times) and treated with piperidine 25% in DMF (1-5 mL) for 15-30 min. Subsequently, the resin was washed thoroughly (DMF, dichloromethane or DMF, methanol, dichloromethane, each solvent several times), and dried at air, with a stream of nitrogen or in high vacuum.

The arylcarboxylic acid (0.2-0.5 mmol) and HOAt (1 equivalent relative to the carboxylic acid) were dissolved in DMF, NMP, DMF/DMSO or NMP/DMSO mixtures (1-3 mL) and treated with DIC (1 equivalent relative to the carboxylic acid). After agitating for 2-5 min, the mixture was added to the resin and shaken for several hours or overnight. After subsequent washing (DMF, dichloromethane or DMF, methanol, dichloromethane, each solvent several times), the target compound was cleaved from the support by treatment with a suitable cleavage mixture (dichloromethane, TFA, triethylsilane 85:10:5 for trityl resins, 45:45:10 for 2-chlorotrityl resins). Typically, the cleavage step was repeated once, followed by rinsing of the resin with dichloromethane. After evaporation of the solvents, the crude residue was purified by preparative reversed-phase HPLC.

With respect to the high robustness of amide coupling chemistry, other coupling protocols may be applied with comparable or identical results. Particularly, TBTU or HATU coupling (1 equivalent relative to the carboxylic acid; additionally 2 equivalents of DIPEA must be added) represents a reliable alternative to DIC/HOAt coupling which proceeds more rapidly (typical reaction times: 30 min to 3 h); however, for some carboxylic acids, particularly those with a free aryl NH like indoles or (benzo)pyrazoles, coupling under basic conditions might lead to unwanted side-products.

**5-[5-(2-Furyl)-1-methylpyrazol-3-yl]carboxamido-2-methoxybenzoic acid N-(3-aminopropyl)amide (L1)**

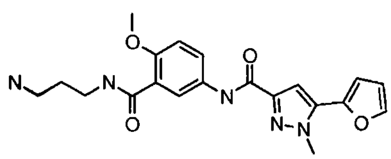

Prepared from 5-(2-furyl)-1-methylpyrazole-3-carboxylic acid following General Procedure A. ESI-MS: 398 (M+1).

**5-[5-(4-Bromo-2-thienyl)-1-methylpyrazol-3-yl]carboxamido-2-methoxybenzoic acid N-(3-aminopropyl)amide (L2)**

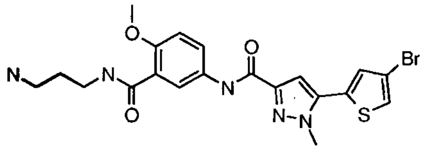

Prepared from 5-(4-bromo-2-thienyl)-1-methylpyrazole-3-carboxylic acid following General Procedure A. ESI-MS: 493, 495 (M+1).

**5-[5-(2,5-Dimethyl-3-thienyl)-1-methylpyrazol-3-yl]carboxamido-2-methoxybenzoic acid N-(3-aminopropyl)amide (L3)**

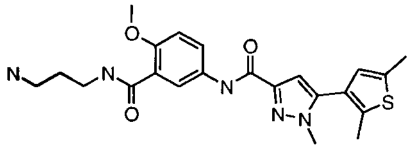

Prepared from 5-(2,5-dimethyl-3-thienyl)-1-methylpyrazole-3-carboxylic acid following General Procedure A. ESI-MS: 442 (M+1).

**2-Methoxy-5-[1-methyl-5-(2-thienyl)pyrazol-3-yl]carboxamidobenzoic acid N-(3-aminopropyl)amide (L6)**

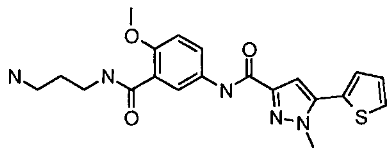

Prepared from 1-methyl-5-(2-thienyl)pyrazole-3-carboxylic acid following General Procedure A. ESI-MS: 414 (M+1).

**2-Methoxy-5-[1-methyl-5-(3-thienyl)pyrazol-3-yl]carboxamidobenzoic acid N-(3-aminopropyl)amide (L8)**

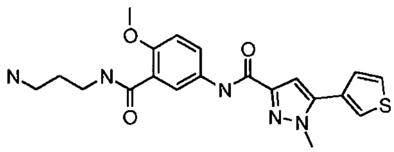

Prepared from 1-methyl-5-(3-thienyl)pyrazole-3-carboxylic acid following General Procedure A. ESI-MS: 414 (M+1).

**5-[5-(3-Chlorophenyl)-1-methylpyrazol-3-yl]carboxamido-2-methoxybenzoic acid N-(3-aminopropyl)amide (L7)**

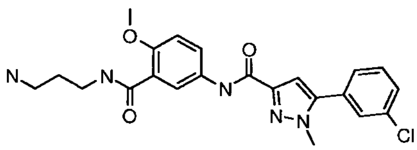

Prepared from 5-(3-chlorophenyl)-1-methylpyrazole-3-carboxylic acid following General Procedure A. ESI-MS: 442, 444 (M+1).

**2-Methoxy-5-(1-methyl-5-phenylpyrazol-3-yl)carboxamidobenzoic acid N-(3-aminopropyl)amide (L15)**

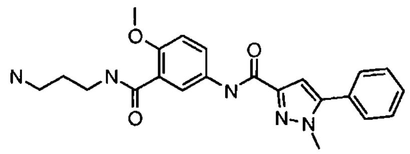

Prepared from 1-methyl-5-phenylpyrazole-3-carboxylic acid following General Procedure A. ESI-MS: 408 (M+1).

**2-Methoxy-5-[5-(1-methyl-2-pyrrolyl)-1-methylpyrazol-3-yl]carboxamidobenzoic acid N-(3-aminopropyl)amide (L16)**

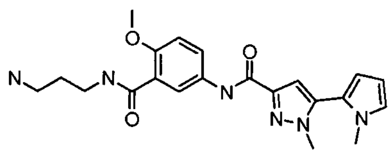

Prepared from 5-(1-methyl-2-pyrrolyl)-1-methylpyrazole-3-carboxylic acid following General Procedure A. ESI-MS: 411 (M+1).

**2-Methoxy-5-[3-(2-thienyl)pyridin-5-yl]carboxamidobenzoic acid N-(3-aminopropyl)amide (L5)**

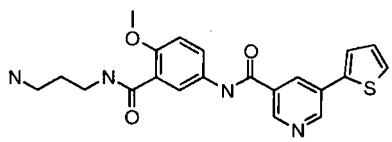

Prepared from 3-(2-thienyl)pyridine-5-carboxylic acid following General Procedure A. ESI-MS:411(M+1).

**2-Methoxy-5-(3-phenylpyridin-5-yl)carboxamidobenzoic acid N-(3-aminopropyl)amide (L11)**

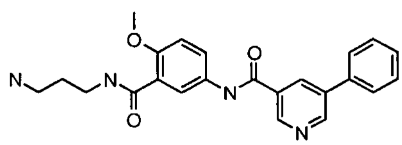

Prepared from 3-phenylpyridine-5-carboxylic acid following General Procedure A. ESI-MS: 405 (M+1).

**5-[5-(2-Furyl)pyrazol-3-yl]carboxamido-2-methoxybenzoic acid N-(3-aminopropyl)amide (L20)**

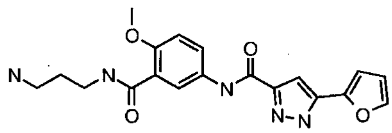

Prepared from 5-(2-furyl)pyrazole-3-carboxylic acid following General Procedure A. ESI-MS: 384 (M+1).

**2-Methoxy-5-(1-methylindazol-3-yl)carboxamidobenzoic acid N-(3-aminopropyl)amide (L27)**

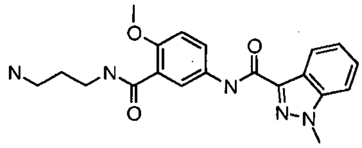

Prepared from 1-methylindazole-3-carboxylic acid following General Procedure A. ESI-MS: 382 (M+1).

**2-Methoxy-5-(1-methylindol-3-yl)carboxamidobenzoic acid N-(3-aminopropyl)amide (L25)**

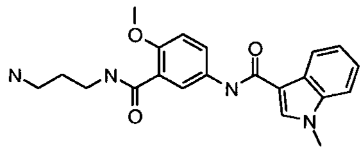

Prepared from 1-methylindole-3-carboxylic acid following General Procedure A. ESI-MS: 381 (M+1).

**5-(5-Chloro-1H-indazol-3-yl)carboxamido-2-methoxybenzoic acid N-(3-aminopropyl)amide (L4)**

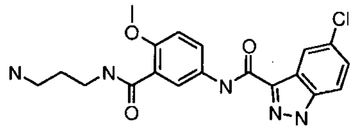

Prepared from 5-chloro-1H-indazole-3-carboxylic acid following General Procedure A. ESI-MS: 402,404 (M+1).

**5-(Indol-3-yl)carboxamido-2-methoxybenzoic acid N-(3-aminopropyl)amide (L26)**

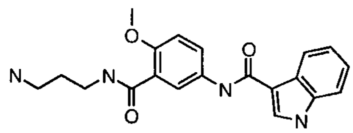

Prepared from indole-3-carboxylic acid following General Procedure A. Indole-3-carboxylic acid was coupled by activation with HOBt (1 equivalent) and DIC (1 equivalent, relative to the carboxylic acid); coupling time: 1 h. ESI-MS: 367 (M+1).

**5-(Benzo[c]isoxazol-3-yl)carboxamido-2-methoxybenzoic acid N-(3-aminopropyl)amide (L31)**

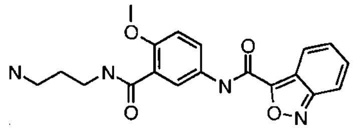

Prepared from benzo[c]isoxazole-3-carboxylic acid following General Procedure A. ESI-MS: 369 (M+1).

**5-(Benzo[c]furan-1-yl)carboxamido-2-methoxybenzoic acid N-(3-aminopropyl)amide (L32)**

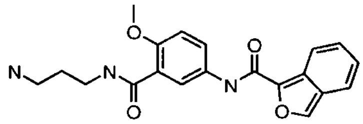

Prepared from benzo[c]furan-1-carboxylic acid following General Procedure A. ESI-MS: 368 (M+1).

**5-(1,5-Dimethylpyrazol-3-yl)carboxamido-2-methoxybenzoic acid N-(3-aminopropyl)amide (L33)**

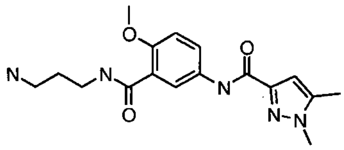

Prepared from 1,5-dimethylpyrazole-3-carboxylic acid following General Procedure A. ESI-MS: 346 (M+1).

**2-Methoxy-5-[1-methyl-5-(trifluoromethyl)pyrazol-3-yl]carboxamidobenzoic acid N-(3-aminopropyl)amide (L34)**

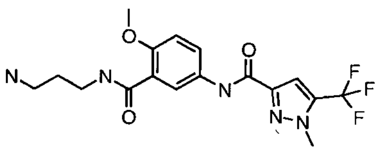

Prepared from 1-methyl-5-(trifluoromethyl)pyrazole-3-carboxylic acid following General Procedure A. ESI-MS: 400 (M+1).

**5-(1-Isoquinolinyl)carboxamido-2-methoxybenzoic acid N-(3-aminopropyl)amide (L35)**

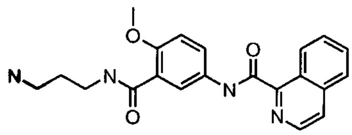

Prepared from isoquinoline-1-carboxylic acid following General Procedure A. ESI-MS: 379 (M+1).

**5-(Indazol-3-yl)carboxamido-2-methoxybenzoic acid N-(3-aminopropyl)amide (L36)**

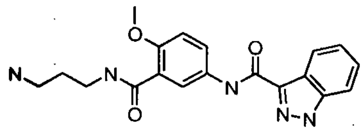

Prepared from indazole-3-carboxylic acid following General Procedure A. Indazole-3-carboxylic acid was coupled by activation with HOBt (1 equivalent) and DIC (1 equivalent, relative to the carboxylic acid); coupling time: 1 h. ESI-MS: 368 (M+1).

**2-Methoxy-5-[5-(2-methylthiazol-4-yl)isoxazol-3-ylcarboxamido]benzoic acid (3-amino-1-propyl)amide (L42)**

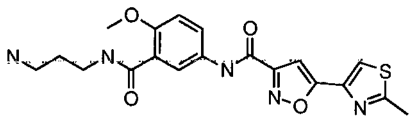

Prepared from 5-(2-methylthiazol-4-yl)isoxazole-3-carboxylic acid following General Procedure A. ESI-MS: 416 (M+1).

**2-Methoxy-5-(1-methylpyrazolo[3,4-b]pyridin-3-ylcarboxamido)benzoic acid (3-amino-1-propyl)amide (L44)**

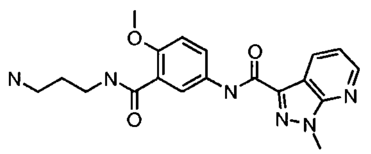

Prepared from 1-methylpyrazolo[3,4-b]pyridine-3-carboxylic acid following General Procedure A. ESI-MS: 383 (M+1).

**5-[5-(2-Furyl)isoxazol-3-ylcarboxamido]-2-methoxybenzoic acid (3-amino-1-propyl)amide (L45)**

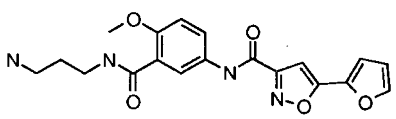

Prepared from 5-(2-furyl)isoxazole-3-carboxylic acid following General Procedure A. ESI-MS: 385 (M+1).

**2-Methoxy-5-(pyrazolo[1,5-a]pyridin-2-ylcarboxamido)benzoic acid (3-amino-1-propyl)amide (L46)**

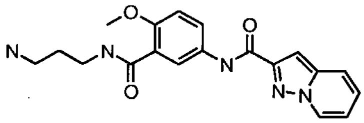

Prepared from pyrazolo[1,5-a]pyridine-2-carboxylic acid following General Procedure A. ESI-MS: 368 (M+1).

**5-(1H-Benzimidazol-2-ylcarboxamido)-2-methoxybenzoic acid (3-amino-1-propyl)amide** (L47)

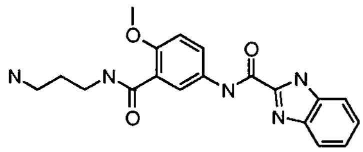

Prepared from 1H-benzimidazole-2-carboxylic acid following General Procedure A. ESI-MS: 368 (M+1).

**5-(Imidazo[1,2-b]pyridazin-2-ylcarboxamido)-2-methoxybenzoic acid (3-amino-1-propyl)amide (L48)**

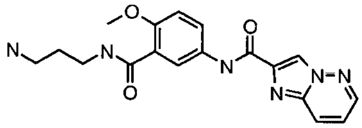

Prepared from imidazo[1,2-b]pyridazine-2-carboxylic acid following General Procedure A. ESI-MS: 369 (M+1).

**2-Methoxy-5-(3-phenylisoxazol-5-ylcarboxamido)benzoic acid (3-amino-1-propyl)amide (L51)**

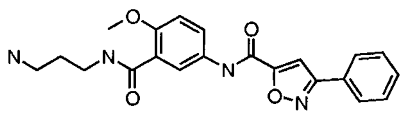

Prepared from 3-phenylisoxazole-5-carboxylic acid following General Procedure A. ESI-MS: 395 (M+1).

**2-Methoxy-5-(pyrazolo[1,5-a]pyrimidin-2-ylcarboxamido)benzoic acid (3-amino-1-propyl)amide (L56)**

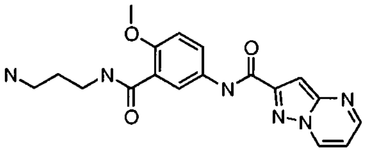

Prepared from pyrazolo[1,5-a]pyrimidine-2-carboxylic acid following General Procedure A. ESI-MS: 369 (M+1).

**5-(Imidazo[1,2-a]pyridin-2-ylcarboxamido)-2-methoxybenzoic acid (3-amino-1-propyl)amide (L57)**

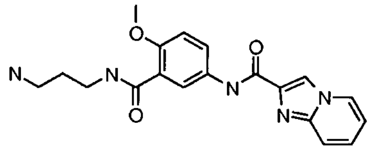

Prepared from imidazo[1,2-a]pyridine-2-carboxylic acid hydrobromide following General Procedure A. One additional equivalent of DIPEA was added when the carboxylic acid hydrobromide was coupled using HATU/DIPEA. ESI-MS: 368 (M+1).

**5-(Imidazo[1,2-a]pyrimidin-2-ylcarboxamido)-2-methoxybenzoic acid (3-amino-1-propyl)amide (L63)**

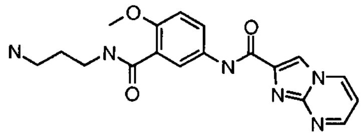

Prepared from imidazo[1,2-a]pyrimidine-2-carboxylic acid following General Procedure A. ESI-MS: 369 (M+1).

**General Procedure B: Synthesis of 5-[3-(aryl or cyclopropyl)pyridin-5-yl]carboxamido-2-methoxybenzoic acid N-(3-aminopropyl)amides by Suzuki coupling** (L12, L13, L14, L22, L23)

To 0.1 mmol of 1,3-diaminopropane-trityl-polystyrene resin, N-Fmoc-5-amino-2-methoxybenzoic acid and 5-bromopyridine-3-carboxylic acid were coupled following General Procedure A. The resulting resin loaded with the bromopyridine carboxylic amide was dried thoroughly and transferred to a glass vial. After addition of caesium carbonate or potassium carbonate (0.3 mmol) and the respective boronic acid (1 mmol), absolute DMF (1 mL) was added and the mixture was extensively flushed with argon. Subsequently, tetrakis-triphenylphosphinepalladium(O) (10 $\mu$mol) was added, the mixture was again flushed with argon and the vial was tightly closed. The mixture was heated to 100 °C and shaken overnight. Then the resin was washed thoroughly (DMF, dichloromethane, each solvent several times) and a small sample was cleaved (conditions, see below). When LCMS indicated incomplete conversion, the coupling step was repeated or conducted at higher temperatures. Otherwise, the resin was treated with dichloromethane-TFA-triethylsilane 85:10:5 (twofold cleavage), followed by rinsing of the resin with dichloromethane. After evaporation to dryness, the residue was purified by preparative reversed-phase HPLC.

**2-Methoxy-5-{3-[4-(trifluoromethyl)phenyl]pyridin-5-yl}carboxamidobenzoic acid N-(3-aminopropyl)amide (L12)**

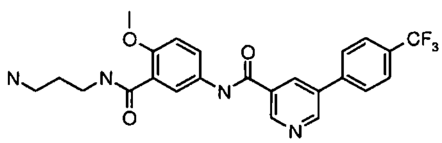

Prepared from 4-(trifluoromethyl)phenylboronic acid following General Procedure B. ESI-MS: 473 (M+1).

**5-[3-(2-Furyl)pyridin-5-yl]carboxamido-2-methoxybenzoic acid N-(3-aminopropyl)amide (L13)**

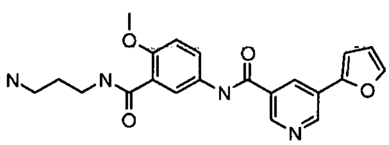

Prepared from 2-furylboronic acid following General Procedure B. ESI-MS: 395 (M+1).

**5-[3-(2-Benzothienyl)pyridin-5-yl]carboxamido-2-methoxybenzoic acid N-(3-aminopropyl)amide (L14)**

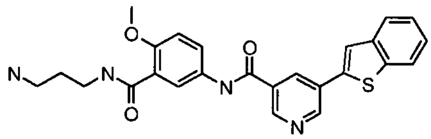

Prepared from 2-benzothienylboronic acid following General Procedure B. ESI-MS: 461 (M+1).

**5-[3-(3-Furyl)pyridin-5-yl]carboxamido-2-methoxybenzoic acid N-(3-aminopropyl)amide (L22)**

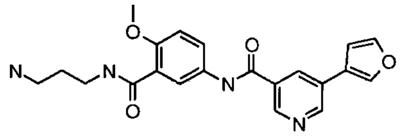

Prepared from 3-furylboronic acid following General Procedure B. ESI-MS: 395 (M+1).

**5-(3-Cyclopropylpyridin-5-yl)carboxamido-2-methoxybenzoic acid N-(3-aminopropyl)amide (L23)**

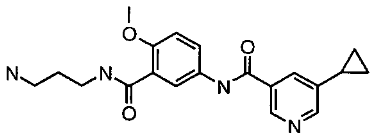

Prepared from cyclopropylboronic acid following General Procedure B. ESI-MS: 369 (M+1).

**5-(3-Ethynylpyridin-5-yl)carboxamido-2-methoxybenzoic acid N-(3-aminopropyl)amide (L24)**

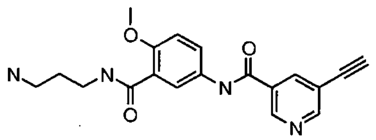

To 0.1 mmol of 1,3-diaminopropane-trityl-polystyrene resin, N-Fmoc-5-amino-2-methoxybenzoic acid and 5-bromopyridine-3-carboxylic acid were coupled following General Procedure A. The resulting resin loaded with the bromopyridine carboxylic amide was dried thoroughly and transferred to a glass vial. Copper(I) chloride (40 μmol), triphenylphosphine (40 μmol), bis(triphenylphosphine)palladium(II) dichloride (10 μmol) and THF (1 mL) were added and the mixture was flushed with argon. Subsequently, trimethylsilylacetylene (1 mmol) and triethylamine (0.5 mL) were added, the mixture was flushed again with argon, then the vial was closed tightly and shaken overnight at 50 °C. Then the resin was washed thoroughly with DMF and dichloromethane (each solvent several times). For TMS deprotection, THF (1 mL) and water (50 μL) were added, followed by tetrabutylammonium fluoride (1 M solution in THF, 0.5 mL). The mixture was agitated for 2 h, then the resin was washed with DMF and dichloromethane (each solvent several times). Cleavage of the target compound from the resin was effected by treatment with dichloromethane-TFA-triethylsilane

85:10:5 (twofold cleavage), followed by rinsing with dichloromethane. After evaporation to dryness, the residue was purified by preparative reversed-phase HPLC. ESI-MS: 353 (M+1).

**General Procedure C: Synthesis of 5-[5-(2-thienyl or 2-furyl)-1-methylpyrazol-3-yl]carboxamidoaryl carboxylic acid N-(3-aminopropyl)amides** (L19, L9, L10, L17, L18)

The respective (N-Fmoc protected) aminoarylcarboxylic acid (0.2-0.5 mmol) and HOAt (1 equivalent relative to the carboxylic acid) were dissolved in DMF, NMP, DMF/DMSO or NMP/DMSO mixtures (1-3 mL) and treated with DIC (1 equivalent relative to the carboxylic acid). After agitating for 2-5 min, the mixture was added to 0.1-0.15 mmol of 1,3-diaminopropane linked to either trityl-polystyrene resin or 2-chlorotrityl polystyrene resin. The mixture was shaken for several hours or overnight. Next, the resin was washed (DMF or DMF, dichloromethane or DMF, methanol, dichloromethane, each solvent several times) and treated with piperidine 25% in DMF (1-5 mL) for 15-30 min. Subsequently, the resin was washed thoroughly (DMF, dichloromethane or DMF, methanol, dichloromethane, each solvent several times), and dried at air, with a stream of nitrogen or in high vacuum. 1-Methyl-5-(2-thienyl)pyrazole-3-carboxylic acid or 5-(2-furyl)-1-methylpyrazole-3-carboxylic acid (0.2-0.5 mmol) and HOAt (1 equivalent relative to the carboxylic acid) were dissolved in DMF, NMP, DMF/DMSO or NMP/DMSO mixtures (1-3 mL) and treated with DIC (1 equivalent relative to the carboxylic acid). After agitating for 2-5 min, the mixture was added to the resin and shaken for several hours or overnight. After subsequent washing (DMF, dichloromethane or DMF, methanol, dichloromethane, each solvent several times), the target compound was cleaved from the support by treatment with a suitable cleavage mixture (dichloromethane, TFA, triethylsilane 85:10:5 for trityl resins, 45:45:10 for 2-chlorotrityl resins). Typically, the cleavage step was repeated once, followed by rinsing of the resin with dichloromethane. After evaporation of the solvents, the crude residue was purified by preparative reversed-phase HPLC.

**2-[1-Methyl-5-(2-thienyl)pyrazol-3-yl]carboxamidothiazole-4-carboxylic acid N-(3-aminopropyl)amide (L19)**

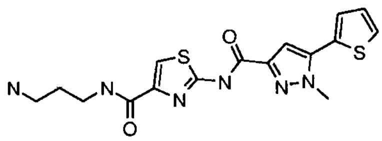

Prepared from N-Fmoc-2-aminothiazole-4-carboxylic acid and 1-methyl-5-(2-thienyl)pyrazole-3-carboxylic acid following General Procedure C. ESI-MS: 391 (M+1).

**2-Methyl-3-[1-methyl-5-(2-thienyl)pyrazol-3-yl]carboxamidobenzoic acid N-(3-aminopropyl)amide (L9)**

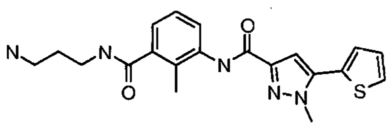

Prepared from N-Fmoc-3-amino-2-methylbenzoic acid and 1-methyl-5-(2-thienyl)pyrazole-3-carboxylic acid following General Procedure C. ESI-MS: 398 (M+1).

**4-Methyl-3-[1-methyl-5-(2-thienyl)pyrazol-3-yl]carboxamidobenzoic acid N-(3-aminopropyl)amide (L10)**

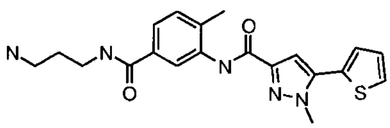

Prepared from N-Fmoc-3-amino-4-methylbenzoic acid and 1-methyl-5-(2-thienyl)pyrazole-3-carboxylic acid following General Procedure C. ESI-MS: 398 (M+1).

**3-[5-(2-Furyl)-1-methylpyrazol-3-yl]carboxamidobenzoic acid N-(3-aminopropyl)amide (L17)**

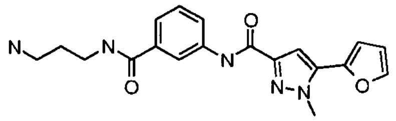

Prepared from N-Fmoc-3-aminobenzoic acid and 5-(2-furyl)-1-methylpyrazole-3-carboxylic acid following General Procedure C. ESI-MS: 368 (M+1).

**4-[5-(2-Furyl)-1-methylpyrazol-3-yl]carboxamidobenzoic acid N-(3-aminopropyl)amide (L18)**

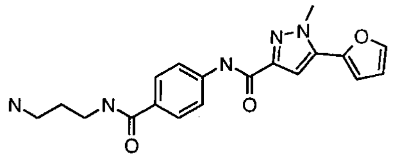

Prepared from N-Fmoc-4-aminobenzoic acid and 5-(2-furyl)-1-methylpyrazole-3-carboxylic acid following General Procedure C. ESI-MS: 368 (M+1).

**3-{N-[5-(2-Furyl)pyrazol-3-yl]carbamoyl}benzoic acid N'-(3-aminopropyl)amide (L21)**

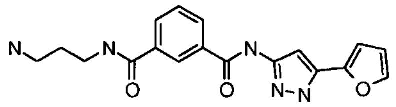

1,3-Diaminopropane-trityl resin (0.1 mmol) was swollen in dichloromethane and treated with excess DIPEA and m-benzenedicarboxylic dichloride. After a few minutes, the resin was quickly washed with dichloromethane (3 times) and immediately treated with excess 3-amino-5-(2-furyl)pyrazole and DIPEA in NMP. When LCMS of a small resin sample which was cleaved (conditions, see below) showed complete conversion, the resin was washed (DMF, dichloromethane, each solvent several times) and treated with dichloromethane-TFA-triethylsilane (85:10:5; twofold cleavage, followed by rinsing of the resin with dichloromethane). The cleavage solution was evaporated to dryness and the residue was purified by preparative reversed-phase HPLC. ESI-MS: 354 (M+1).

**5-(Imidazo[2,1-b]thiazol-6-yl)carboxamido-2-methoxybenzoic acid N-(3-aminopropyl)amide (L37)**

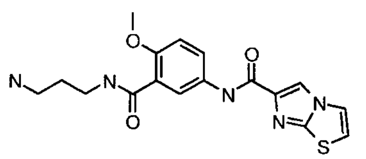

1,3-Diaminopropane-trityl-resin (0.15 mmol) was pre-swollen in NMP. N-Fmoc-5-amino-2-methoxybenzoic acid (0.2 mmol) and HATU (0.2 mmol) were dissolved in NMP (1.5 mL) and treated with DIPEA (0.4 mmol). After 2 min, the solution was added to the resin and the reaction mixture was shaken for 30 min. After washing the resin with DMF (3

times), 25% piperidine in DMF was added and shaking was continued for 30 min. Next, the resin was washed thoroughly with DMF, methanol and dichloromethane (each solvent 3 times) and dried at air.
Imidazo[2,1-b]thiazole-6-carboxylic acid hydrobromide (0.2 mmol) and HATU (0.2 mmol) were dissolved or suspended in NMP (1.5 mL) and treated with DIPEA (0.8 mmol). After 2 min, the solution was added to the resin and shaken overnight. After washing the resin with DMF, methanol and dichloromethane (each solvent 3 times), the target compound was cleaved from the resin by treatment with dichloromethane-TFA-triethylsilane 85:10:5 (twofold cleavage), followed by rinsing with dichloromethane. After evaporation to dryness, the residue was purified by preparative reversed-phase HPLC.
ESI-MS: 374 (M+1).

**5-[5-(2-Furyl)-1-methylpyrazol-3-yl]carboxamido-2-methoxybenzoic acid N-(3-aminopropyl)-N-methylamide (L28)**

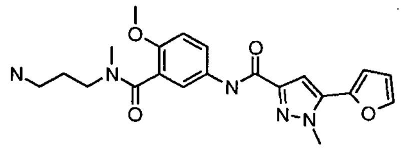

2-Chlorotrityl chloride resin (150 μmol) was pre-swollen in NMP and treated with NMP (1.5 mL), followed by 3-aminopropanol (1.5 mL). After shaking the resin for 2 h, it was washed with DMF, methanol and dichloromethane (each solvent several times) and dried at air. Then dichloromethane (2 mL) and DIPEA (2 mmol) were added, followed by dropwise addition of methanesulfonic chloride (1 mmol). The resin was agitated for 2 h, then it was washed quickly with dichloromethane (3 times) and swollen in NMP (1 mL). After addition of methylamine solution (8 M in ethanol, 1 mL), the resin was shaken overnight, then it was washed with DMF, methanol and dichloromethane (each solvent several times) and dried at air.

N-Fmoc-5-amino-2-methoxybenzoic acid (250 μmol) and HOAt (250 μmol) were dissolved in NMP (approx. 1.5 mL) and treated with DIC (250 μmol). After 2 min, the solution was added to the resin and shaken for 5 h. After washing with DMF and dichloromethane (each solvent several times), the resin was treated with 25% piperidine in DMF (30 min, followed by washing as described before).

5-(2-Furyl)-1-methylpyrazole-3-carboxylic acid (250 μmol) and HOAt (250 μmol) were dissolved in NMP (approx. 1.5 mL) and treated with DIC (250 μmol). After 2 min, the solution was added to the resin and shaken overnight. Then the resin was washed as described before and the target compound was cleaved from the resin by treatment with dichloromethane-TFA-triethylsilane 45:45:10 (twofold cleavage), followed by rinsing with dichloromethane. After evaporation to dryness, the residue was purified by preparative reversed-phase HPLC. ESI-MS: 412 (M+1).

**5-[5-(2-Furyl)-1-methylpyrazol-3-yl]carboxamido-2-ethoxybenzoic acid N-(3-aminopropyl)amide (L29)**

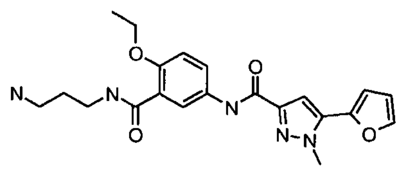

5-Nitrosalicylic acid (0.3 mmol) and HOBt (0.3 mmol) were dissolved in DMSO (approx. 1.5 mL) and treated with DIC (0.3 mmol). After 2 min the solution was added to 1,3-diaminopropane-trityl resin (0.15 mmol) and shaken for 1 h, whereupon the resin was washed with DMF, water, diluted sodium carbonate solution, water, methanol and dichloromethane (each solvent several times). A small resin sample was treated with benzoyl chloride and DIPEA, washed and cleaved (conditions, see below), and the resulting product was analyzed by HPLC-MS, indicating incomplete loading. Thus, the coupling step was repeated for 30 min. In order to hydrolyze the amount of carbamoyl-nitrophenylester formed in the meantime, the resin was subsequently treated with THF (1 mL), methanol (0.5 mL) and aqueous NaOH (1 M, 0.5 mL) for 5 min, while the supernatant turning yellow indicated successful ester hydrolysis. After washing with DMF, methanol and dichloromethane (each solvent 3 times), DMSO (2 mL), caesium carbonate (1 mmol) and ethyl bromide

(1 mmol) were added and the resin was shaken for approx. 2 h, then the reaction was repeated overnight (0.2 mL of water were added to dissolve the carbonate salt). As the conversion was still incomplete, the reaction was repeated using 0.5 mL of ethyl bromide and water (0.2 mL), leading to sufficient reaction progress after three days, then the resin was washed with DMF, water, methanol and dichloromethane (each solvent several times).

The nitro group was reduced by addition of NMP (1 mL), pyridine (0.5 mL) and a solution of tin(II) chloride (1 mmol) in NMP (1 mL). The mixture was shaken overnight, then the resin was washed with DMF, water and methanol, and insoluble side-products were carefully removed by flotation in methanol. After washing with dichloromethane, the resin was dried. 5-(2-Furyl)-1-methylpyrazole-3-carboxylic acid (0.25 mmol) and HOAt (0.25 mmol) were dissolved in NMP (approx. 1.5 mL) and treated with DIC (0.25 mmol). After 2 min, the solution was added to the resin and shaken overnight. Then the resin was washed with DMF and dichloromethane (each solvent several times) and the target compound was cleaved from the resin by treatment with dichloromethane-TFA-triethylsilane 85:10:5 (twofold cleavage), followed by rinsing with dichloromethane. After evaporation to dryness, the residue was purified by preparative reversed-phase HPLC. ESI-MS: 412 (M+1).

**5-[5-(2-Furyl)-1-methylpyrazol-3-yl]carboxamido-2-hydroxybenzoic acid N-(3-aminopropyl)amide (L30)**

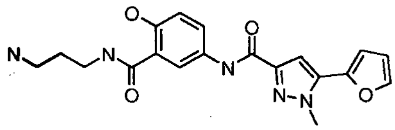

5-Nitrosalicylic acid (0.3 mmol) and HOBt (0.3 mmol) were dissolved in DMSO (approx. 1.5 mL) and treated with DIC (0.3 mmol). After 2 min the solution was added to 1,3-diaminopropane-trityl resin (0.15 mmol) and shaken for 1 h, whereupon the resin was washed with DMF, water, diluted sodium carbonate solution, water, methanol and dichloromethane (each solvent several times). A small resin sample was treated with benzoyl chloride and DIPEA, washed and cleaved (conditions, see below), and the resulting product was analyzed by HPLC-MS, indicating incomplete loading. Thus, the coupling step was repeated for 30 min. In order to hydrolyze the amount of carbamoyl-nitrophenylester formed in the meantime, the resin was subsequently treated with THF (1 mL), methanol (0.5 mL) and aqueous NaOH (1 M, 0.5 mL) for 5 min. while the supernatant turning yellow indicated successful ester hydrolysis. After washing with DMF, methanol and dichloromethane (each solvent 3 times), DMSO (2 mL), caesium carbonate (1 mmol) and allyl bromide (1 mmol) were added and the resin was shaken for 1 h, then the reaction was repeated overnight (0.2 mL of water were added). As the conversion was still incomplete, the reaction was once again repeated for three days, then the resin was washed with DMF, water, methanol and dichloromethane (each solvent several times)

The nitro group was reduced by addition of NMP (1 mL), pyridine (0.5 mL) and a solution of tin(II) chloride (1 mmol) in NMP (1 mL). The mixture was shaken overnight, then the resin was washed with DMF, water and methanol, and insoluble side-products were carefully removed by flotation in methanol. After washing with dichloromethane, the resin was dried. 5-(2-Furyl)-1-methylpyrazole-3-carboxylic acid (0.25 mmol) and HOAt (0.25 mmol) were dissolved in NMP (approx. 1.5 mL) and treated with DIC (0.25 mmol). After 2 min, the solution was added to the resin and shaken overnight. Then the resin was washed with DMF and dichloromethane (each solvent several times).

The allyl ether was cleaved by treatment of the resin with dichloromethane (2 mL), piperidine (0.4 mL) and tetrakis-triphenylphosphinepalladium(0) (approx. 10 mg) for approx. 2 h. Then the resin was thoroughly washed with DMF, water, methanol and dichloromethane (each solvent several times) and the target compound was cleaved from the resin by treatment with dichloromethane-TFA-triethylsilane 85:10:5 (twofold cleavage), followed by rinsing with dichloromethane. After evaporation to dryness, the residue was purified by preparative reversed-phase HPLC. ESI-MS: 384 (M+1).

**5-(1-Ethylindazol-3-ylcarboxamido)-2-methoxybenzoic acid (3-amino-1-propyl)amide (L38)**

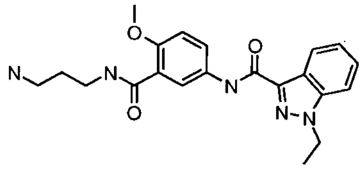

Trityl polystyrene resin preloaded with 1,3-diaminopropane (150 μmol) was pre-swollen with NMP. Fmoc-5-amino-2-methoxybenzoic acid (200 μmol) and HATU (200 μmol) in NMP (1.5 mL) were treated with DIPEA (400 μmol). After two minutes, the solution was added to the resin and the mixture was shaken at room temperature for 3 h. After washing the resin with DMF, piperidine (25% in DMF, approx. 2 mL) was added and the resin was shaken for approx. 30 min, followed by thorough washing with DMF and dichloromethane. Indazole-3-carboxylic acid (0.5 mmol) and HOBt (0.5 mmol) were dissolved in NMP (1.5 mL). DIC (0.5 mmol) was added and the solution was added to the resin after 2 min. The mixture was shaken for 1 h, afterwards the resin was washed with DMF and dichloromethane. Caesium carbonate (1 mmol) and dry DMF (1.5 mL) were added to the resin, followed by ethyl bromide (1.5 mmol). The mixture was shaken over night, followed by washing with DMF, water, methanol and dichloromethane. The caesium carbonate/ethyl bromide step was repeated once over three days, then the resin was washed thoroughly. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 396 (M+1).

**5-[N-(1-Methylindazol-3-yl)carbamoyl]-2-methoxybenzoic acid (3-amino-1-propyl)amide (L39)**

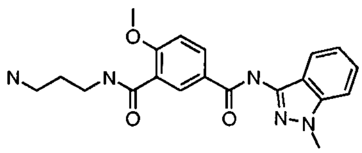

Trityl polystyrene resin preloaded with 1,3-diaminopropane (150 μmol) was pre-swollen in NMP. 5-Formylsalicylic acid (0.5 mmol) and HOAt (0.5 mmol) were dissolved in NMP (1.5 mL) and treated with DIC (0.5 mmol). After 2 min the solution was added to the resin and the mixture was agitated for 2 h, whereupon the resin was washed several times with DMF and DCM. Then the resin was treated with THF (1 mL), methanol (1 mL) and aqueous NaOH (2 M, 0.5 mL) for 1 h at room temperature, subsequently the resin was washed with methanol/water, DMF, methanol/water, DMF and DCM. After addition of acetonitrile (1 mL), tert-butanol (1 mL) and 2-methyl-2-butene (200 μL), the resin was cooled to 0 °C and treated dropwise with a solution of sodium chlorite (0.25 mmol) and monosodium phosphate (0.2 mmol) in water (0.2 mL). After 30 min, acetonitrile (1 mL) and tert-butanol (1 mL) were added and sodium chlorite (1 mmol) and monosodium phosphate (0.8 mmol) in water (0.4 mL) were added dropwise and the mixture was allowed to warm to room temperature. The resin was washed with methanol/water, DMF, methanol/water, DMF and DCM. After thorough drying, triphenylphosphine (1 mmol), THF (2 mL) and methanol (0.2 mL) were added, followed by diethyl azodicarboxylate (1 mmol). The resin was agitated for 1 h at room temperature, then it was washed several times with DMF and dichloromethane. THF (1 mL), methanol (1 mL) and aqueous NaOH (2 M, 0.5 mL) were added and the mixture was shaken for four days at room temperature, subsequently the resin was washed with methanol/water, DMF, methanol/water, DMF and DCM. A solution of HATU (0.25 mmol) and DIPEA (0.5 mmol) in NMP (1 mL) was added and the mixture was shaken for 15 min at room temperature. Then 3-amino-1-methylindazole (0.25 mmol) in NMP (0.5 mL) was added and the reaction mixture was shaken over night, followed by washing with DMF and dichloromethane. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 382 (M+1).

**(RS)-5-[1-(2,3-Dihydroxy-1-propyl)indazol-3-ylcarboxamido]-2-methoxybenzoic acid (3-amino-1-propyl)amide (L40)**

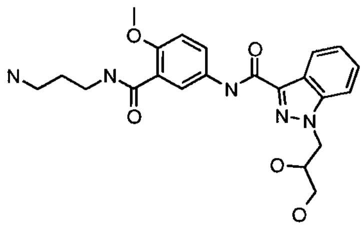

Trityl polystyrene resin preloaded with 1,3-diaminopropane (150 μmol) was pre-swollen with NMP. Fmoc-5-amino-2-methoxybenzoic acid (200 μmol) and HATU (200 μmol) in NMP (1.5 mL) were treated with DIPEA (400 μmol). After two minutes, the solution was added to the resin and the mixture was shaken at room temperature for 3 h. After washing the resin with DMF, piperidine (25% in DMF, approx. 2 mL) was added and the resin was shaken for approx. 30 min, followed by thorough washing with DMF and dichloromethane. Indazole-3-carboxylic acid (0.5 mmol) and HOBt (0.5 mmol) were dissolved in NMP (1.5 mL). DIC (0.5 mmol) was added and the solution was added to the resin after 2 min. The mixture was shaken for 1 h, afterwards the resin was washed with DMF and dichloromethane. Caesium carbonate (1 mmol) and dry DMF (1.5 mL) were added to the resin, followed by allyl bromide (1 mmol). The mixture was shaken over night, followed by washing with DMF, water, methanol and dichloromethane. The caesium carbonate/allyl bromide step was repeated once over night, then the resin was washed thoroughly, dried and transferred to a glass vial. N-Methylmorpholine oxide (0.5 mmol) in acetone (2 mL) and water (0.5 mL) were added to the resin. Osmium-(VIII)-oxide (50 μL of a 2.5% solution in tert-butanol) was added and the vial was closed firmly and shaken at room temperature over night. After addition of another portion of osmium-(VIII)-oxide (50 μL of a 2.5% solution in tert-butanol) and further shaking for 2 h, the reaction was quenched with aqueous sodium dithionite (174 mg in 1 mL of water) and shaken for 30 min. Subsequently, the resin was washed several times with water, DMF, water, methanol, DMF and dichloromethane. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 442 (M+1).

**5-(3H-Imidazo[4,5-b]pyridin-2-ylcarboxamido)-2-methoxybenzoic acid (3-amino-1-propyl)amide (L41)**

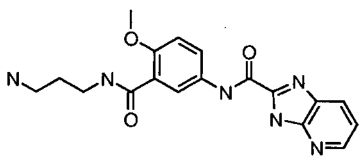

Trityl polystyrene resin preloaded with 1,3-diaminopropane (150 μmol) was pre-swollen with NMP. Fmoc-5-amino-2-methoxybenzoic acid (200 μmol) and HATU (200 μmol) in NMP (1.5 mL) were treated with DIPEA (400 μmol). After two minutes, the solution was added to the resin and the mixture was shaken at room temperature for 2 h. After washing the resin with DMF and dichloromethane, piperidine (25% in DMF, approx. 2 mL) was added and the resin was shaken for approx. 30 min, followed by thorough washing with DMF and dichloromethane. The resin was swollen in dichloromethane (1 mL) and DIPEA (1 mmol) was added, followed by oxalic acid monoethyl ester monochloride (0.5 mmol) in dichloromethane (1 mL). After shaking for 5 min, the resin was washed with DMF, methanol and dichloromethane (three times each). 2,3-Diaminopyridine (0.5 mmol) in NMP (2 mL) was added and the mixture was shaken at approx. 100 °C for four days. After washing the resin (DMF, methanol, dichloromethane), the reaction was repeated at approx. 120 °C over night. After washing as described before, THF (1 mL), methanol (0.5 mL) and aqueous NaOH (2 M, 0.5 mL) were added to the resin and the mixture was agitated for 15 min, whereupon the resin was washed with methanol (five times) and dichloromethane (three times). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 369 (M+1).

**2-Methoxy-5-([1,2,4]triazolo[4,3-a]pyridin-3-ylcarboxamido)benzoic acid (3-amino-1-propyl)amide (L43)**

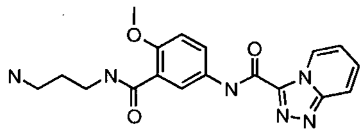

Trityl polystyrene resin preloaded with 1,3-diaminopropane (150 µmol) was pre-swollen in NMP. Fmoc-5-Amino-2-methoxybenzoic acid (200 µmol) and HATU (200 µmol) in NMP (1.5 mL) were treated with DIPEA (400 µmol) and after 2 min the mixture was added to the resin which was agitated for 3 h. After washing the resin with DMF, piperidine (25% in DMF, approx. 2 mL) was added and the resin was shaken for 30 min. Subsequently, the resin was washed thoroughly with DMF and dichloromethane and swollen in dichloromethane. Dichloromethane (1.5 mL), DIPEA (1 mmol) and oxalic acid monoethyl ester monochloride (0.5 mmol) were added and the mixture was agitated for 10 min, then the resin was washed several times with DMF and dichloromethane. Ethylene glycol (1 mL), 2-pyridylhydrazine (1 mmol) and DIPEA (1 mmol) were added and the mixture was heated to 100 °C for 4 h, then the resin was washed several times with DMF and dichloromethane. Triphenylphosphine (0.5 mmol), N-methylmorpholine (0.5 mmol) and dichloromethane (2 mL) were added to the resin, followed by drop-wise addition of trichloroacetonitrile (0.4 mmol). After shaking the mixture for 48 h, a solution of triphenylphosphine (0.5 mmol) and N-methylmorpholine (1 mmol) in dichloromethane (2 mL) were added along with tetrachlorocarbon (0.5 mL). The mixture was kept at 40 °C for 2 h, then THF (2 mL) and aqueous sodium carbonate (2 M, 0.5 mL) were added and the mixture was agitated over night. Finally, the resin was washed thoroughly with DMF, water, methanol and dichloromethane. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 369 (M+1).

**(S)-2,6-Bis[5-(Imidazo[2,1-b]thiazol-6-ylcarboxamido)-2-methoxyphenylcarboxamido]hexanoic acid (3-amino-1-propyl)amide (L49)**

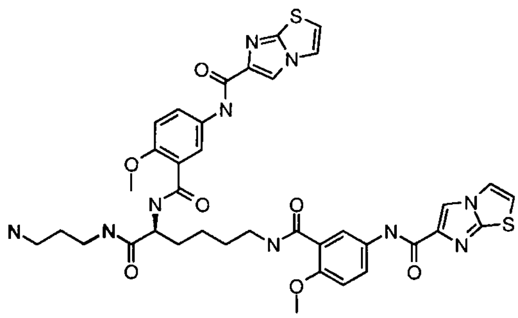

Trityl polystyrene resin preloaded with 1,3-diaminopropane (150 µmol) was swollen in NMP. Then (S)-bis-Fmoc-lysine (200 µmol) and HATU (200 µmol) were dissolved in NMP (approx. 1.5 mL) and treated with DIPEA (400 µmol) for 2 min, whereupon the solution was added to the resin and the mixture was agitated at room temperature. After 1 h the resin was washed with DMF and piperidine (25% in DMF, 2 mL) was added and the resin was agitated for 30 min. Subsequently, the resin was washed (DMF, methanol and dichloromethane). Fmoc-5-Amino-2-methoxybenzoic acid (400 µmol) and HATU (400 µmol) were dissolved in NMP (approx. 2 mL) and treated with DIPEA (800 µmol) for 2 min, whereupon the solution was added to the resin and the mixture was shaken for 1 h. After washing the resin with DMF, deprotection was achieved by treatment with 25% piperidine in DMF, followed by washing as described before. Imidazo[2,1-b]thiazole-6-carboxylic acid (400 µmol) and HATU (400 µmol) were dissolved in NMP (approx. 2 mL) and treated with DIPEA (800 µmol) for 2 min, whereupon the solution was added to the resin. After shaking the mixture for 1 h, the resin was washed with DMF. methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 801 (M+1), 401 ((M+2)/2).

**(S)-2,6-Bis{8-[5-(Imidazo[2,1-b]thiazol-6-ylcarboxamido)-2-methoxyphenylcarboxamido]-3,6-dioxaoctanoylamido}hexanoic acid (3-amino-1-propyl)amide (L50)**

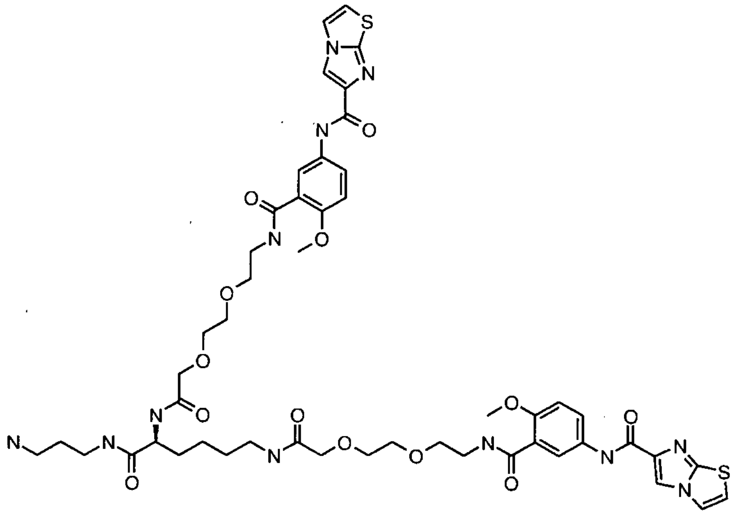

Trityl polystyrene resin preloaded with 1,3-diaminopropane (150 μmol) was swollen in NMP. Then (S)-bis-Fmoc-lysine (200 μmol) and HATU (200 μmol) were dissolved in NMP (approx. 1.5 mL) and treated with DIPEA (400 μmol) for 2 min, whereupon the solution was added to the resin and the mixture was agitated at room temperature. After 1 h the resin was washed with DMF and piperidine (25% in DMF, 2 mL) was added and the resin was agitated for 30 min. Subsequently, the resin was washed (DMF, methanol and dichloromethane). Fmoc-8-Amino-3,6-dioxaoctanoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Fmoc-5-Amino-2-methoxybenzoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Imidazo[2,1-b]thiazole-6-carboxylic acid (400 μmol) and HATU (400 μmol) were dissolved in NMP (approx. 2 mL) and treated with DIPEA (800 μmol) for 2 min, whereupon the solution was added to the resin. After shaking the mixture for 1 h, the resin was washed with DMF, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 547 ((M+2)/2).

**5-[4-(2-Furyl)pyridin-2-ylcarboxamido]-2-methoxybenzoic acid (3-amino-1-propyl)amide (L52)**

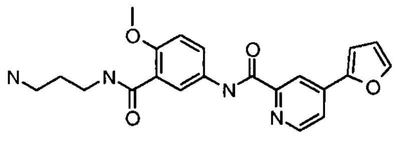

Trityl polystyrene resin preloaded with 1,3-diaminopropane (150 μmol) was pre-swollen in NMP. Fmoc-5-Amino-2-methoxybenzoic acid (200 μmol) and HATU (200 μmol) in NMP (1.5 mL) were treated with DIPEA (400 μmol), and after 2 min the solution was added to the resin. After shaking for 105 min, the resin was washed with DMF and subsequently treated with piperidine (25% in DMF, 2 mL) for 1 h, followed by thorough washing with DMF, methanol and dichloromethane. 4-Bromopyridine-2-carboxylic acid (0.3 mmol) and HOAt (0.3 mmol) in NMP (3 mL) were treated with DIC (0.3 mmol). After 2 min the solution was added to the resin and the mixture was shaken for 1 h, whereupon the resin was washed as described before, dried thoroughly with a stream of dry nitrogen and transferred to a glass vial. Caesium

carbonate (0.3 mmol) and 2-furylboronic acid (0.5 mmol) were added along with DMF (2 mL). The mixture was flushed thoroughly with argon. After addition of tetrakis-triphenylphosphinylpalladium-0 (10 μmol), the mixture was once again flushed with argon, then the vial was closed tightly and the mixture was agitated at 100 °C over night. Subsequently, the resin was washed with DMF, water, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 395 (M+1).

**(S)-2,6-Bis{5-[5-(Imidazo[2,1-b]thiazol-6-ylcarboxamido)-2-methoxyphenylcarboxamido]-3-oxapentanoylamido}hexanoic acid (3-amino-1-propyl)amide (L53)**

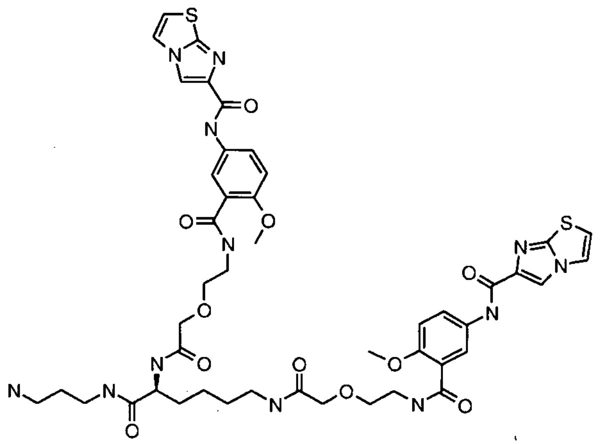

Trityl polystyrene resin preloaded with 1,3-diaminopropane (150 μmol) was swollen in NMP. Then (S)-bis-Fmoc-lysine (200 μmol) and HATU (200 μmol) were dissolved in NMP (approx. 1.5 mL) and treated with DIPEA (400 μmol) for 2 min, whereupon the solution was added to the resin and the mixture was agitated at room temperature. After 1 h the resin was washed with DMF and piperidine (25% in DMF, 2 mL) was added and the resin was agitated for 30 min. Subsequently, the resin was washed (DMF, methanol and dichloromethane). Fmoc-5-Amino-3-oxapentanoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Fmoc-5-Amino-2-methoxybenzoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Imidazo[2,1-b]thiazole-6-carboxylic acid (400 μmol) and HATU (400 μmol) were dissolved in NMP (approx. 2 mL) and treated with DIPEA (800 μmol) for 2 min, whereupon the solution was added to the resin. After shaking the mixture for 1 h, the resin was washed with DMF, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 503 ((M+2)/2).

**2-Methoxy-5-[3-(4-methoxyphenyl)-1,2,4-oxadiazol-5-ylcarboxamido]benzoic acid (3-amino-1-propyl)amide (L54)**

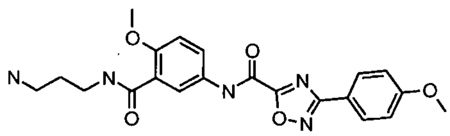

Trityl polystyrene resin preloaded with 1,3-diaminopropane (450 µmol, approx. 0.5 g) was pre-swollen in dichloromethane, followed by NMP. Fmoc-5-Amino-2-methoxybenzoic acid (0.6 mmol) and HATU (0.6 mmol) in NMP (4.5 mL) were treated with DIPEA (1.2 mmol) for 2 min. Subsequently the solution was added to the resin and the mixture was agitated for 40 min prior to threefold washing with DMF. After addition of piperidine (25% in DMF, approx. 6 mL), the resin was agitated for 30 min and subsequently washed with DMF, methanol and dichloromethane (three times each). After drying the resin in high vacuum, dichloromethane (3 mL) and DIPEA (3 mmol) were added and oxalic acid monoethyl ester monochloride (1.5 mmol) in dichloromethane was added cautiously. After shaking the mixture for 2 min, the resin was washed with dichloromethane, methanol and dichloromethane (three times each). After drying the resin, 150 mg were taken out. The remaining amount was treated with THF (4 mL), methanol (2 mL) and aqueous NaOH (2 M, 2 mL) for 2 h at room temperature. The resin was washed (DMF, acetic acid [2.5% in DMF], methanol and dichloromethane; three times each). Of this resin, 150 mg were reacted with HOAt (0.5 mmol) and DIC (0.5 mmol) in NMP (1 mL) for 2 min. Subsequently, 4-methoxy-N-hydroxybenzamidine (0.5 mmol) in NMP (1 mL) was added. After agitating the resin for 1 h, DIC (0.5 mmol) was added and the mixture was shaken over night. After washing the resin with DMF, methanol and dichloromethane (three times each), the coupling step was repeated. 3 h after the second addition of DIC, the resin was washed as described before, dried and transferred to a glass vial. NMP (2 mL) and DIC (0.5 mmol) were added and the mixture was heated to 120 °C for 30 min. Then the resin was washed as described before. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 426 (M+1).

**5-[3-(2-Furyl)-1,2,4-oxadiazol-5-ylcarboxamido]-2-methoxybenzoic acid (3-amino-1-**propyl)amide (L55)

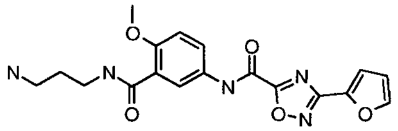

**Synthesis of N-hydroxy-2-furylcarboxamidine:** To a stirred solution of 2-cyanofuran (5 mmol) in ethanol (5 mL) was added aqueous hydroxylamine (50%, 5 mmol), whereupon slight heating of the reaction mixture was observed. After approx. 20 min, additional aqueous hydroxylamine (0.5 mmol) was added. After stirring for 1 h, the solvent was evaporated with a stream of nitrogen and the residue was purified by flash column chromatography ($NH_2$-modified stationary phase, 0-5% methanol in dichloromethane, 1% triethylamine), furnishing a slightly turbid oil that solidified upon standing.

**Synthesis of the target compound:** Dry 2-chlorotrityl chloride resin (150 µmol) was treated with 1,3-diaminopropane (2 mL), followed by dichloromethane (2 mL). After 5 min the resin was washed with DMF, methanol and dichloromethane (three times each) and swollen in NMP. Fmoc-5-Amino-2-methoxybenzoic acid (200 µmol) and HATU (200 µmol) in NMP (1.5 mL) were treated with DIPEA (400 µmol), and after 2 min the solution was added to the resin which was shaken at room temperature for 30 min. The resin was washed with DMF (three times) and treated with piperidine (25% in DMF, approx. 4 mL) for 30 min, whereupon the resin was washed with DMF, methanol and dichloromethane (three times each) and swollen in dichloromethane. After addition of dichloromethane (2 mL) and DIPEA (1 mmol), a solution of oxalic acid monoethyl ester monochloride (0.5 mmol) in dichloromethane (1 mL) was added cautiously and the mixture was agitated for 5 min, whereupon the resin was washed with DMF, methanol and dichloromethane (three times each), dried and transferred to a glass vial. Potassium carbonate (0.5 mmol), toluene (2 mL) and N-hydroxy-2-furylcarboxamidine (0.5 mmol; synthesis: see above) were added and the vial was firmly closed and agitated at 100 °C over night and at 110 °C for 2 h, whereupon the resin was washed with DMF, methanol and dichloromethane (three times each). The target compound was cleaved from the support by twofold treatment with trifluoroacetic acid (45%) and triethylsilane (10%) in dichloromethane, followed by thorough rinsing of the resin with dichloromethane and removal of the solvents with a stream of nitrogen. The residue was purified by preparative reverse-phase HPLC. ESI-MS: 386 (M+1).

**5-[5-(2-Furyl)-1,3,4-oxadiazol-2-yl]carboxamido-2-methoxybenzoic acid (3-amino-1-propyl)amide (L58)**

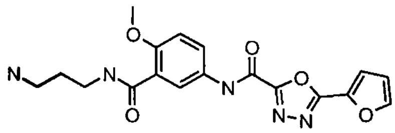

Trityl polystyrene resin preloaded with 1,3-diaminopropane (150 μmol) was pre-swollen with NMP, followed by removal of excess solvent. Fmoc-5-amino-2-methoxybenzoic acid (200 μmol) and HATU (200 μmol) in NMP (1.5 mL) were treated with DIPEA (400 μmol). After two minutes, the solution was added to the swollen resin and the mixture was shaken at room temperature for 2 h. After washing the resin with DMF, piperidine (25% in DMF, approx. 2 mL) was added and the resin was shaken for approx. 30 min, followed by thorough washing with DMF and dichloromethane. Next, the resin was swollen in dichloromethane and treated with a solution of oxalic acid monoethyl ester monochloride (0.5 mmol) and DIPEA (1 mmol) in dichloromethane (1.5 mL) for 10 min, followed by repeated washing with DMF and dichloromethane. After swelling the resin in THF (1.5 mL), hydrazine hydrate (0.5 mL) was added and the resin was agitated for 3 h at room temperature, followed by washing with DMF and dichloromethane. Furan-2-carboxylic acid (0.5 mmol) and HOAt (0.5 mmol) were dissolved in NMP (1.5 mL) and treated with DIC (0.5 mmol). After 2 min the solution was added to the resin and the mixture was agitated at room temperature for 2 h, followed by washing with DMF and dichloromethane. Subsequently, a solution of triphenylphosphine (0.5 mmol) and N-methylmorpholine (1 mmol) in dichloromethane (2 mL) was added, followed by tetrachlorocarbon (0.5 mL). The mixture was heated to 40 °C for 2 h in a thoroughly closed glass vial. After addition of THF (2 mL) and aqueous $Na_2CO_3$ (2 M, 0.5 mL), the mixture was agitated over night at room temperature, followed by washing with DMF, water, methanol and dichloromethane. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 386 (M+1).

**5-[1-(2-Hydroxyethyl)indazol-3-ylcarboxamido]-2-methoxybenzoic acid (3-amino-1-propyl)amide (L59)**

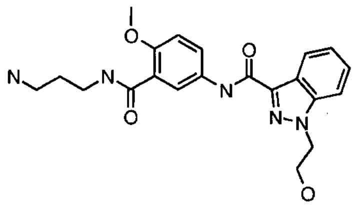

Trityl polystyrene resin preloaded with 1,3-diaminopropane (150 μmol) was pre-swollen with NMP. Fmoc-5-amino-2-methoxybenzoic acid (200 μmol) and HATU (200 μmol) in NMP (1.5 mL) were treated with DIPEA (400 μmol). After two minutes, the solution was added to the resin and the mixture was shaken at room temperature for 1 h. After washing the resin with DMF, piperidine (25% in DMF, approx. 2 mL) was added and the resin was shaken for 1 h, followed by thorough washing with DMF, methanol and dichloromethane. Indazole-3-carboxylic acid (0.5 mmol) and HOBt (0.5 mmol) were dissolved in NMP (1.5 mL). DIC (0.5 mmol) was added and after 2 min the solution was added to the resin. The mixture was shaken for 40 min, afterwards the resin was washed with DMF, methanol and dichloromethane (three times each). The resin was transferred to a glass vial and a solution of bromoacetic acid methyl ester (0.5 mmol) and DIPEA (1 mmol) in NMP (2 mL) was added. The vial was closed thoroughly and shaken at 80 °C for 75 min. Subsequently, the resin was washed (DMF, methanol, dichloromethane, three times each), swollen in THF (2 mL) and methanol (0.5 mL) and treated with sodium borohydride (large excess, added in portions). After the last addition, the mixture was shaken for 30 min at room temperature, then the resin was washed with methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 412 (M+1).

**8-[2-Methoxy-5-(1-methylindazol-3-ylcarboxamido)phenylcarboxamido]-3,6-dioxaoctanoic acid (3-amino-1-propyl)amide (L60)**

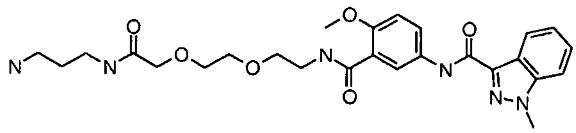

Trityl polystyrene preloaded with 1,3-diaminopropane (150 μmol) was pre-swollen in NMP. Fmoc-8-Amino-3,6-dioxaoctanoic acid (200 μmol) and HATU (200 μmol) in NMP (1.5 mL) were treated with DIPEA (400 μmol) and after 2 min the solution was added to the resin which was shaken at room temperature for 1 h, whereupon it was washed with DMF (three times). Piperidine (25% in DMF, approx. 2 mL) was added and the mixture was shaken for approx. 30 min. The resin was washed with DMF, methanol and dichloromethane (three times each). Next, the coupling/deprotection protocol was applied to Fmoc-5-amino-2-methoxybenzoic acid (same amount as described before). Finally, 1-methylindazole-3-carboxylic acid was coupled using the HATU coupling protocol described above. The resin was washed with DMF, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 527 (M+1).

**8-{8-[2-Methoxy-5-(1-methylindazol-3-ylcarboxamido)phenylcarboxamido]-3,6-dioxaoctanoylamido}-3,6-dioxaoctanoic acid (3-amino-1-propyl)amide (L61)**

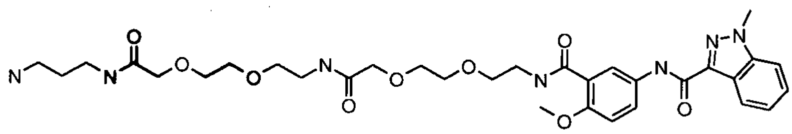

Trityl polystyrene preloaded with 1,3-diaminopropane (150 μmol) was pre-swollen in NMP. Fmoc-8-Amino-3,6-dioxaoctanoic acid (200 μmol) and HATU (200 μmol) in NMP (1.5 mL) were treated with DIPEA (400 μmol) and after 2 min the solution was added to the resin which was shaken at room temperature for 1 h, whereupon it was washed with DMF (three times). Piperidine (25% in DMF, approx. 2 mL) was added and the mixture was shaken for approx. 30 min. The resin was washed with DMF, methanol and dichloromethane (three times each). Next, the coupling/deprotection protocol was repeated with Fmoc-8-amino-3,6-dioxaoctanoic acid, afterwards it was applied to Fmoc-5-amino-2-methoxybenzoic acid (same amounts as described before). Finally, 1-methylindazole-3-carboxylic acid was coupled using the HATU coupling protocol described above. The resin was washed with DMF, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 672 (M+1).

**(S)-2,6-Bis{8-[5-(Imidazo[1,2-b]pyridazin-2-ylcarboxamido)-2-methoxyphenylcarboxamido]-3,6-dioxaoctanoylamido}hexanoic acid (3-amino-1-propyl)amide (L62)**

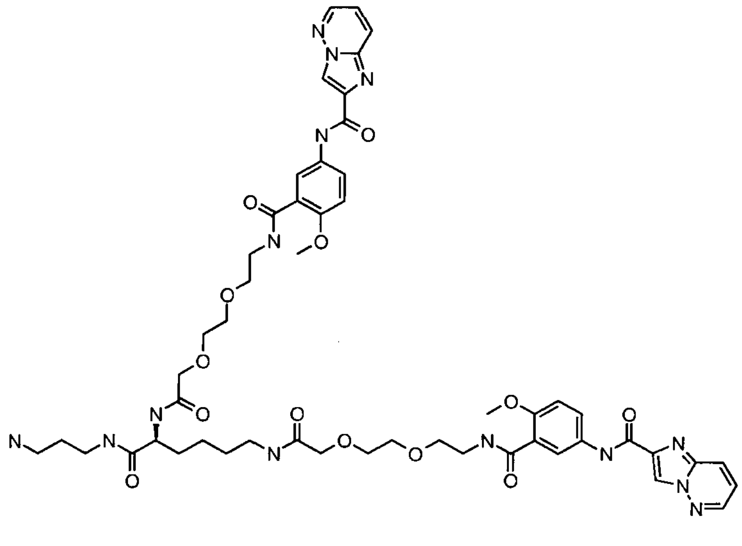

Trityl polystyrene resin preloaded with 1,3-diaminopropane (150 μmol) was swollen in NMP. (S)-Bis-Fmoc-lysine (200 μmol) and HATU (200 μmol) were dissolved in NMP (approx. 1.5 mL) and treated with DIPEA (400 μmol) for 2 min, whereupon the solution was added to the resin and the mixture was agitated at room temperature. After 1 h the resin was washed with DMF and piperidine (25% in DMF, 2 mL) was added and the resin was agitated for 30 min. Subsequently, the resin was washed (DMF, methanol and dichloromethane). Fmoc-8-Amino-3,6-dioxaoctanoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Fmoc-5-Amino-2-methoxybenzoic acid (400 μmol) and HATU (400 μmol) were dissolved in NMP (approx. 2 mL) and treated with DIPEA (800 μmol) for 2 min, whereupon the solution was added to the resin and the mixture was shaken for 1 h. After washing the resin with DMF, deprotection was achieved by treatment with 25% piperidine in DMF, followed by washing as described before. Imidazo[1,2-b]pyridazine-2-carboxylic acid (400 μmol) and HATU (400 μmol) were dissolved in NMP (approx. 2 mL) and treated with DIPEA (800 μmol) for 2 min, whereupon the solution was added to the resin. After shaking the mixture for 1 h, the resin was washed with DMF, methanol and dichloromethane (three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 542 ((M+2)/2).

**(S)-1-Aminopropane-1,3-dicarboxylic acid bis{3-[5-(imidazo[2,1-b]thiazol-6-ylcarboxamido)-2-methoxyphenyl-carboxamido]propyl}amide (L64)**

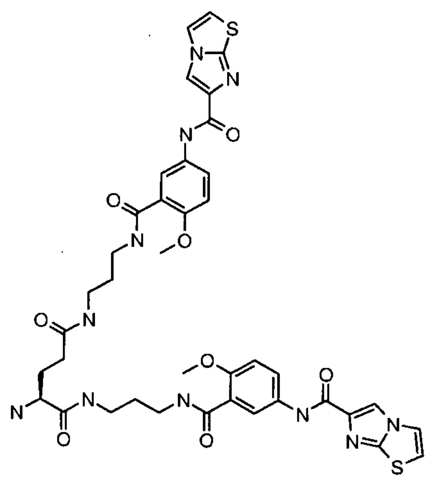

**Synthesis of 5-amino-2-methoxybenzoic acid methyl ester:** 2-methoxy-5-nitrobenzoic acid methyl ester (1 mmol) and palladium(0) on charcoal (5%, 25 mg [containing approx. 50% w/w water]) in methanol (5 mL) were treated drop wise with triethylsilane (1 mL) over 10 min, followed by methanol (2 mL). After agitating for additional 5 min, the mixture was filtered through celite, followed by thorough rinsing of the celite layer. The filtrate was concentrated, dried in high vacuum and the residue was used without further purification. **Solution-phase synthesis of the aminopropyl-linked ligand (identical to L37):** imidazo[2,1-b]thiazole-6-carboxylic acid (1 mmol) and TBTU (1 mmol) in DMF (2 mL) were treated with DIPEA (2 mmol) for 5 min. Subsequently, the mixture was added to the complete amount of 5-amino-2-methoxybenzoic acid methyl ester obtained before, and the mixture was agitated for 30 min. After addition of saturated aqueous sodium carbonate (1 mL) and water (3 mL) the mixture was extracted four times with ethyl acetate. The combined organic layers were washed twice with aqueous citric acid (5%), once with saturated aqueous sodium carbonate and twice with water. The remaining organic layer was concentrated and dried over night with a stream of dry nitrogen. 1,3-Diaminopropane (2 mL) was added to the residue and the mixture was heated to 80 °C for 4 h. Subsequently, the solution was concentrated to dryness, the residue was re-dissolved in methanol, concentrated and dried in high vacuum, furnishing an oil that solidified upon refrigerating. Yield: 453 μmol (45%).

**Synthesis of the target compound:** N-Boc-glutamic acid (227 μmol) and TBTU (453 μmol) were dissolved in DMF (2 mL) and treated with DIPEA (906 μmol) for 3 min, then the solution was added to the residue obtained before. After 90 min, another portion of TBTU-activated N-Boc-glutamic acid (0.2 mmol) in DMF (1 mL; activation for 2 min as described before) was added. After 45 min the solvent was evaporated over night with a stream of nitrogen. The residue was re-dissolved in methanol, evaporated to dryness and treated with trifluoroacetic acid (25% in dichloromethane), followed by evaporation to dryness and purification of the residue by preparative reverse-phase HPLC. ESI-MS: 858 (M+1), 430 ((M+2)/2).

**General Procedure D: Synthesis of 5-amino-2-methoxybenzoic acid linked to two Ado units and one 1,3-diaminopropane unit (base resin)**

Trityl polystyrene resin preloaded with 1,3-diaminopropane was pre-swollen in DCM and NMP. Fmoc-8-Amino-3,6-dioxaoctanoic acid (200 μmol) and HATU (200 μmol) in NMP (1.5 mL) were treated with DIPEA (400 μmol) for 2 min, then the solution was added to the resin and the mixture was agitated for 30 min to several hours. After washing the resin with DMF or DMF, methanol and dichloromethane, piperidine (25% in DMF, approx. 2 mL) was added and the resin was agitated for at least 20 min. Subsequently, the resin was washed thoroughly with DMF, methanol and dichloromethane (three times each) and the coupling/deprotection procedure was repeated once. Fmoc-5-Amino-2-methoxybenzoic acid (200 μmol) was coupled to the resin employing the HATU-coupling protocol described before, followed by deprotection with piperidine as described before. The resin was dried at air prior to additional synthetic steps.

**8-{8-{5-[5-(2-Furyl)-1-methylpyrazol-3-ylcarboxamido]-2-methoxyphenylcarboxamido}-3,6-dioxaoctanoylamido}-3,6-dioxaoctanoic acid (3-amino-1-propyl)amide (L65)**

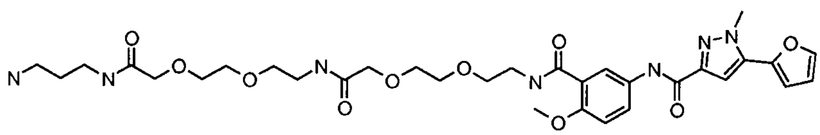

5-(2-Furyl)-1-methylpyrazole-3-carboxylic acid (200 μmol) and HATU (200 μmol) in NMP (1.5 mL) were treated with DIPEA (400 μmol) for 2 min. Subsequently, the solution was added to the base resin obtained by General Procedure D. After agitating the mixture for approx. 2 h, the resin was washed (DMF, methanol, dichloromethane, three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 688 (M+1).

**8-{8-[5-(Imidazo[2,1-b]thiazol-6-ylcarboxamido)-2-methoxyphenylcarboxamido]-3,6-dioxaoctanoylamido}-3,6-dioxaoctanoic acid (3-amino-1-propyl)amide (L66)**

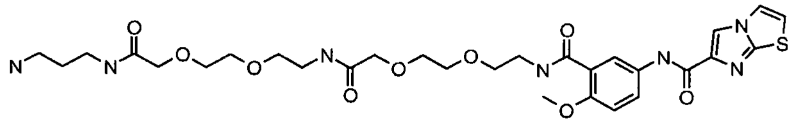

Imidazo[2,1-b]thiazole-6-carboxylic acid (200 μmol) and HATU (200 μmol) in NMP (1.5 mL) were treated with DIPEA (400 μmol) for 2 min. Subsequently, the solution was added to the base resin obtained by General Procedure D. After agitating the mixture for approx. 2 h, the resin was washed (DMF, methanol, dichloromethane, three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 664 (M+1).

**8-{8-{5-[5-(2-Furyl)-1,3,4-oxadiazol-2-ylcarboxamido]-2-methoxyphenylcarboxamido}-3,6-dioxaoctanoylamido}-3,6-dioxaoctanoic acid (3-amino-1-propyl)amide (L67)**

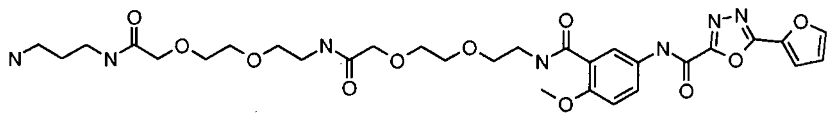

The dry base resin obtained by General Procedure D was treated with dichloromethane (2 mL) and DIPEA (1 mmol), followed by drop-wise addition of oxalic acid monoethyl ester monochloride (0.5 mmol) in dichloromethane. The mixture was agitated for 10 min at room temperature, then the resin was washed with DMF, methanol and dichloromethane (three times each). Subsequently, the resin was swollen in NMP (2 mL) and treated with hydrazine hydrate (1 mmol) for 50 min at room temperature, followed by washing as described before. 2-Furoic acid (200 μmol) and HATU (200 μmol) in NMP (1.5 mL) were treated with DIPEA (400 μmol) for 2 min, then the solution was added to the resin. After approx. 1 h the resin was washed as described before. Next, 4-toluenesulfonyl chloride (0.5 mmol) and DIPEA (1 mmol) in dichloromethane (approx. 2 mL) was added and the mixture was agitated until cyclization was completed. The resin was washed as described before. The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 676 (M+1).

**8-{8-{2-Methoxy-5-[5-(2-methylthiazol-4-yl)isoxazol-3-ylcarboxamido]phenylcarboxamido}-3,6-dioxaoctanoylamido}-3,6-dioxaoctanoic acid (3-amino-1-propyl)amide (L68)**

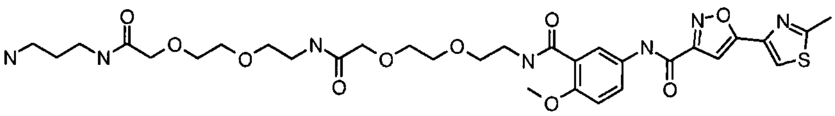

5-(2-Methylthiazol-4-yl)isoxazole-3-carboxylic acid (200 μmol) and HATU (200 μmol) in NMP (1.5 mL) were treated with DIPEA (400 μmol) for 2 min. Subsequently, the solution was added to the base resin obtained by General Procedure D. After agitating the mixture for approx. 2 h, the resin was washed (DMF, methanol, dichloromethane, three times each). The target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 706 (M+1).

**N-(8-Amino-3,6-dioxaoctyl)-*N'*-{8-[5-(imidazo[2,1-b]thiazol-6-ylcarboxamido)-2-methoxyphenylcarboxamido]-3,6-dioxaoctyl}urea (L69)**

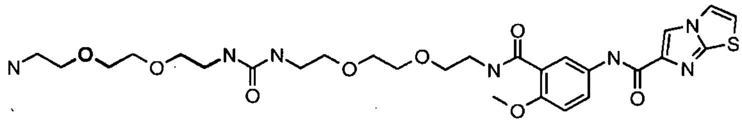

To 2-chlorotrityl chloride polystyrene resin (150 μmol) was added 1,8-diamino-3,6-dioxaoctane (2 mL). After shaking the mixture for 5 min, dichloromethane (1 mL) was added to ensure sufficient swelling. After 25 min the resin was washed (DMF, methanol, dichloromethane, three times each). The resin was swollen in dichloromethane, then dichloromethane (2 mL) and DIPEA (1 mmol) were added, followed by chloroformic acid (4-nitrophenyl)ester (0.5 mmol) in dichloromethane (1 mL). The mixture was agitated for 5 min, then the resin was filtered off and immediately treated with 1,8-diamino-3,6-dioxaoctane (2 mL) for 20 min. Then the resin was washed as described before. Fmoc-5-Amino-2-methoxybenzoic acid (200 μmol) and HATU (200 μmol) in NMP/DCM (1.5+1.5 mL) was treated with DIPEA (400 μmol) for 2 min, subsequently the solution was added to the resin. After agitating the mixture for 5 h the resin was washed as described before and treated with piperidine (25% in DMF, approx. 2 mL) for 30 min, followed by washing as described before. Finally, imidazo[2,1-b]thiazole-6-carboxylic acid (200 μmol) was coupled employing the HATU coupling protocol described before (neat NMP, approx. 1.5 mL; reaction time: over night), followed by washing of the resin as described before. The target compound was cleaved from the support by twofold treatment with trifluoroacetic acid (45%) and triethylsilane (10%) in dichloromethane, followed by thorough washing of the resin with dichloromethane. After evaporation of the solvents, the crude product was purified by reverse-phase HPLC. ESI-MS: 622 (M+1), 312 ((M+2)/2).

**General Procedure E: Synthesis of isophthalic acid N-(3-amino-1-propyl)-*N'*-aryl amides**

Dry trityl polystyrene resin preloaded with 1,3-diaminopropane (150 μmol) was treated with isophthaloyl chloride (1 mmol) and DIPEA (2 mmol) in dichloromethane (1.5 mL) for 3 min. The resin was washed quickly with dichloromethane or NMP (two or three times). Subsequently, the respective aromatic amine (0.3 mmol) and DIPEA (0.3 mmol) in NMP (1.5 mL) were added and the mixture was agitated for 1 h prior to washing with DMF, methanol and dichloromethane (three times each). The target compounds were cleaved from the support by twofold treatment with trifluoroacetic acid (10%) and triethylsilane (5%) in dichloromethane, followed by thorough washing of the resin with dichloromethane. Due to the rather poor solubility of the compounds, methanol needed to be added to the cleavage or washing solutions in some cases. After evaporation of the solvents, the crude products were purified by reverse-phase HPLC.

**Isophthalic acid N-(3-aminopropyl)-N'-[5-(2-furyl)-1,3,4-thiadiazol-2-yl]amide (L70)**

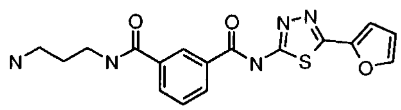

Prepared from 2-amino-5-(2-furyl)-1,3,4-thiadiazole following General Procedure E. ESI-MS: 372 (M+1).

**Isophthalic acid N-(3-aminopropyl)-N'-(1-methylindazol-3-yl)amide (L71)**

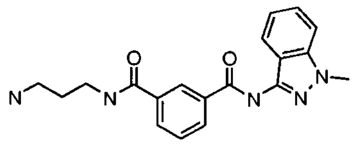

Prepared from 3-amino-1-methylindazole following General Procedure E. ESI-MS: 352 (M+1)

**Isophthalic acid N-(3-aminopropyl)-N'-(benzothiazol-2-yl)amide (L72)**

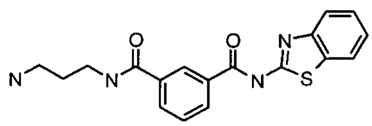

Prepared from 2-aminobenzothiazole following General Procedure E. ESI-MS: 355 (M+1).

**Isophthalic acid N-(3-aminopropyl)-*N'*-(4-phenylthiazol-2-yl)amide (L73)**

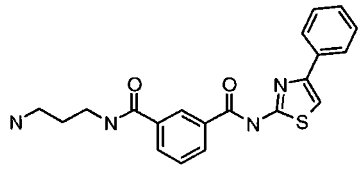

Prepared from 2-amino-4-phenylthiazole following General Procedure E. ESI-MS: 381 (M+1).

**Isophthalic acid N-(3-aminopropyl)-*N'*-(5-phenylthiazol-2-yl)amide (L74)**

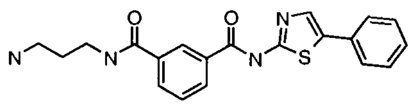

Prepared from 2-amino-5-phenylthiazole following General Procedure E. ESI-MS: 381 (M+1).

**5-(Imidazo[2,1-b]thiazol-6-ylcarboxamido)-2-methoxybenzoic acid [3-(6-aminohexanoylamido)-1-propyl]amide (L75)**

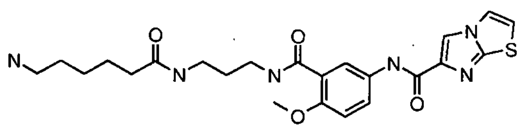

5-(Imidazo[2,1-b]thiazol-6-ylcarboxamido)-2-methoxybenzoic acid (3-amino-1-propyl)amide (L37) was prepared from imidazo[2,1-b]thiazole-6-carboxylic acid and 150 μmol of 1,3-diaminopropane-preloaded trityl polystyrene resin following General Procedure A. The crude product that was obtained by acidic cleavage of the support bound ligand was dried with a stream of nitrogen. Saturated aqueous sodium carbonate (3 mL) was added and the mixture was extracted with ethyl acetate (three times) and dichloromethane (four times). The combined organic layers were evaporated to dryness. Boc-6-Aminohexanoic acid (100 μmol) and HATU (100 μmol) in DMF (1 mL) were treated with DIPEA (200 μmol) and after 2 min the solution was added to the residue obtained before. After agitating the mixture for 20 min, water (2 mL) and saturated aqueous sodium carbonate (1 mL) were added and the mixture was extracted twice with ethyl acetate. The combined organic layers were washed with aqueous citric acid (5%, twice), saturated sodium carbonate and water, and concentrated to dryness. The residue was treated with dichloromethane (1 mL) and trifluoroacetic acid (1 mL) for 40 min. After evaporation of the solvents the crude product was purified by reverse-phase preparative HPLC. ESI-MS: 487 (M+1).

**6-[5-(Imidazo[2,1-b]thiazol-6-ylcarboxamido)-2-methoxyphenylcarboxamido]hexanoic acid (8-amino-3,6-dioxa-1-octyl)amide (L76)**

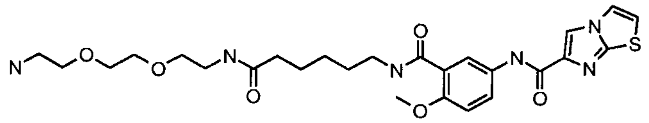

2-Chlorotrityl chloride polystyrene resin (150 μmol) was treated with 1,8-diamino-3,6-dioxaoctane (500 μL). After shaking the mixture for 3 min, dichloromethane (1 mL) was added and shaking was continued for 15 min prior to washing with DMF, methanol and dichloromethane (three times each). Subsequently the resin was swollen in NMP. Fmoc-6-Aminohexanoic acid (200 μmol) and HATU (200 μmol) in NMP (1.5 mL) were treated with DIPEA (400 μmol). After 2 min, the solution was added to the resin and the mixture was shaken for 2 h. Subsequently, the resin was washed as described before, followed by treatment with piperidine (25% in DMF, approx. 2 mL) for 1 h and washing as described before. Following this coupling/deprotection cycle, Fmoc-5-amino-2-methoxybenzoic acid was coupled to the resin (coupling time: over night; deprotection time: 30 min), followed by coupling of imidazo[2,1-b]thiazole-6-carboxylic acid (coupling time: 3 h). After final washing as described before, the target compound was cleaved from the support by twofold treatment with trifluoroacetic acid (45%) and triethylsilane (10%) in dichloromethane, followed by thorough washing of the resin with dichloromethane. After evaporation of the solvents, the crude product was purified by reverse-phase HPLC. ESI-MS: 561 (M+1).

**8-[5-(Imidazo-[2,1-b]thiazol-6-ylcarboxamido)-2-methoxyphenylcarboxamido]-3,6-dioxaoctanoic acid (3-amino-1-propyl)amide (L77)**

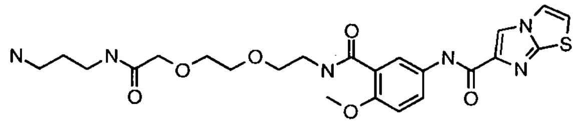

Trityl polystyrene resin preloaded with 1,3-diaminopropane (150 μmol) was pre-swollen in NMP. Fmoc-8-Amino-3,6-dioxaoctanoic acid (200 μmol) and HATU (200 μmol) in NMP (1.5 mL) were treated with DIPEA (400 μmol). After 2 min, the solution was added to the resin and the mixture was shaken for 2 h. Subsequently, the resin was washed as described before, followed by treatment with piperidine (25% in DMF, approx. 2 mL) for 1 h and washing as described before. Following this coupling/deprotection cycle, Fmoc-5-amino-2-methoxybenzoic acid was coupled to the resin (coupling time: over night; deprotection time: 30 min), followed by coupling of imidazo[2,1-b]thiazole-6-carboxylic acid (coupling time: 3 h). After final washing as described before, the target compound was cleaved from the support by twofold treatment with trifluoroacetic acid (10%) and triethylsilane (5%) in dichloromethane, followed by thorough washing of the resin with dichloromethane. After evaporation of the solvents, the crude product was purified by reverse-phase HPLC. ESI-MS: 519 (M+1).

**8-[5-(Imidazo-[2,1-b]thiazol-6-ylcarboxamido)-2-methoxyphenylcarboxamido]-3,6-dioxaoctanoic acid (6-amino-1-hexyl)amide (L78)**

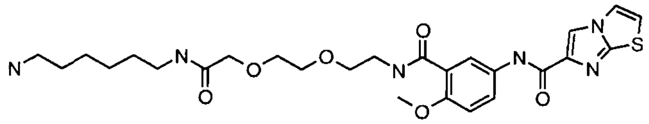

2-Chlorotrityl chloride polystyrene resin (150 μmol) was treated with 1,6-diaminohexane (500 μL). After shaking the mixture for 3 min, dichloromethane (1 mL) was added and shaking was continued for 15 min prior to washing with DMF, methanol and dichloromethane (three times each). Subsequently the resin was swollen in NMP. Fmoc-8-Amino-3,6-dioxaoctanoic acid (200 μmol) and HATU (200 μmol) in NMP (1.5 mL) were treated with DIPEA (400 μmol). After 2 min, the solution was added to the resin and the mixture was shaken for 2 h. Subsequently, the resin was washed as described before, followed by treatment with piperidine (25% in DMF, approx. 2 mL) for 1 h and washing as described before. Following this coupling/deprotection cycle, Fmoc-5-amino-2-methoxybenzoic acid was coupled to the resin (coupling time: over night; deprotection time: 30 min), followed by coupling of imidazo[2,1-b]thiazole-6-carboxylic acid (coupling time: 3 h). After final washing as described before, the target compound was cleaved from the support by twofold

treatment with trifluoroacetic acid (45%) and triethylsilane (10%) in dichloromethane, followed by thorough washing of the resin with dichloromethane. After evaporation of the solvents, the crude product was purified by reverse-phase HPLC. ESI-MS: 561 (M+1).

**Example 3: Immobilization of Ligands**

**Experimental**

Dried ligands were redissolved in DMSO at an approximate concentration of 100 mM based on gravimetric analyses. Precise concentrations of stock solutions were determined by the o-phthaldialdehyde assay (2). Dye-ligand molecules were quantitated by measuring absorbance at 340 nm. Measurements were calibrated with 1-(3-aminopropyl)imidazole. In coupling reactions, one volume of ligand dissolved at an approximate concentration of 15 to 20 mM in 90% DMSO and 10% N-Methyl-2-pyrrolidone containing 1 M N,N-Diisopropylethylamine was added to one volume of settled NHS-activated Sepharose 4 FF (GE Healthcare) equilibrated with isopropanol. Reactions were conducted for 2 h at 25 °C while shaking vigorously. The supernatant of reactions was withdrawn and resins were washed twice with appropriate solvent. Remaining active groups on resins were blocked with 1 M Ethanolamine for 1 h at 25 °C. Final resins were washed and stored in 20% ethanol at 4 °C until used in subsequent experiments. Reaction yields were determined based on mass balances after analysis of ligand concentrations in all fractions.

**Summary and Results**

Ligands from example 2 were immobilized on NHS-activated Sepharose 4 FF for subsequent chromatography and batch adsorption experiments. Coupling was achieved by formation of an amide bond between the amino group of the aminopropyl linker on ligands and the NHS-activated carboxylic group of the pre-activated resin.

The table below gives the results for coupling reactions with ligands from example 2. On average, an immobilized ligand density of 15.5 μmol per ml of resin was obtained. Reaction yields averaged at 74.5 %.

| Ligand | Initial (μml per ml gel) | Supernatant (μmol per ml gel) | Immobilized (μmol per ml gel) | Yield (%) |
|---|---|---|---|---|
| L1 | 18.8 | 7.8 | 11.0 | 58.5 |
| L2 | 18.6 | 5.4 | 13.2 | 71.0 |
| L3 | 18.0 | 6.1 | 11.9 | 66.1 |
| L4 | - | - | - | - |
| L5 | 20.6 | 6.8 | 13.8 | 67.1 |
| L6 | 21.9 | 7.7 | 14.2 | 64.9 |
| L7 | 22.1 | 8.4 | 13.7 | 62.2 |
| L8 | 21.9 | 6.9 | 15.0 | 68.3 |
| L9 | 20.7 | 7.9 | 12.8 | 62.0 |
| L10 | 22.1 | 8.0 | 14.1 | 63.6 |
| L11 | 19.0 | 4.4 | 14.6 | 76.8 |
| L12 | 19.8 | 4.5 | 15.3 | 77.2 |
| L13 | 20.1 | 4.7 | 15.4 | 76.8 |
| L14 | 20.9 | 5.5 | 15.4 | 73.6 |
| L15 | 19.5 | 4.5 | 15.0 | 77.0 |
| L16 | 21.0 | 4.4 | 16.6 | 78.9 |
| L17 | 20.8 | 4.4 | 16.4 | 78.8 |
| L18 | 19.9 | 5.0 | 14.9 | 75.0 |
| L19 | 19.6 | 6.1 | 13.5 | 68.8 |
| L20 | 21.9 | 6.3 | 15.6 | 71.3 |
| L21 | 11.8 | 5.1 | 6.7 | 56.7 |
| L22 | 19.0 | 4.1 | 14.9 | 78.4 |
| L23 | 19.5 | 3.8 | 15.7 | 80.6 |
| L24 | 20.1 | 4.6 | 15.5 | 77.2 |
| L25 | 19.3 | 4.3 | 15.0 | 77.7 |
| L26 | 20.0 | 4.4 | 15.6 | 78.1 |

(continued)

| Ligand | Initial (μml per ml gel) | Supernatant (μmol per ml gel) | Immobilized (μmol per ml gel) | Yield (%) |
|---|---|---|---|---|
| L27 | 20.5 | 5.2 | 15.3 | 74.7 |
| L28 | 22.4 | 4.4 | 18.0 | 80.4 |
| L29 | 22.6 | 2.5 | 20.1 | 88.9 |
| L30 | 22.0 | 4.0 | 18.0 | 82.0 |
| L31 | 24.7 | 2.7 | 22.0 | 89.0 |
| L32 | 24.6 | 3.3 | 21.3 | 86.7 |
| **Ligand** | **Initial (μmol per ml gel)** | **Supernatant (μmol per ml gel)** | **Immobilized (μmol per ml gel)** | **Yield (%)** |
| L33 | 22.7 | 5.7 | 17.0 | 74.9 |
| L34 | 19.0 | 4.3 | 14.7 | 77.4 |
| L35 | 23.1 | 2.7 | 20.4 | 88.2 |
| L36 | 23.8 | 4.4 | 19.4 | 81.5 |
| L37 | 23.8 | 6.8 | 17.0 | 71.3 |
| | | Averages | 15.5 | 74.5 |

| Ligand | Initial (μmol per ml gel) | Supernatant (μmol per ml gel) | Immobilized (μmol per ml gel) | Yield (%) |
|---|---|---|---|---|
| L1 | 18.8 | 7.8 | 11.0 | 58.5 |
| L2 | 18.6 | 5.4 | 13.2 | 71.0 |
| L3 | 18.0 | 6.1 | 11.9 | 66.1 |
| L4 | - | - | - | - |
| L5 | 20.6 | 6.8 | 13.8 | 67.1 |
| L6 | 21.9 | 7.7 | 14.2 | 64.9 |
| L7 | 22.1 | 8.4 | 13.7 | 62.2 |
| L8 | 21.9 | 6.9 | 15.0 | 68.3 |
| L9 | 20.7 | 7.9 | 12.8 | 62.0 |
| L10 | 22.1 | 8.0 | 14.1 | 63.6 |
| L11 | 19.0 | 4.4 | 14.6 | 76.8 |
| L12 | 19.8 | 4.5 | 15.3 | 77.2 |
| L13 | 20.1 | 4.7 | 15.4 | 76.8 |
| L14 | 20.9 | 5.5 | 15.4 | 73.6 |
| L15 | 19.5 | 4.5 | 15.0 | 77.0 |
| L16 | 21.0 | 4.4 | 16.6 | 78.9 |
| L17 | 20.8 | 4.4 | 16.4 | 78.8 |
| L18 | 19.9 | 5.0 | 14.9 | 75.0 |
| L19 | 19.6 | 6.1 | 13.5 | 68.8 |
| L20 | 21.9 | 6.3 | 15.6 | 71.3 |
| L21 | 11.8 | 5.1 | 6.7 | 56.7 |
| L22 | 19.0 | 4.1 | 14.9 | 78.4 |
| L23 | 19.5 | 3.8 | 15.7 | 80.6 |
| L24 | 20.1 | 4.6 | 15.5 | 77.2 |
| L25 | 19.3 | 4.3 | 15.0 | 77.7 |
| L26 | 20.0 | 4.4 | 15.6 | 78.1 |
| L27 | 20.5 | 5.2 | 15.3 | 74.7 |
| L28 | 22.4 | 4.4 | 18.0 | 80.4 |
| L29 | 22.6 | 2.5 | 20.1 | 88.9 |
| L30 | 22.0 | 4.0 | 18.0 | 82.0 |
| L31 | 24.7 | 2.7 | 22.0 | 89.0 |
| L32 | 24.6 | 3.3 | 21.3 | 86.7 |

(continued)

| Ligand | Initial (μmol per ml gel) | Supernatant (μmol per ml gel) | Immobilized (μmol per ml gel) | Yield (%) |
|---|---|---|---|---|
| L33 | 22.7 | 5.7 | 17.0 | 74.9 |
| L34 | 19.0 | 4.3 | 14.7 | 77.4 |
| L35 | 23.1 | 2.7 | 20.4 | 88.2 |
| L36 | 23.8 | 4.4 | 19.4 | 81.5 |
| L37 | 23.8 | 6.8 | 17.0 | 71.3 |
| L38 | 24.9 | 5.0 | 19.9 | 79.7 |
| L39 | 22.6 | 4.7 | 17.9 | 79.2 |
| L40 | 26.2 | 5.9 | 20.3 | 77.7 |
| L41 | 24.7 | 14.7 | 10.0 | 40.5 |
| L42 | 27.5 | 8.1 | 19.4 | 70.4 |
| L43 | 27.9 | 8.7 | 19.2 | 68.7 |
| L44 | 22.8 | 4.1 | 18.7 | 82.0 |
| L45 | 23.1 | 9.9 | 13.1 | 57.0 |
| L46 | 26.0 | 10.2 | 15.8 | 60.9 |
| L47 | 25.9 | 10.7 | 15.1 | 58.5 |
| L48 | 23.5 | 12.1 | 11.4 | 48.5 |
| L49 | 23.4 | 8.8 | 14.5 | 62.2 |
| L50 | 25.9 | 9.0 | 16.9 | 65.1 |
| L51 | 21.4 | 11.8 | 9.6 | 44.7 |
| L52 | 23.6 | 5.3 | 18.3 | 77.6 |
| **Ligand** | **Initial (μmol per ml gel)** | **Supernatant (μmol per ml gel)** | **Immobilized (μmol per ml gel)** | **Yield (%)** |
| L54 | 24.1 | 9.7 | 14.4 | 59.8 |
| L55 | 26.0 | 6.6 | 19.4 | 74.6 |
| L56 | 23.7 | 4.8 | 19.0 | 79.9 |
| L57 | 23.8 | 7.2 | 16.6 | 69.7 |
| L58 | 21.7 | 7.3 | 14.5 | 66.6 |
| L59 | 23.8 | 5.6 | 18.2 | 76.4 |
| L60 | 29.0 | 14.1 | 14.9 | 51.5 |
| L61 | 31.9 | 15.7 | 16.2 | 50.9 |
| L62 | 24.2 | 8.2 | 16.0 | 66.1 |
| L63 | 23.7 | 10.6 | 13.0 | 55.1 |
| L64 | 27.5 | 13.4 | 14.2 | 51.5 |
| L65 | 30.1 | 13.5 | 16.6 | 55.0 |
| L66 | 21.2 | 6.9 | 14.3 | 67.6 |
| L67 | 23.1 | 8.8 | 14.3 | 61.8 |
| L68 | 22.5 | 9.7 | 12.8 | 57.1 |
| L69 | 24.0 | 11.9 | 12.1 | 50.3 |
| L70 | 21.8 | 8.2 | 13.6 | 62.6 |
| L71 | 29.5 | 12.6 | 16.9 | 57.2 |
| L72 | - | - | - | - |
| L73 | - | - | - | - |
| L74 | - | - | - | - |
| L75 | 23.5 | 8.6 | 14.9 | 63.5 |
| L76 | 24.5 | 9.8 | 14.7 | 59.9 |
| L77 | 24.3 | 8.4 | 15.9 | 65.4 |
| L78 | 26.1 | 10.7 | 15.4 | 58.8 |
| | | Averages | 15.6 | 68.7 |

**Example 4: Chromatographic Evaluation of Ligands (from Example 2)**

**Experimental**

Resins were assessed in packed mode by microtiter plate chromatography. For each column, approximately 30 μl of resin were transferred to a 384-well filter plate and sealed with appropriate top frits. Columns with rProtein A Sepharose FF and Mabsorbent A2P HF were included as controls.

Columns were equilibrated with 6.7 column volumes (cv) of phosphate buffered saline (0.15 M NaCl, 20 mM sodium phosphate, pH 7.3; PBS) prior to injection. Either 3.3 cv of whole IgGs dissolved at 0.75 mg $ml^{-1}$, Fab or Fc fragments dissolved at 0.25 mg $ml^{-1}$ (all three in PBS) or host cell proteins (HCP) were injected onto columns. Antibody fragments of Bevacizumab (Fab and Fc) were prepared by cleavage of whole IgG with papain protease (3) according to the protocol of the manufacturer. Unbound protein was washed from columns with 5 cv of PBS prior to elution with 5 cv of glycine buffer at pH 2.5. Volumes transferred were spun through columns at 50 g for 1 - 2 min. During injection of samples, acceleration was reduced to 10 g and centrifugation time increased to 5 - 10 min. Effluent fractions were collected in 384-well plates and concentrations of protein analyzed by Bradford assay. Protein masses $m_i$, $m_{ft}$ and $m_e$ (see below) were calculated as the product of fraction volume and measured protein concentration.

**Summary and Results**

Binding and elution of several antibodies was demonstrated. Among them were the three humanized therapeutic antibodies Bevacizumab, Tocilizumab and Palivizumab and a human poly-IgG (h-poly-IgG) mixture isolated from human serum. In some cases, purified Bevacizumab Fab and Fc fragments or a mixture of rabbit IgGs (h-poly-IgG, isolated from serum) were used as additional feed. Selectivity of ligands toward antibodies was determined by investigation of binding behavior of host cell proteins. Results for commercial resins rProtein A Sepharose FF (rProtein A), Mabsorbent A2P (A2P) and MEP Hypercel (Hyper) were included for comparison.

The fraction of 'bound' protein was calculated as the difference between the total mass of protein injected $m_i$ and the mass of protein detected in the flowthrough $m_{ft}$, divided by the total mass of protein injected.

$$\text{bound} = \frac{m_i - m_{ft}}{m_i}$$

The 'yield' of protein was calculated as the mass of protein eluted $m_e$, divided by the mass of protein injected $m_i$..

$$\text{yield} = \frac{m_e}{m_i}$$

The 'Selectivity' of resins was calculated as the fraction of bound antibody $B_{Ab}$, divided by the fraction of bound host cell proteins $B_{HCP}$.

$$\text{selectivity} = \frac{B_{Ab}}{B_{HCP}}$$

The fraction of 'bound' protein and the 'yield' of protein were calculated for each resin basted on data for whole IgG-antibodies. In the case of Bevacizumab fragments and host cell proteins, only the fraction of 'bound' protein is given. Selectivity of resins was calculated from data for Bevacizumab whole IgG and host cell proteins. Due to Limited experimental precision, selectivity was truncated beyond values of 10.

Chromatography results for resins from example 3 and reference resins are given in the table below. Values for L53 are estimate values predicted on the basis of the close analogs L49 and L50.

| Ligand | Bevacizumab IgG | | Bevacizumab Fab | Bevacizumab Fc | Tocilizumab | | Palivizumab | | h-poly-IgG | | r-poly-IgG | | HCP | |
|---|---|---|---|---|---|---|---|---|---|---|---|---|---|---|
| | Bound (%) | Yield (%) | Bound (%) | Bound (%) | Bound (%) | Yield (%) | Bound (%) | Yield (%) | Bound (%) | Yield (%) | Bound (%) | Yield (%) | Bound (%) | Selectivity |
| L1 | 100 | 91 | 18 | 100 | 100 | 97 | 100 | 92 | 100 | 90 | 100 | 100 | 30 | 3.3 |
| L2 | 100 | 96 | 100 | 100 | 100 | 91 | - | - | - | - | - | - | 59 | 1.7 |
| L3 | 100 | 89 | - | - | 97 | 95 | 100 | 93 | 102 | 72 | - | - | 62 | 1.4 |
| L4 | 100 | 98 | 38 | 100 | 100 | 93 | 100 | 97 | 100 | 83 | - | - | 57 | 1.8 |
| L5 | 100 | 96 | 77 | 84 | 100 | 80 | 71 | 72 | 97 | 69 | - | - | 48 | 2.0 |
| L6 | 100 | 100 | 79 | 100 | 100 | 102 | 98 | 100 | 94 | 100 | - | - | 54 | 2.0 |
| L7 | 100 | 110 | - | - | 92 | 109 | 100 | 99 | 100 | 91 | - | - | 70 | 1.4 |
| L8 | 100 | 96 | 82 | 99 | 100 | 97 | 100 | 89 | 100 | 86 | - | - | 44 | 2.3 |
| L9 | 100 | 91 | - | - | 71 | 64 | 41 | 33 | 65 | 53 | - | - | 58 | 1.7 |
| L10 | 100 | 91 | - | - | 86 | 73 | 59 | 62 | 72 | 60 | - | - | 68 | 1.5 |
| L11 | 95 | 94 | - | - | 100 | 89 | 78 | 75 | 89 | 79 | - | - | 56 | 1.7 |
| L12 | 100 | 50 | - | - | 61 | 49 | 52 | 41 | 68 | 37 | - | - | 63 | 1.6 |
| L13 | 100 | 94 | 9 | 64 | 91 | 79 | 65 | 58 | 76 | 75 | - | - | 38 | 2.7 |
| L14 | 99 | 89 | - | - | 82 | 74 | 66 | 61 | 73 | 62 | - | - | 74 | 1.3 |
| L15 | 99 | 94 | - | - | 100 | 98 | 100 | 90 | 98 | 83 | - | - | 64 | 1.6 |
| L16 | 100 | 93 | - | - | 100 | 96 | 100 | 90 | 96 | 83 | - | - | 51 | 2.0 |
| L17 | 100 | 94 | - | - | 97 | 89 | 81 | 70 | 91 | 87 | - | - | 36 | 2.8 |
| L18 | 100 | 95 | - | - | 76 | 76 | 54 | 43 | 68 | 71 | - | - | 46 | 2.2 |
| L19 | 100 | 99 | - | - | 47 | 48 | 18 | 16 | 49 | 48 | - | - | 56 | 1.8 |
| L20 | 100 | 96 | - | - | 100 | 96 | 97 | 84 | 93 | 82 | - | - | 47 | 2.1 |
| L21 | 99 | 95 | - | - | 52 | 53 | 26 | 26 | 54 | 61 | - | - | 50 | 2.0 |
| L22 | 100 | 92 | - | - | 62 | 58 | 40 | 34 | 63 | 58 | - | - | 33 | 3.1 |
| L23 | 100 | 98 | 12 | 67 | 86 | 85 | 61 | 57 | 75 | 74 | - | - | 32 | 3.1 |
| L24 | 100 | 97 | - | - | 41 | 40 | 24 | 21 | 45 | 47 | - | - | 29 | 3.5 |
| L25 | 100 | 95 | - | - | 100 | 93 | 82 | 78 | 93 | 81 | - | - | 46 | 2.2 |
| L26 | 100 | 94 | - | - | 77 | 84 | 58 | 59 | 75 | 74 | - | - | 42 | 2.4 |
| L27 | 100 | 94 | 6 | 96 | 100 | 104 | 100 | 94 | 98 | 89 | - | - | 35 | 2.9 |
| L28 | 100 | 81 | - | - | 63 | 61 | 55 | 50 | 58 | 44 | - | - | 35 | 2.9 |
| L29 | 100 | 84 | - | - | 100 | 96 | 100 | 88 | 95 | 70 | - | - | 43 | 2.4 |
| L30 | 100 | 93 | - | - | 83 | 79 | 68 | 66 | 74 | 53 | - | - | 42 | 2.4 |
| L31 | 100 | 96 | - | - | 100 | 98 | 99 | 94 | 95 | 74 | - | - | 23 | 4.4 |

(continued)

| Ligand | Bevacizumab | | | | Tocilizumab | | Palivizumab | | h-poly-IgG | | r-poly-IgG | | HCP | |
|---|---|---|---|---|---|---|---|---|---|---|---|---|---|---|
| | IgG | | Fab | Fc | | | | | | | | | | |
| | Bound (%) | Yield (%) | Bound (%) | Bound (%) | Bound (%) | Yield (%) | Bound (%) | Yield (%) | Bound (%) | Yield (%) | Bound (%) | Yield (%) | Bound (%) | Selectivity |
| L32 | 100 | 87 | - | - | 100 | 95 | 100 | 95 | 95 | 74 | - | - | 49 | 2.0 |
| L33 | 99 | 83 | - | - | 48 | 51 | 33 | 31 | 51 | 39 | - | - | 17 | 5.8 |
| L34 | 100 | 89 | - | - | 85 | 80 | 73 | 72 | 78 | 61 | - | - | 39 | 2.6 |
| L35 | 100 | 96 | - | - | 100 | 92 | 100 | 95 | 95 | 76 | - | - | 51 | 2.0 |
| L36 | 100 | 100 | - | - | 100 | 95 | 100 | 100 | 95 | 77 | - | - | 54 | 1.9 |
| L37 | 100 | 92 | - | - | 99 | 95 | 92 | 87 | 94 | 70 | - | - | 29 | 3.4 |
| rProtA | 100 | 94 | - | - | 100 | 95 | 100 | 100 | 95 | 79 | - | - | 0 | 10.0 |
| A2P | 100 | 84 | - | - | 97 | 77 | 100 | 89 | 100 | 82 | - | - | 100 | 1.0 |
| Hyper | 100 | 83 | - | - | 100 | 71 | 100 | 59 | 95 | 57 | - | - | 77 | 1.3 |

| | Bevacizumab | | | | Tocilizumab | | Palivizumab | | h-poly-IgG | | r-poly-IgG | | HCP | |
|---|---|---|---|---|---|---|---|---|---|---|---|---|---|---|
| | IgG | | Fab | Fc | | | | | | | | | | |
| Ligand | Bound (%) | Yield (%) | Bound (%) | Bound (%) | Bound (%) | Yield (%) | Bound (%) | Yield (%) | Bound (%) | Yield (%) | Bound (%) | Yield (%) | Bound (%) | Selectivity |
| L1 | 100 | 91 | 18 | 100 | 100 | 97 | 100 | 92 | 100 | 90 | 100 | 100 | 30 | 3.3 |
| L2 | 100 | 96 | 100 | 100 | 100 | 91 | - | - | - | - | - | - | 59 | 1.7 |
| L3 | 100 | 89 | - | - | 97 | 95 | 100 | 93 | 102 | 72 | - | - | 62 | 1.4 |
| L4 | 100 | 98 | 38 | 100 | 100 | 93 | 100 | 97 | 100 | 83 | - | - | 57 | 1.8 |
| L5 | 100 | 96 | 77 | 84 | 100 | 80 | 71 | 72 | 97 | 69 | - | - | 48 | 2.0 |
| L6 | 100 | 100 | 79 | 100 | 100 | 102 | 98 | 100 | 94 | 100 | - | - | 54 | 2.0 |
| L7 | 100 | 110 | - | - | 92 | 109 | 100 | 99 | 100 | 91 | - | - | 70 | 1.4 |
| L8 | 100 | 96 | 82 | 99 | 100 | 97 | 100 | 89 | 100 | 86 | - | - | 44 | 2.3 |
| L9 | 100 | 91 | - | - | 71 | 64 | 41 | 33 | 65 | 53 | - | - | 58 | 1.7 |
| L10 | 100 | 91 | - | - | 86 | 73 | 59 | 62 | 72 | 60 | - | - | 68 | 1.5 |
| L11 | 95 | 94 | - | - | 100 | 89 | 78 | 75 | 89 | 79 | - | - | 56 | 1.7 |
| L12 | 100 | 50 | - | - | 61 | 49 | 52 | 41 | 68 | 37 | - | - | 63 | 1.6 |
| L13 | 100 | 94 | 9 | 64 | 91 | 79 | 65 | 58 | 76 | 75 | - | - | 38 | 2.7 |
| L14 | 99 | 89 | - | - | 82 | 74 | 66 | 61 | 73 | 62 | - | - | 74 | 1.3 |
| L15 | 99 | 94 | - | - | 100 | 98 | 100 | 90 | 98 | 83 | - | - | 64 | 1.6 |
| L16 | 100 | 93 | - | - | 100 | 96 | 100 | 90 | 96 | 83 | - | - | 51 | 2.0 |
| L17 | 100 | 94 | - | - | 97 | 89 | 81 | 70 | 91 | 87 | - | - | 36 | 2.8 |
| L18 | 100 | 95 | - | - | 76 | 76 | 54 | 43 | 68 | 71 | - | - | 46 | 2.2 |
| L19 | 100 | 99 | - | - | 47 | 48 | 18 | 16 | 49 | 48 | - | - | 56 | 1.8 |
| L20 | 100 | 96 | - | - | 100 | 96 | 97 | 84 | 93 | 82 | - | - | 47 | 2.1 |
| L21 | 99 | 95 | - | - | 52 | 53 | 26 | 26 | 54 | 61 | - | - | 50 | 2.0 |
| L22 | 100 | 92 | - | - | 62 | 58 | 40 | 34 | 63 | 58 | - | - | 33 | 3.1 |
| L23 | 100 | 98 | 12 | 67 | 86 | 85 | 61 | 57 | 75 | 74 | - | - | 32 | 3.1 |
| L24 | 100 | 97 | - | - | 41 | 40 | 24 | 21 | 45 | 47 | - | - | 29 | 3.5 |
| L25 | 100 | 95 | - | - | 100 | 93 | 82 | 78 | 93 | 81 | - | - | 46 | 2.2 |
| L26 | 100 | 94 | - | - | 77 | 84 | 58 | 59 | 75 | 74 | - | - | 42 | 2.4 |
| L27 | 100 | 94 | 6 | 96 | 100 | 104 | 100 | 94 | 98 | 89 | - | - | 35 | 2.9 |
| L28 | 100 | 81 | - | - | 63 | 61 | 55 | 50 | 58 | 44 | - | - | 35 | 2.9 |
| L29 | 100 | 84 | - | - | 100 | 96 | 100 | 88 | 95 | 70 | - | - | 43 | 2.4 |
| L30 | 100 | 93 | - | - | 83 | 79 | 68 | 66 | 74 | 53 | - | - | 42 | 2.4 |
| L31 | 100 | 96 | - | - | 100 | 98 | 99 | 94 | 95 | 74 | - | - | 23 | 4.4 |

(continued)

| | Bevacizumab | | | | Tocilizumab | | Palivizumab | | h-poly-IgG | | r-poly-IgG | | HCP | |
|---|---|---|---|---|---|---|---|---|---|---|---|---|---|---|
| | IgG | | Fab | Fc | | | | | | | | | | |
| Ligand | Bound (%) | Yield (%) | Bound (%) | Bound (%) | Bound (%) | Yield (%) | Bound (%) | Yield (%) | Bound (%) | Yield (%) | Bound (%) | Yield (%) | Bound (%) | Selectivity |
| L32 | 100 | 87 | - | - | 100 | 95 | 100 | 95 | 95 | 74 | - | - | 49 | 2.0 |
| L33 | 99 | 83 | - | - | 48 | 51 | 33 | 31 | 51 | 39 | - | - | 17 | 5.8 |
| L34 | 100 | 89 | - | - | 85 | 80 | 73 | 72 | 78 | 61 | - | - | 39 | 2.6 |
| L35 | 100 | 96 | - | - | 100 | 92 | 100 | 95 | 95 | 76 | - | - | 51 | 2.0 |
| L36 | 100 | 100 | - | - | 100 | 95 | 100 | 100 | 95 | 77 | - | - | 54 | 1.9 |
| L37 | 100 | 92 | - | - | 99 | 95 | 92 | 87 | 94 | 70 | - | - | 29 | 3.4 |
| L38 | 100 | 100 | - | - | 100 | 99 | 91 | 79 | 100 | 99 | - | - | 45 | 2.2 |
| L39 | 100 | 100 | - | - | 100 | 100 | 100 | 100 | 100 | 100 | - | - | 35 | 2.9 |
| L40 | 100 | 100 | - | - | 90 | 90 | 62 | 62 | 98 | 97 | - | - | 22 | 4.5 |
| L41 | 100 | 100 | - | - | 91 | 91 | 74 | 71 | 85 | 85 | - | - | 9 | 10.0 |
| L42 | 100 | 92 | - | - | 100 | 99 | 99 | 99 | 100 | 100 | - | - | 32 | 3.1 |
| L43 | 100 | 90 | - | - | 88 | 88 | 61 | 43 | 90 | 90 | - | - | 13 | 8.0 |
| L44 | 100 | 100 | - | - | 78 | 78 | 56 | 56 | 73 | 73 | - | - | 4 | 10.0 |
| L45 | 100 | 82 | - | - | 100 | 100 | 100 | 100 | 100 | 100 | - | - | 44 | 2.3 |
| L46 | 100 | 82 | - | - | 100 | 97 | 85 | 85 | 93 | 93 | - | - | 20 | 5.0 |
| L47 | 100 | 97 | - | - | 100 | 100 | 85 | 85 | 71 | 65 | - | - | 35 | 2.9 |
| L48 | 100 | 97 | - | - | 87 | 87 | 58 | 54 | 84 | 84 | - | - | 8 | 10.0 |
| L49 | 100 | 88 | - | - | 99 | 97 | - | - | 88 | 88 | - | - | 17 | 6.1 |
| L50 | 100 | 92 | - | - | 100 | 100 | - | - | 91 | 91 | - | - | 15 | 6.9 |
| L51 | 100 | 95 | - | - | 95 | 93 | 85 | 85 | 89 | 89 | - | - | 30 | 3.3 |
| L52 | 100 | 99 | - | - | 100 | 100 | 100 | 83 | 100 | 100 | - | - | 41 | 2.4 |
| L53 | >95% | >85% | - | - | >90% | >90% | - | - | - | - | - | - | <20% | >5 |
| L54 | 100 | 100 | - | - | 100 | 100 | 100 | 84 | 100 | 98 | - | - | 36 | 2.8 |
| L55 | 100 | 95 | - | - | 100 | 100 | 100 | 99 | 100 | 93 | - | - | 31 | 3.3 |
| L56 | 100 | 95 | - | - | 95 | 95 | 81 | 79 | 83 | 83 | - | - | 21 | 4.9 |
| L57 | 100 | 90 | - | - | 78 | 78 | 44 | 42 | 53 | 53 | - | - | 16 | 6.3 |
| L58 | 100 | 90 | - | - | 100 | 100 | 97 | 83 | 98 | 93 | - | - | 23 | 4.3 |
| L59 | 100 | 97 | - | - | 89 | 89 | 64 | 64 | 71 | 71 | - | - | 32 | 3.1 |
| L60 | 100 | 82 | - | - | 93 | 93 | - | - | 95 | 94 | - | - | 30 | 3.3 |
| L61 | 100 | 92 | - | - | 84 | 84 | 67 | 66 | 81 | 81 | - | - | 20 | 5.1 |

(continued)

| Ligand | Bevacizumab IgG | | Bevacizumab Fab | Bevacizumab Fc | Tocilizumab | | Palivizumab | | h-poly-IgG | | r-poly-IgG | | HCP | |
|---|---|---|---|---|---|---|---|---|---|---|---|---|---|---|
| | Bound (%) | Yield (%) | Bound (%) | Bound (%) | Bound (%) | Yield (%) | Bound (%) | Yield (%) | Bound (%) | Yield (%) | Bound (%) | Yield (%) | Bound (%) | Selectivity |
| L62 | 100 | 100 | - | - | 95 | 91 | 84 | 77 | 89 | 89 | - | - | 3 | 10.0 |
| L63 | 100 | 95 | - | - | 4 | 4 | 0 | 0 | 11 | 10 | - | - | 3 | 10.0 |
| L64 | 100 | 93 | - | - | 99 | 99 | - | - | 100 | 97 | - | - | 36 | 2.8 |
| L65 | 100 | 100 | - | - | 74 | 71 | 58 | 58 | 67 | 67 | - | - | 11 | 9.5 |
| L66 | 100 | 100 | - | - | 54 | 53 | 41 | 27 | 46 | 46 | - | - | 0 | 10.0 |
| L67 | 100 | 100 | - | - | 91 | 86 | 81 | 80 | 89 | 87 | - | - | 2 | 10.0 |
| L68 | 100 | 100 | - | - | 71 | 71 | 65 | 65 | 65 | 65 | - | - | 4 | 10.0 |
| L69 | 100 | 100 | - | - | 78 | 73 | 45 | 45 | 63 | 63 | - | - | 2 | 10.0 |
| L70 | 100 | 83 | - | - | 100 | 100 | 55 | 55 | 64 | 64 | - | - | 19 | 5.4 |
| L71 | 100 | 88 | - | - | 51 | 49 | 24 | 24 | 45 | 45 | - | - | 25 | 4.0 |
| L72 | 100 | 83 | - | - | 93 | 93 | 91 | 91 | 90 | 90 | - | - | 40 | 2.5 |
| L73 | 100 | 92 | - | - | 97 | 95 | 87 | 87 | 92 | 87 | - | - | 52 | 1.9 |
| L74 | 100 | 96 | - | - | 97 | 91 | 88 | 86 | 91 | 90 | - | - | 53 | 1.9 |
| L75 | 100 | 97 | - | - | 100 | 98 | 91 | 91 | 90 | 90 | - | - | 18 | 5.7 |
| L76 | 100 | 98 | - | - | 93 | 89 | 59 | 59 | 73 | 73 | - | - | 9 | 10.0 |
| L77 | 100 | 96 | - | - | 80 | 80 | 55 | 55 | 65 | 65 | - | - | 10 | 10.0 |
| L78 | 100 | 94 | - | - | 94 | 94 | 61 | 61 | 76 | 76 | - | - | 14 | 7.4 |
| rProtA | 100 | 94 | - | - | 100 | 95 | 100 | 100 | 95 | 79 | - | - | 0 | 10.0 |
| A2P | 100 | 84 | - | - | 97 | 77 | 100 | 89 | 100 | 82 | - | - | 100 | 1.0 |
| Hyper | 100 | 83 | - | - | 100 | 71 | 100 | 59 | 95 | 57 | - | - | 77 | 1.3 |

Controls bound all antibodies to nearly 100%. Whereas Protein A bound no host cell proteins (HCP) at all, A2P and Hypercel showed high binding properties towards them. Resulting selectivity indices were 10.0 for Protein A, 1.0 for A2P and 1.3 for Hypercel.

Among the tested ligands from example 2, L1, L27, L31 and L37 showed best results in respect of antibody binding and selectivity. All of them bound all tested human whole IgG antibodies to more than 90% and thus have binding characteristics of generic Fc-binders. Consistent with this, L1 and L27 bound the Fc-fragment of Bevacizumab but not the Fab-fragment. Additionally, for L1 efficient binding of rabbit poly-IgGs was demonstrated. Yield of Bevacizumab, Tocilizumab and Palivizumab after chromatography varied between 87% and 100%. Yield of poly-IgG was slightly lower and ranged from 70% to 90%. Selectivity indices were 3.3 for L1, 2.9 for L27, 4.4 for L31 and 3.4 for L37.

Other ligands from example 2 showed either reduced binding of at least one of the tested antibodies or binding of HCP to more than 40% (resulting selectivity indices were 2.5 or lower).

**Example 5: Isotherms and Time-Scales of Binding**

**Experimental**

Experiments were performed with Bevacizumab as antibody. Parameters of Langmuir isotherms for purified antibody were determined by batch adsorption experiments in 96-well microtiter plates. In each well, 10 $\mu$l of sorbent slurry (50% v/v) were mixed with 100 $\mu$l of protein solution. Initial concentrations varied from 0.05 - 5 mg $ml^{-1}$ in phosphate-buffered saline (0.15 M NaCl, 20 mM phosphate buffer, pH 7.3; PBS). Reactions were agitated vigorously at 25 °C for at least 3 h. Afterwards, antibody concentration in the supernatents were measured by Bradford assay.

Uptake kinetics were investigated similarly by batch adsorption in 96-well filter plates. Again, 10 $\mu$l of sorbent slurry (50% v/v) were mixed with 100 $\mu$l of protein solution. However, a fixed initial concentration of 0.75 mg $ml^{-1}$ Bevacizumab in PBS was used. Reactions were agitated vigorously at 25 °C for up to 80 min. Supernatants were separated rapidly by spinning through filters after 2.5, 5, 10, 20, 40 and 80 minutes and sampled for analysis. Concentrations of the antibody were analyzed by Bradford assay.

**Summary and Results**

A subset of immobilized ligands from example 3 was characterized with respect to their affinity and maximum capacity towards Bevacizumab. Furthermore, for L1 the time-scale required for binding was determined. Commercial resins rProtein A Sepharose FF (rProtein A) and Mabsorbent A2P (A2P) were included for comparison.

Parameters of Langmuir isotherms, i.e. dissociation constants $K_d$ and maximum capacities $q_m$, were determined from the measured concentrations in supernatants. Parameters were estimated by numerical fitting of the model equation derived by Chase (4).

Uptake kinetics were characterized by a time-scale of binding $t_{0.8}$, which was defined as the time after which 80% of the equilibrium saturation of resin with antibody had occurred. For determination of $t_{0.8}$, measured concentrations in the supernatant were interpolated by fitting of double-exponentials (5) and values of $t_{0.8}$ were read out from graphs. Parameters of Langmuir isotherms and time-scales of binding for in-house resins as well as for reference resins are reported in the table below.

| Ligand | $K_d$ ($\mu$g $ml^{-1}$) | $q_m$ (mg per ml resin) | $t_{0.8}$ (min) |
|---|---|---|---|
| L1 | 61 | 36 | 9.9 |
| L13 | 162 | 37 | - |
| L17 | 252 | 26 | - |
| L22 | 147 | 29 | - |
| L23 | 103 | 33 | - |
| L24 | 210 | 33 | - |
| L27 | 92 | 33 | - |
| rProtein A | 4.7 | 41 | 8.9 |
| A2P | 49 | 68 | 9.2 |

Highest affinity was observed for rProtein A, which had a $K_d$ of 4.7 $\mu$g $ml^{-1}$. Dissociation constants of A2P (49 $\mu$g $ml^{-1}$), resins L1 (61 $\mu$g $ml^{-1}$) and L27 (92 $\mu$g $ml^{-1}$) were lower by one order of magnitude. Dissociation constants of the residual resins were lower by two orders of magnitude and ranged from 103 $\mu$g $ml^{-1}$ (L23) to 252 $\mu$g $ml^{-1}$ (L17).

Concerning maximum capacity, the highest capacity was measured for A2P (69 mg per ml of resin). rProtein A had a maximum capacity of 41 mg per ml of resin. Maximum capacities of in-house resins varied between 26 and 37 mg per ml of resin. Uptake of antibody was fastest with rProtein A (8.9 min), followed by A2P (9.3 min) and L1 (9.9 min).

## Example 6: Dynamic Binding Capacity

### Experimental

Dynamic binding capacities were determined by column chromatography with purified Bevacizumab. Resins were packed into Analytical columns of 25 mm length and 3 mm inner diameter. Columns were fed with 1 mg $ml^{-1}$ antibody in phosphate-buffered saline (150 mM NaCl, 20 mM phosphate buffer, pH 7.3) at constant flow rate, while the concentration of antibody in effluents was monitored online via absorbance at 280 nm. Antibody was loaded until effluent concentration reached at least 10% of the feed concentration. Dynamic binding capacities were determined at a flow rate of 50 cm $h^{-1}$, corresponding to 3 min empty column residence time. Equilibrium capacities were also determined at a flow rate of 50 cm $h^{-1}$ by loading until complete breakthrough of antibody. Bound antibody was stripped from columns by washing with sodium citrate at pH 3.0 followed by glycine hydrochloride at pH 2.5.

### Summary and Results

Dynamic (binding) capacity for Bevacizumab as a function of flow rate was determined for L1 (from example 3) and rProtein A Sepharose 4 FF (rProtein A).

'Dynamic capacity' was defined as the amount of antibody that can be injected onto a column at constant flow rate until the effluent concentration reaches 10% of the feed concentration. It was calculated as the time $t_{0.1}$ after which 10% breakthrough occurred, multiplied by the flow rate F and feed concentration $c_f$.

$$\text{dynamic capacity} = t_{0.1} \cdot F \cdot c_f$$

Equilibrium capacity is defined as the maximum amount of antibody that binds to a column for a given feed stream concentration. It was calculated by evaluation of the following integral

$$\text{equilibrium capacity} = \int_0^\infty \left( c_f - c(t) \right) \cdot F dt$$

containing the feed concentration $c_f$, effluent concentration over time c(t) and flow rate F.

The integral was evaluated numerically. Integration was limited to the time after which complete breakthrough had occurred. Calculations of dynamic capacity and equilibrium capacity were both corrected for the hold-up of columns and the chromatography system. Capacities were normalized by the volume of resin in columns

Dynamic binding capacities for L1 and rProtein A Sepharose 4 FF as a function of flow rate and equilibrium binding capacities for a feed concentration of 1 mg $ml^{-1}$ Bevacizumab are given in the table below.

For rProtein A a dynamic binding capacity of 28.4 mg per ml or resin was determined. L1 had a dynamic capacity of 20.2 mg per ml of resin. The calculated equilibrium capacity was 40 mg per ml of resin for rProtein A and 25.7 mg per ml of resin for L1.

| Ligand | Dynamic Capacity (mg per ml of resin) | Equilibrium Capacity (mg per ml of resin) |
|---|---|---|
| L1 | 20.2 | 25.7 |
| rProtein A | 28.4 | 40.2 |

## Example 7: Alkaline Stability

Ligand L1 from example 2 was tested for its alkaline stability over a period of 8 days. It was either treated with 0.1 M or 0.5 M sodium hydroxide at 25 °C. Hydrolysis was monitored by LC-MS analysis.

At 0.5 M NaOH, half-life of L1 was 288 h. At 0.1 M NaOH, no degradation of the ligand was detected.

**Example 8: Purification of Antibody from Cell Culture Supernatant**

**Experimental**

Suitability of resins for the purification of antibody from cell culture supernatant was assessed either in packed mode by microtiter plate chromatography similar to example 3 or by regular column chromatography. In both cases, Bevacizumab spiked into host cell proteins at a concentration of 0.05 mg $ml^{-1}$ was used as feed. In addition, chromatography runs with host cell proteins (microtiter plate and column chromatography) and pure antibody alone (only microtiter plate chromatography) were conducted. For microtiter plate chromatography, 25 column volumes were injected per run. For column chromatography 100 column volumes were injected. Columns were equilibrated and washed with PBS before and after injection. Bound protein was eluted with sodium citrate at pH 3.0. Protein concentrations in feeds and column effluents were analyzed either by Bradford assay or by UV-absorbance at 280 nm.

**Summary and Results**

Antibody was purified from cell culture supernatant by chromatography on resin L1 from example 3. Chromatographies on commercial resins rProtein A Sepharose FF (rProtein A) and Mabsorbent A2P (A2P) were included for comparison. For microtiter plate chromatography, three chromatography runs under identical conditions were conducted on each resin, either injecting the antibody, host cell proteins or a mixture of the latter. For column chromatography, only two runs were performed, either with antibody spiked into host cell proteins or host cell proteins alone.

Operation 'yield' after Chromatography of the mixture was calculated from the mass of total protein $m_{mix,e}$ recovered upon elution after injection of the mixture, the mass recovered after injection of host cell proteins alone $m_{HCP,e}$ and the mass of antibody injected $m_{Ab,i}$.

[redacted]

The 'purity' of antibody after chromatography of the mixture was calculated from the mass of total protein recovered upon elution $m_{mix,e}$ after injection of the mixture and the mass $m_{HCP,e}$ recovered after injection of host cell proteins.

$$purity = \frac{m_{mix,e} - m_{HCP,e}}{m_{mix,e}}$$

Purity and yield obtained after chromatography on resin L1 from example 3 and reference resins are given in the table below.

Within experimental precision, yields were 100% for protein A, 85% for L1 And 12% for A2P after microliter plate chromatography. Recovery from A2P (12%) was low due to moderate acidic pH of 3.0 upon elution. Purity was highest after chromatography on protein A (100%), followed by chromatography on resin L1 (74%). Purity was lowest after chromatography on A2P with only 32%. Results after column chromatography were in similar range. Here, rProtein A showed a yield of 97% and a purity of 100%. L1 showed a yield of 90% and a purity of 83%.

| | Microtiter Plate Chromatography | | Column Chromatography | |
|---|---|---|---|---|
| **Ligand** | **Yield (%)** | **Purity (%)** | **Yield (%)** | **Purity (%)** |
| L1 | 85 | 74 | 90 | 83 |
| rProtein A | 106 | 100 | 97 | 100 |
| A2P | 12 | 32 | - | - |

Documents cited in the examples:

1. T. Neumann, HD Junker, K. Schmidt, R. Sekul, SPR Based Fragment Screening: Advantages and Applications,

Current Topics in Medicinal Chemistry 2007; 7: 1630-1642

2. Roth M. Fluorescence reaction for amino acids. Anal Chem 197; 43(7):880-2.

3. Rousseaux J, Rousseaux-Prévost R, Bazin H. Optimal conditions for the preparation of Fab and F(ab')2 fragments from monoclonal IgG of different rat IgG subclasses. J. Immunol. Methods 1983; 64(1-2):141-146.

4. Chase HA. Prediction of the performance of preparative affinity chromatography. J Chromatogr 1984; 297:179-202.

5. Coffman JL, Kramarczyk JF, Kelley BD. High-throughput screening of chromatographic separations: I. Method development and column modeling. Biotechnology and Bioengineering 2008; 100(4):605-618.

## Claims

1. Use, for affinity purification of an antibody or a fragment of an antibody, of a ligand-substituted matrix comprising a support material and at least one ligand covalently bonded to the support material, the ligand being represented by formula (I)

(I)

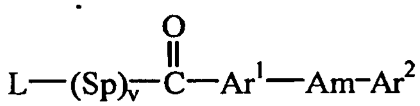

wherein

L is the linking point on the support material to which the ligand is attached;
Sp is a spacer group;
v is 0 or 1;
Am is an amide group $-NR^1-C(O)-$, and wherein either $NR^1$ is attached to $Ar^1$ and - C(O)- is attached to $Ar^2$, or -C(O)- is attached to $Ar^1$ and $NR^1$ is attached to $Ar^2$; and
$R^1$ is hydrogen or $C_1$ to $C_4$ alkyl, preferably hydrogen or methyl; and more preferably hydrogen;
$Ar^1$ is a divalent 5- or 6-membered substituted or unsubstituted aromatic ring, wherein the substituents are selected from $C_1$ to $C_4$ alkoxy, $C_1$ to $C_4$ alkyl, and combinations thereof;
$Ar^2$ is 5- or 6-membered unsubstituted or substituted heterocyclic aromatic ring which is

(a) attached to a further 5- or 6-membered aromatic ring via a single bond; or
(b) fused to a further 5- or 6-membered aromatic ring as part of a multicyclic ring system; or
(c) attached to at least one substituent selected from $C_1$ to $C_4$ alkyl; $C_2$ to $C_4$ alkenyl; $C_2$ to $C_4$ alkynyl; a halogen; $C_1$ to $C_4$ haloalkyl; hydroxyl-substituted $C_1$ to $C_4$ alkyl; $C_1$ to $C_4$ alkoxy; hydroxyl-substituted $C_1$ to $C_4$ alkoxy; $C_1$ to $C_4$ alkylamino; $C_1$ to $C_4$ alkylthio; and combinations thereof;

and wherein the first aromatic ring or the second aromatic ring or both, in alternative (a) and (b) may optionally carry one or more further substituents from those mentioned under (c).

2. The use of claim 1 wherein $Ar^1$ is phenylene, preferably methoxy-substituted phenylene.

3. The use of claim 1 or 2 wherein the C=O and the NH group are bonded to $Ar^1$ in meta position to each other.

4. The use of any of claims 1 to 3 wherein the 5- or 6-membered heterocyclic aromatic ring of $Ar^2$ is attached to the C=O group via a carbon ring atom which is adjacent to a ring heteroatom, preferably a nitrogen or oxygen atom.

5. The use of any of claims 1 to 4 wherein the 5- or 6-membered heterocyclic aromatic ring of $Ar^2$ contains two or more nitrogen atoms or one or more nitrogen atoms and an oxygen atom.

6. The use of any of claim 5 wherein the 5- or 6-membered heterocyclic aromatic ring of $Ar^2$ is N-methyl-substituted

pyrazole, pyridine, isoxazole or oxadiazole.

7. The use according to any of claims 1 to 7 wherein the support material comprises a material selected from carbohydrates or crosslinked carbohydrates, preferably agarose, cellulose, dextran, starch, alginate and carrageenan, Sepharose, Sephadex; synthetic polymers, preferably polystyrene, styrene-divinylbenzene copolymers, polyacrylates, PEG-Polycacrylate copolymers polymethacrylates, polyvinyl alcohol, polyamides and perfluorocarbons; inorganic materials, preferably glass, silica and metal oxides; and composite materials.

8. The use according to any of claims 1 to 7 wherein the protein is an antibody, preferably an IgG type antibody, or an Fc fusion protein.

9. The use of claim 8 wherein the purification is attained by binding of the ligand of the ligand-substituted matrix to an Fc fragment or domain of the antibody or the fusion protein.

10. The use according to claims 8 or 9 wherein the Fc fragment or domain or the antibody belongs to the IgG antibody class, more preferably to human, IgG or to polyclonal or monoclonal IgG of human origin, in particular to $IgG_1$, $IgG_2$ and $IgG_4$.

11. A ligand-substituted matrix as defined in any of claims 1 to 7.

12. A method of synthesis of a ligand-substituted matrix according to claim 11 wherein a ligand according to formula (I) as specified in any of claims 1 to 7 is attached to the support material.

13. A method for affinity purification, preferably affinity chromatography, of a protein, wherein a protein to be purified is contacted with a ligand-substituted matrix as defined in claims any of claims 1 to 7.

14. The method of claim 13 wherein the protein is an antibody or an Fc fusion protein and the ligand binds to the Fc region of the antibody or the Fc fusion protein.

15. A ligand as shown in formula (II), wherein Sp, $Ar^1$, $Ar^2$ and Am have the meanings defined in claims 1-7.

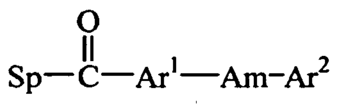

(II)

## Patentansprüche

1. Verwendung einer Liganden-substituierten Matrix umfassend ein Trägermaterial und mindestens einen kovalent an das Trägermaterial gebundenen Liganden zur Affinitätsreinigung eines Antikörpers oder eines Fragments eines Antikörpers, wobei der Ligand durch die Formel (I) dargestellt ist

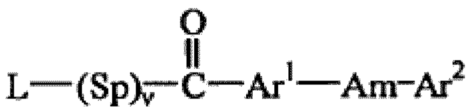

(I)

wobei

L den Verknüpfungspunkt auf dem Trägermaterial darstellt, an welchen der Ligand gebunden ist;
Sp eine Spacer-Gruppe ist;
v 0 oder 1 ist;
Am eine Amid-Gruppe $-NR^1-C(O)-$ ist, wobei entweder $NR^1$ an $Ar^1$ gebunden und -C(O)- an $Ar^2$ gebunden ist, oder -C(O) - an $Ar^1$ gebunden und $NR^1$ an $Ar^2$ gebunden ist; und $R^1$ Wasserstoff oder $C_1$- bis $C_4$-Alkyl ist, vorzugsweise Wasserstoff oder Methyl; und noch mehr bevorzugt Wasserstoff;
$Ar^1$ ein bivalenter 5- oder 6-gliedriger substituierter oder unsubstituierter aromatischer Ring ist, wobei die Sub-

stituenten ausgewählt sind aus $C_1$- bis $C_4$-Alkoxy, $C_1$- bis $C_4$-Alkyl und Kombinationen hiervon;
$Ar^2$ ein 5- oder 6-gliedriger unsubstituierter oder substituierter heterozyklischer aromatischer Ring ist, der

(a) an einen weiteren 5- oder 6-gliedrigen aromatischen Ring über eine Einfachbindung gebunden ist; oder
(b) mit einem 5- oder 6-gliedrigen aromatischen Ring als Teil eines multizyklischen Ringsystems vereinigt ist; oder
(c) an mindestens einen Substituenten gebunden ist, der ausgewählt ist aus $C_1$- bis $C_4$-Alkyl; $C_2$- bis $C_4$-Alkenyl; $C_2$- bis $C_4$-Alkinyl; einem Halogen; $C_1$- bis $C_4$-Halogenalkyl; Hydroxyl-substituiertem $C_1$- bis $C_4$-Alkyl; $C_1$- bis $C_4$-Alkoxy; Hydroxyl-substituiertem $C_1$-bis $C_4$-Alkoxy; $C_1$- bis $C_4$-Alkylamino; $C_1$- bis $C_4$-Alkylthio und Kombinationen hiervon,

und wobei in Alternative (a) und (b) der erste aromatische Ring oder der zweite aromatische Ring oder beide optional einen oder mehrere weitere Substituenten aus den unter (c) genannten tragen kann.

**2.** Verwendung nach Anspruch 1, wobei $Ar^1$ Phenylen ist, vorzugsweise Methoxy-substituiertes Phenylen.

**3.** Verwendung nach Anspruch 1 oder 2, wobei die C=O- und die NH-Gruppe in meta-Stellung zueinander an $Ar^1$ gebunden sind.

**4.** Verwendung nach einem der Ansprüche 1 bis 3, wobei der 5-oder 6-gliedrige heterozyklische aromatische Ring gemäß $Ar^2$ an die C=O-Gruppe über ein Ring-Kohlenstoff-Atom gebunden ist, welches benachbart zu einem Ring-Heteroatom ist, bevorzugt ein Stickstoff- oder Sauerstoffatom.

**5.** Verwendung nach einem der Ansprüche 1 bis 4, wobei der 5-oder 6-gliedrige heterozyklische aromatische Ring gemäß $Ar^2$ zwei oder mehr Stickstoffatome oder ein oder mehr Stickstoffatome und ein Sauerstoffatom enthält.

**6.** Verwendung nach einem der Ansprüche 1 bis 5, wobei der 5-oder 6-gliedrige heterozyklische aromatische Ring gemäß $Ar^2$ N-Methyl-substituiertes Pyrazol, Pyridin, Isoxazol oder Oxadiazol ist.

**7.** Verwendung nach einem der Ansprüche 1 bis 6, wobei das Trägermaterial ein Material umfasst, das ausgewählt ist aus Kohlenhydraten oder vernetzten Kohlenhydraten, vorzugsweise Agarose, Cellulose, Dextran, Stärke, Alginat und Karrageen, Sepharose, Sephadex; synthetischen Polymeren, vorzugsweise Polystyrol, Styrol-Divinylbenzol-Copolymeren, Polyacrylaten, PEG-Polyacrylat-Copolymeren, Polymethacrylaten, Polyvinylalkohol, Polyamiden und Perfluorkohlenstoffen; anorganischen Materialien, vorzugsweise Glas, Silizium und Metalloxiden; und Kompositmaterialien.

**8.** Verwendung nach einem der Ansprüche 1 bis 7, wobei das Protein ein Antikörper ist, vorzugsweise ein IgG-Typ Antikörper, oder ein Fc-Fusionsprotein.

**9.** Verwendung nach Anspruch 8, wobei die Reinigung durch Bindung des Liganden der Liganden-substituierten Matrix zu einem Fc-Fragment oder einer Domäne des Antikörpers oder des Fusionsproteins erreicht wird.

**10.** Verwendung nach Anspruch 8 oder 9, wobei das Fc-Fragment oder die Domäne oder der Antikörper zur Klasse der IgG-Antikörper gehört, mehr bevorzugt zu menschlichem IgG oder zu polyklonalem oder monoklonalem IgG menschlichen Ursprungs, insbesondere zu $IgG_1$, $IgG_2$ und $IgG_4$.

**11.** Liganden-substituierte Matrix wie in einem der Ansprüche 1 bis 7 definiert.

**12.** Verfahren zur Herstellung einer Liganden-substituierten Matrix nach Anspruch 11, wobei ein Ligand gemäß Formel (I), wie in einem der Ansprüche 1 bis 7 spezifiziert, an dem Trägermaterial befestigt ist.

**13.** Verfahren zur Affinitätsreinigung, vorzugsweise Affinitätschromatographie, eines Proteins, wobei ein zu reinigendes Protein mit einer wie in einem der Ansprüche 1 bis 7 definierten Liganden-substituierten Matrix in Kontakt gebracht wird.

**14.** Verfahren nach Anspruch 13, wobei das Protein ein Antikörper oder ein Fc-Fusionsprotein ist und der Ligand an den Fc-Bereich des Antikörpers oder des Fc-Fusionsproteins bindet.

**15.** Ligand wie in Formel (II) gezeigt, wobei Sp, $Ar^1$, $Ar^2$ und Am die gleiche Bedeutung wie in den Ansprüchen 1 bis 7 definiert haben.

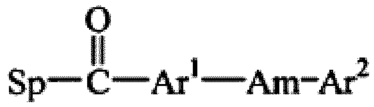

(II)

## Revendications

**1.** Utilisation pour purification par affinité d'un anticorps ou d'un fragment d'un anticorps, d'une matrice substituée par un ligand comprenant un matériau support et au moins un ligand lié par covalence au matériau support, le ligand étant représenté par la formule (I)

(I)

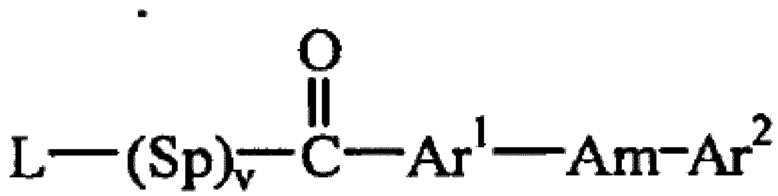

dans laquelle

L est le point de liaison sur le matériau support auquel le ligand est relié ;
Sp est un groupe écarteur ;
v est 0 ou 1 ;
Am est un groupe amide $-NR^1-C(O)-$, et dans laquelle $NR^1$ est relié à $Ar^1$ et -C(O)- est relié à $Ar^2$, ou -C(O)-est relié à $Ar^1$ et $NR^1$ est relié à $Ar^2$ ; et
$R^1$ est hydrogène ou alkyle en $C_1$ à $C_4$, de préférence hydrogène ou méthyle ; ou plus préférablement hydrogène ;
$Ar^1$ est un anneau aromatique divalent substitué ou non substitué à 5 ou 6 membres, dans laquelle les substituants sont sélectionnés parmi alkoxy en $C_1$ à $C_4$, alkyle en $C_1$ à $C_4$ et des combinaisons de ceux-ci ;
$Ar^2$ est un anneau aromatique hétérocyclique substitué ou non substitué à 5 ou 6 membres qui est

(a) relié à un autre anneau aromatique à 5 ou 6 membres par le biais d'une seule liaison ; ou
(b) fusionné à un autre anneau aromatique à 5 ou 6 membres dans le cadre d'un système d'anneaux multicyclique ; ou
(c) relié à au moins un substituant sélectionné parmi alkyle en $C_1$ à $C_4$ ; alkényle en $C_2$ à $C_4$ ; alkynyle en $C_2$ à $C_4$ ; un halogène ; halogénoalkyle en $C_1$ à $C_4$ ; alkyle en $C_1$ à $C_4$ hydroxyle-substitué ; alkoxy en $C_1$ à $C_4$ hydroxy-substitué ; alkylamino en $C_1$ à $C_4$ ; alkylthio en $C_1$ à $C_4$ ; et des combinaisons de ceux-ci ;

et dans laquelle le premier anneau aromatique ou le second anneau aromatique ou les deux, dans les variantes (a) et (b), peuvent porter en option un ou plusieurs autres substituants parmi ceux mentionnés en (c).

**2.** Utilisation selon la revendication 1, dans laquelle $Ar^1$ est phénylène, de préférence phénylène méthoxy-substitué.

**3.** Utilisation selon la revendication 1 ou 2, dans laquelle le groupe C=O et le groupe NH sont liés à $Ar^1$ en position méta l'un par rapport à l'autre.

**4.** Utilisation selon l'une quelconque des revendications 1 à 3, dans laquelle l'anneau aromatique hétérocyclique à 5 ou 6 membres de $Ar^2$ est relié au groupe C=O par le biais d'un atome cyclique de carbone qui est adjacent à un hétéroatome cyclique, de préférence un atome d'azote ou d'oxygène.

5. Utilisation selon l'une quelconque des revendications 1 à 4, dans laquelle l'anneau aromatique hétérocyclique à 5 ou 6 membres de $Ar^2$ contient deux atomes d'azote ou plus ou un ou plusieurs atomes d'azote et un atome d'oxygène.

6. Utilisation selon l'une quelconque de la revendication 5, dans laquelle l'anneau aromatique hétérocyclique à 5 ou 6 membres de $Ar^2$ est pyrazole N-méthyl-substitué, pyridine, isoxazole ou oxadiazole.

7. Utilisation selon l'une quelconque des revendications 1 à 7, dans laquelle le matériau support comprend un matériau sélectionné parmi des glucides ou glucides à réticulation croisée, de préférence l'agarose, la cellulose, la dextrane, l'amidon, l'alginate et la carraghénine, le Sepharose, le Sephadex ; des polymères synthétiques, de préférence le polystyrène, des copolymères styrène-divinylbenzène, des polyacrylates, des copolymères PEG-polyacrylate, des polyméthacrylates, l'alcool polyvinylique, des polyamides et des perfluorocarbones ; des matériaux inorganiques, de préférence le verre, le silice et des oxydes métalliques ; et des matériaux composites.

8. Utilisation selon l'une quelconque des revendications 1 à 7, dans laquelle la protéine est un anticorps, de préférence un anticorps de type IgG, ou une protéine de fusion Fc.

9. Utilisation selon la revendication 8, dans laquelle la purification est obtenue par la liaison du ligand de la matrice substituée par un ligand à un fragment ou domaine Fc de l'anticorps ou de la protéine de fusion.

10. Utilisation selon la revendication 8 ou 9, dans laquelle le fragment ou domaine Fc ou l'anticorps appartient à la classe d'anticorps IgG, plus préférablement à l'IgG humaine ou à l'IgG polyclonale ou monoclonale d'origine humaine, notamment à $IgG_1$, $IgG_2$ et $igG_4$.

11. Matrice substituée par un ligand telle que définie dans l'une quelconque des revendications 1 à 7.

12. Procédé de synthèse d'une matrice substituée par un ligand selon la revendication 11, dans lequel un ligand selon la formule (I) telle que spécifiée dans l'une quelconque des revendications 1 à 7 est relié au matériau support.

13. Procédé pour purification par affinité, de préférence chromatographie d'affinité, d'une protéine, dans lequel une protéine devant être purifiée est mise en contact avec une matrice substituée par un ligand telle que définie dans l'une quelconque des revendications 1 à 7.

14. Procédé selon la revendication 13, dans lequel la protéine est un anticorps ou une protéine de fusion Fc et le ligand se lie à la région Fc de l'anticorps ou de la protéine de fusion Fc.

15. Ligand tel qu'illustré à la formule (II), dans lequel Sp, $Ar^1$, $Ar^2$ et Am ont les significations définies aux revendications 1 à 7.

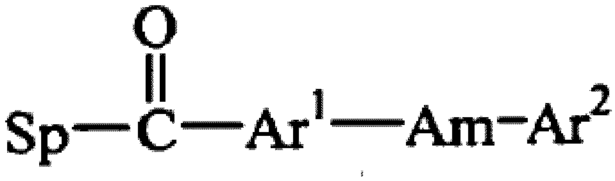

(II)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 10008089 A


### Non-patent literature cited in the description


- **A.CECILIA ; A.ROQUE, C.R. ; LOWE, M.A.** Taipa: Antibodies and Genetically Engineered Related Molecules: Production and Purification. *Biotechnol. Prog.,* 2004, vol. 20, 639-654
- **K.L.CARSON.** Flexibility-the guiding principle for antibody manufacturing. *Nature Biotechnology,* 2005, vol. 23, 1054-1058
- **S.HOBER ; K.NORD ; M. LINHULT.** Protein A chromatography for antibody purification. *J. Chromatogr. B.,* 2007, vol. 848, 40-47
- **T.ARAKAWA ; Y.KITA ; H.SATO ; D. EJIMA.** *Protein Expression and Purification,* 2009, vol. 63, 158-163
- **S. GHOSE ; B. HUBBARD ; S.M. CRAMER.** *Journal of Chromatography A,* 2006, vol. 1122, 144-152
- **T. NEUMANN ; HD JUNKER ; K. SCHMIDT ; R. SEKUL.** SPR Based Fragment Screening: Advantages and Applications. *Current Topics in Medicinal Chemistry,* 2007, vol. 7, 1630-1642
- **ROTH M.** Fluorescence reaction for amino acids. *Anal Chem,* vol. 43 (7), 880-2
- **ROUSSEAUX J ; ROUSSEAUX-PRÉVOST R ; BAZIN H.** Optimal conditions for the preparation of Fab and F(ab')2 fragments from monoclonal IgG of different rat IgG subclasses. *J. Immunol. Methods,* 1983, vol. 64 (1-2), 141-146
- **CHASE HA.** Prediction of the performance of preparative affinity chromatography. *J Chromatogr,* 1984, vol. 297, 179-202
- **COFFMAN JL ; KRAMARCZYK JF ; KELLEY BD.** High-throughput screening of chromatographic separations: I. Method development and column modeling. *Biotechnology and Bioengineering,* 2008, vol. 100 (4), 605-618